# EUROPEAN PATENT APPLICATION

(11) **EP 4 729 073 A1**
(43) Date of publication of application: **22.04.2026**
(21) Application number: 24207074.6
(22) Date of filing: 16.10.2024
(51) Int. Cl.: A61K 47/64, A61P 31/04, C07K 16/12

(54) **SYNTHETIC VACCINES AGAINST FRANCISELLA TULARENSIS**

(71) Applicant: Max-Planck-Gesellschaft zur Förderung der Wissenschaften e.V., 80539 München (DE)
(72) Inventor: The designation of the inventor has not yet been filed
(74) Representative: Arth, Hans-Lothar

(57) **Abstract**

The present invention relates to a synthetic saccharide of general formula (**I**) that is related to O-antigen capsular polysaccharide of *Francisella tularensis,* a conjugate thereof and the use of said saccharide and conjugate for raising a protective immune response in a human and/or animal host. Furthermore, the synthetic saccharide of general formula (**I**) is useful as marker in immunological assays for detection of antibodies against *Francisella tularensis* bacteria type A and type B. Another aspect of the present invention is directed to a monoclonal antibody having specificity for a synthetic saccharide of general formula (**I**).

## Description

### Field of the invention

The present invention relates to a synthetic saccharide of general formula (**I**) that is related to O-antigen capsular polysaccharide of *Francisella tularensis,* a conjugate thereof and the use of said saccharide and conjugate for raising a protective immune response in a human and/or animal host. Furthermore, the synthetic saccharide of general formula (**I**) is useful as marker in immunological assays for detection of antibodies against *Francisella tularensis* bacteria. Another aspect of the present invention is directed to a monoclonal antibody having specificity for a synthetic saccharide of general formula (**I**).

### Background of the invention

*Francisella tularensis,* the causative agent of tularemia (rabbit fever), is an aerobic, Gram-negative, facultative intracellular bacterium. As a zoonotic pathogen, it can affect a variety of animals and can be transmitted to humans in different ways with various clinical outcomes (see e.g. Appelt, S.; Faber, M.; Köppen, K.; Jacob, D.; Grunow, R.; Heuner, K. Francisella tularensis Subspecies holarctica and Tularemia in Germany. Microorganisms 2020, 8, 1448). *F. tularensis* can be the result of infected insect bites (in Germany mainly ticks), inhalation, ingestion of contaminated food or water, or inoculation through a break in the skin. This highly virulent pathogen requires approximately ten organisms to cause disease in humans. The disease outcome depends on the virulence of the infecting organisms and the infection route, where pneumonic tularemia is the most severe form with a 30% to 60% mortality rate if left untreated (J. Sharon et al., Clinical and Vaccine Immunology 2014, 21, 227-233). Classified as a category A bioterrorism agent by the Center for Disease Control and Prevention (CDC), *F. tularensis* can be easily aerosolized for high infectivity in humans (A. Chong, J. Celli, Front. Microbiol. 2010, 1, 138). The subspecies *tularensis* (type A) and *holarctica* (type B) mainly cause human tularemia, with type A being more severe and life-threatening, while type B is more prevalent (G. Stefanetti, N. Okan, A. Fink, E. Gardner, D. L. Kasper, Proc. Natl. Acad. Sci. U. S. A. 2019, 116, 7062-7070).

A live attenuated vaccine strain of type B *F. tularensis* strain has been developed clinically but it did not provide complete protection against all forms of the infections especially against pulmonary infection caused by inhalation. A new defined subunit vaccine comprised of *F. tularensis* cell surface *O*-antigen components such as lipopolysaccharide (LPS), or *O*-antigen capsular polysaccharide (CPS) was developed (see R. W. Titball et al., Nat. Rev. Microbiol. 2004, 2, 967-978; and J. Sharon, Clinical and Vaccine Immunology 2014, 21, 227-233). Mice vaccinated with *O*-antigen from mild acid hydrolysis of *F. tularensis* LPS, conjugated with bovine serum albumin (BSA), showed complete protection against intradermal challenge with highly virulent type B strain and partial protection against aerosol challenge with more severe type A strain. A highly branched synthetic hexasaccharide resembling inner core of *F. tularensis* LPS, unlike the full length LPS, did not elicit an antigenic response in mice (see G.-J. Boons, J. Am. Chem. Soc. 2012, 134, 14255-14262).

The *O*-antigen capsular polysaccharide of *F. tularensis* strain 15 is an alternative for the development of diagnostics and vaccines against tularemia. Both type A *tularensis* and type B *holarctica* subspecies share a common *O*-antigen capsular polysaccharide on their cell surfaces containing a tetrasaccharide repeating unit (RU), wherein one or two repeating units are sufficient to provide protection against tularemia. 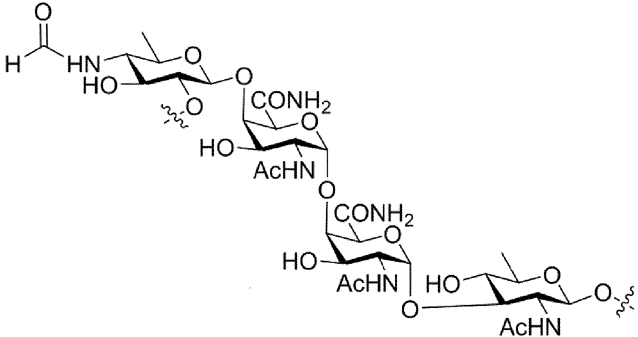 → 2)-*β*-D-Quip4NFm-(1→4)-*α*-D-GalNAcAN-(1→4)-*α*-D-GalNAcAN-(1→3)-*β*-D-QuipNAc-(1→

Bacterial capsular polysaccharides are promising vaccine candidates against a number of pathogens given their presentation of unique glycans at the termini (P. H. Seeberger, Chem. Rev., 2021, 121, 3598-3626.). Nevertheless, the advancement of vaccines, diagnostics, or therapeutic tools necessitates a comprehensive structural understanding concerning the glycan motif that is recognized by protective antibodies (D. L. Kasper et al., Proc. Natl. Acad. Sci. U. S. A. 2019, 116, 7062-7070). Although oligosaccharide fragments from *F. tularensis* can be obtained by mild hydrolysis of LPS, their isolation is hazardous (G.-J. Boons et al., J. Am. Chem. Soc. 2012, 134, 14255-14262.). Therefore, the chemical synthesis of oligosaccharide fragments resembling *F. tularensis* strains is an attractive alternative. The *O*-antigen repeating unit of *F. tularensis* strain 15 CPS consists of two rare deoxy amino sugars, *N*-acetyl-D-quinovosamine (QuipNAc) and *N*-formyl-4-amino-D-quinovose (Quip4NFm), and two *N*-acetyl-D-galactosamine uronamides (GalNAcAN). The presence of two different types of rare deoxy amino sugars, two oxidative centers in D-galactosamine derivatives, two consecutive 1,2-*cis α*-glycosidic linkages, and three different *N*-functional groups in the RU provides a multitude of synthetic challenges.

It is the objective of the present invention to provide a saccharide of general formula (**I**) that is related to the *Francisella tularensis* strain 15 *O*-antigen capsular polysaccharide, as well as a conjugate of the saccharide of general formula (**I**) with an immunogenic carrier, such as a carrier protein. The saccharide of general formula (**I**), and particularly the conjugate of said saccharide with an immunogenic carrier is able to raise a protective immune response against *F. tularensis O*-antigen CPS in a human and/or animal host. Thus, a vaccine composition for immunization against *F. tularensis* comprising the saccharide of general formula (**I**), and/or a conjugate thereof is provided. Furthermore, the synthetic saccharide of general formula (**I**) is useful as marker in immunological assays for detection of antibodies against *F. tularensis* bacteria.

The objective of the present invention is solved by the teaching of the independent claims. Further advantageous features, aspects and details of the invention are evident from the dependent claims, the description, the figures, and the examples of the present application.

### Description of the invention

### Definitions

The term *"linker"* as used herein encompasses molecular fragments capable of connecting the reducing-end monosaccharide of a saccharide with an immunogenic carrier or a solid support, optionally by binding to at least one interconnecting molecule. Thus, the function of the linker *per se* or together with the interconnecting molecule is to establish, keep and/or bridge a special distance between the reducing-end monosaccharide and an immunogenic carrier or a solid support. More specifically, one extremity of the linker is connected to the exocyclic oxygen atom at the anomeric center of the reducing-end monosaccharide and the other extremity is connected *via* the nitrogen atom with the interconnecting molecule, or directly with the immunogenic carrier or the solid support.

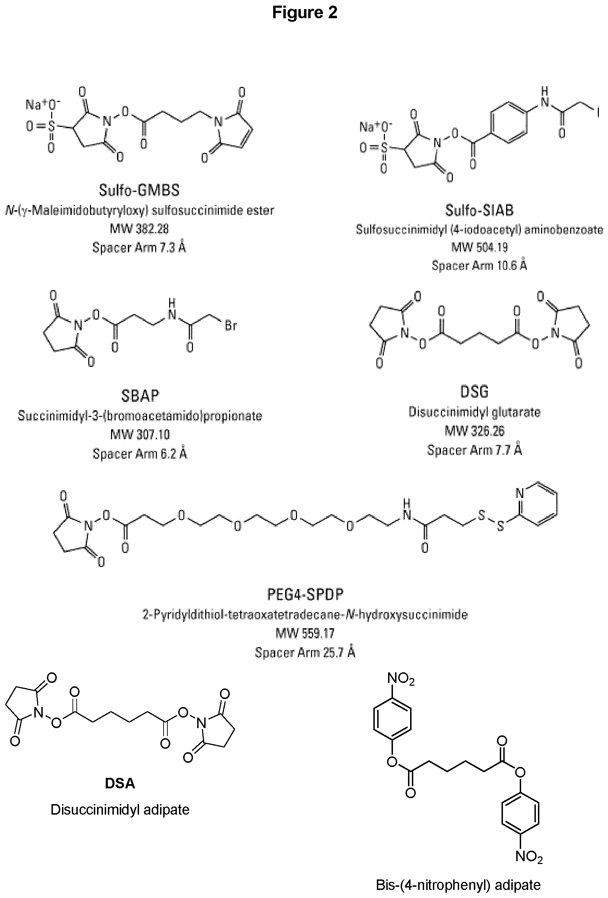

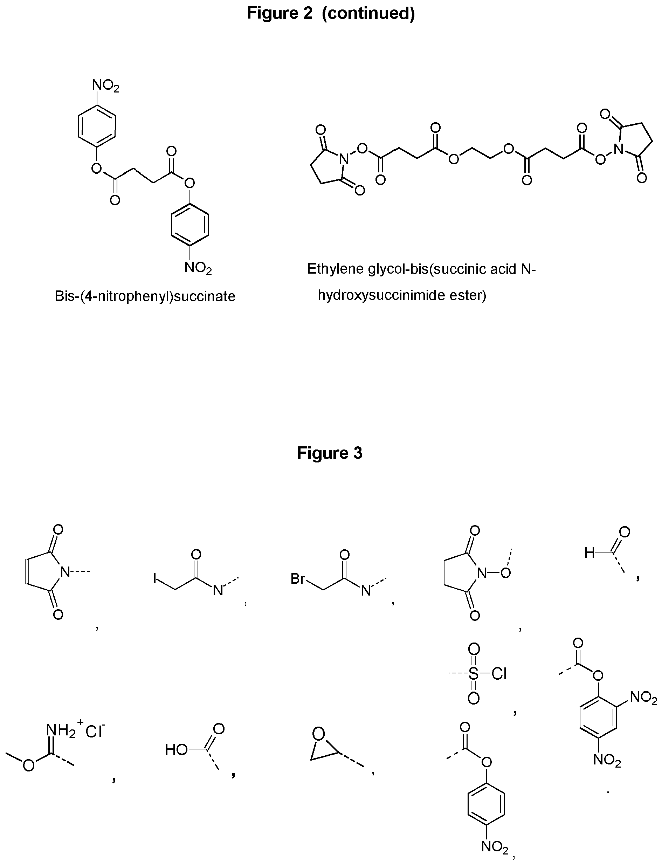

As used herein, the term "*interconnecting molecule*" refers to a bifunctional molecule containing functional group X and functional group Y, wherein functional group X is capable of reacting with the terminal amino group on the linker L and the functional group Y is capable of reacting with a functionality present on an immunogenic carrier or on a solid support. Figure 2 displays examples of commercially available interconnecting molecules, but does not restrict the interconnecting molecules that can be used according to the present invention to the examples displayed herein.

The term "*adjuvant*" as used herein refers to an immunological adjuvant i.e. a material used in a vaccine composition that modifies or augments the effects of said vaccine by enhancing the immune response to a given antigen contained in the vaccine without being antigenically related to it. For the person skilled in the art, classically recognized examples of adjuvants include:
- mineral-containing compositions, including calcium salts and aluminium salts (or mixtures thereof). Calcium salts include calcium phosphate. Aluminium salts include hydroxides, phosphates, sulfates, etc., with the salts taking any suitable form (e.g. gel, crystalline, amorphous, etc.). Adsorption to these salts is preferred. The mineral containing compositions may also be formulated as a particle of metal salt. The adjuvants known as aluminium hydroxide and aluminium phosphate may be also used. The invention can use any of the "hydroxide" or "phosphate" adjuvants that are in general used as adjuvants. The adjuvants known as "aluminium hydroxide" are typically aluminium oxyhydroxide salts, which are usually at least partially crystalline. The adjuvants known as "aluminium phosphate" are typically aluminium hydroxyphosphates, often also containing a small amount of sulfate (i. e. aluminium hydroxyphosphate sulfate). They may be obtained by precipitation, and the reaction conditions and concentrations during precipitation influence the degree of substitution of phosphate for hydroxyl in the salt. Mixtures of both an aluminium hydroxide and an aluminium phosphate can be employed in the formulation according to the present invention;
- saponins, which are a heterologous group of sterol glycosides and triterpenoid glycosides that are found in the bark, leaves, stems, roots and even flowers of a wide range of plant species. Saponins from the bark of the *Quillaia saponaria,* Molina tree have been widely studied as adjuvants. Saponins can also be commercially obtained from Smilax ornata (sarsaprilla), Gypsophilla paniculata (brides veil), and Saponaria oficianalis (soap root). Saponin adjuvant formulations include purified formulations, such as QS21, as well as lipid formulations, such as ISCOMs. Saponin compositions have been purified using HPLC and RP-HPLC. Specific purified fractions using these techniques have been identified, including QS7, QS 17, QS 18, QS2 1, QH-A, QH-B and QH-C. Saponin formulations may also comprise a sterol, such as cholesterol. Combinations of saponins and cholesterols can be used to form unique particles called immunostimulating complexes (ISCOMs). ISCOMs generally include a phospholipid such as phosphatidylethanolamine or phosphatidylcholine. Any known saponin can be used in ISCOMs. Preferably, the ISCOM includes one or more of QuilA, QHA & QHC;

- microparticles (i.e. a particle of 100 nm to 150 pm in diameter, more preferably 200 nm to 30 pm in diameter, or 500 nm to 10 pm in diameter) formed from materials that are biodegradable and non-toxic. Such non-toxic and biodegradable materials include, but are not restricted to poly(α-hydroxy acid), polyhydroxybutyric acid, polyorthoester, polyanhydride, polycaprolactone;
- CD1d ligands, such as an α-glycosylceramide, phytosphingosine-containing α-glycosylceramides, OCH, KRN7000 [(2*S*,3*S*,4*R*)-1-*O*-(α-D-galactopyranosyl)-2-(*N-*hexacosanoylamino)-1,3,4-octadecanetriol], CRONY- 101, 3"-sulfo-galactosyl-ceram ide;
- immunostimulatory oligonucleotides, such CpG motif containing ones (a dinucleotide sequence containing an unmethylated cytosine residue linked by a phosphate bond to a guanosine residue), or Cpl motif containing ones (a dinucleotide sequence containing cytosine linked to inosine), or a double-stranded RNA, or an oligonucleotide containing a palindromic sequence, or an oligonucleotide containing a poly(dG) sequence. Immunostimulatory oligonucleotides can include nucleotide modifications/analogs such as phosphorothioate modifications and can be double-stranded or (except for RNA) single-stranded;
- compounds containing lipids linked to a phosphate-containing acyclic backbone, such as the TLR4 antagonist E5564;
- oil emulsions (e.g. Freund's adjuvant).

Theoretically, each molecule or substance that is able to favor or amplify a particular situation in the cascade of immunological events, ultimately leading to a more pronounced immunological response, can be defined as an adjuvant.

In principle, through the use of adjuvants in vaccine formulations, one can
- direct and optimize immune responses that are appropriate or desirable for the vaccine;
- enable mucosal delivery of vaccines, i.e. administration that results in contact of the vaccine with a mucosal surface such as buccal or gastric or lung epithelium and the associated lymphoid tissue;
- promote cell-mediated immune responses;
- enhance the immunogenicity of weaker immunogens, such as highly purified or recombinant antigens;
- reduce the amount of antigen or the frequency of immunization required to provide protective immunity; and
- improve the efficacy of vaccines in individuals with reduced or weakened immune responses, such as newborns, the aged, and immunocompromised vaccine recipients.

Although little is known about their mode of action, it is currently believed that adjuvants augment immune responses by one of the following mechanisms:
- increasing the biological or immunologic half-life of antigens;
- improving antigen delivery to antigen-presenting cells (APCs), as well as antigen processing and presentation by the APCs e.g., by enabling antigen to cross endosomal membranes into the cytosol after ingestion of antigen-adjuvant complexes by APC;
- mimicking danger inducing signals from stressed or damaged cells, which serve to initiate an immune response;
- inducing the production of immunomodulatory cytokines;
- biasing the immune response towards a specific subset of the immune system; and
- blocking the rapid dispersal of the antigen challenge.

Saccharides are known by the person skilled in the art as TI-2 (T cell independent-2) antigens and poor immunogens. Therefore, to produce a saccharide-based vaccine, said saccharide is conjugated to an immunogenic carrier to provide a conjugate, which presents an increased immunogenicity in comparison with the saccharide. In this context the term "immunogenic carrier" is defined as a structure, which is conjugated to the saccharide to form a conjugate that presents an increased immunogenicity in comparison with the saccharide *per se*. Thus, the conjugation of the saccharides to the immunogenic carrier has as effect the stimulation of the immune response against said saccharide, without inducing an immune response against the said immunogenic carrier.

Surprisingly, it was found that a pure saccharide of general formula (**I**) according to the present invention contains a protective immunogenic glycan epitope and is able to induce a protective immune response against *Francisella tularensis* bacteria in a human and/or animal host. The saccharide of general formula (**I**) elicits antibodies that are cross-reacting with the *F. tularensis* strain 15 O-antigen CPS, recognize specifically *F. tularensis* bacteria of type A and type B.

Thus, the present invention relates to a saccharide of general formula (**I**)

**H-Uₓ₊₁-Uₓ-T-O-L-NH₂** (**I**)

wherein
x is an integer selected from 1, 2, 3, and 4; 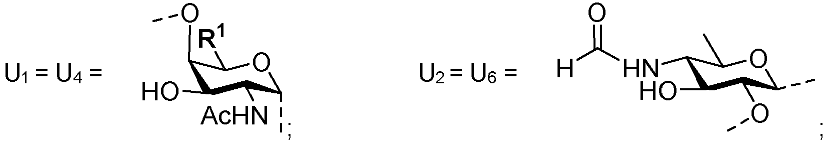 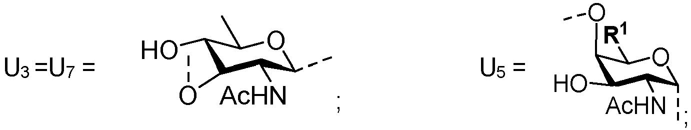 -T- represents a bond, -Uₓ₊₃-, -Uₓ₊₃-Uₓ₊₂-, -Uₓ₊₃-Uₓ₊₂-Uₓ₊₁-, or -[Uₓ₊₃-Uₓ₊₂-Uₓ₊₁-Uₓ]ₙ- with n being an integer selected from 1, 2 and 3;
R¹ represents -CH₂OH or -CONH₂;
L represents a linker; or a pharmaceutically acceptable salt thereof
-L- is defined as a linker and is part of the fragment -O-L-NH₂. Thus, the linker -L- is bound to an oxygen atom and to the nitrogen atom of the NH₂-group. It is preferred that at least two carbon atoms of the linker are between the oxygen atom and the NH₂-group, like -O-C-C-NH₂. The linker -L- can be an aliphatic chain, wherein the aliphatic chain can optionally include an aromatic chain inserted in it, or a number of heteroatoms oscillating from 0 to 10.

The linker L preferably contains between 2 and 40 carbon atoms (including the carbon atoms of optional side chains), more preferably between 2 and 30, more preferably between 2 and 20, more preferably between 2 and 14, more preferably between 2 and 12, and still more preferably between 2 and 10 carbon atoms.

The shortest atom chain between the oxygen atom (i.e. the oxygen of -O-L-NH₂) and the NH₂-group consists preferably of 2 to 14 atoms, more preferably of 2 to 12 atoms, more preferably of 2 to 10 atoms, more preferably of 2 to 8 atoms. In case the shortest chain (which is the shortest possible connection between the oxygen at the anomeric center and the NH₂-group) consists of 2 to 6 atoms, these are preferably carbon atoms. In case the shortest chain consists of 4 to 8 atoms, the chain may contain 1, 2 or 3 heteroatoms selected from O, N and S. In case the shortest chain consists of 9 to 14 atoms, the chain may contain 1, 2, 3, 4, 5, or 6 heteroatoms selected from O, N and S.

It is also preferred that the linker -L-, or the shortest chain is fully or partially fluorinated. The linker -L- may contain a 3-membered or a 4-membered or a 5-membered or a 6-membered saturated carbocycle or a 5-membered partly unsaturated (and not aromatic) carbocycle or a 4-membered or a 5-membered or a 6-membered saturated oxygen heterocycle or a 4-membered or a 5-membered or a 6-membered saturated nitrogen heterocycle or a 6-membered aromatic carbocycle.

The linker -L- may also contain amide (-NH-CO-, -CO-NH-) and/or urea (-NH-CO-NH-) residues and preferably only one amide or urea residue. The linker may also contain substituents and preferably two substituents, such as R¹⁰ and R¹¹, or four substituents such as R¹⁰, R¹¹, R¹⁵ and R¹⁴, which have the meanings as defined herein and which are preferably selected from: -F, -Cl, -CH₃, -C₂H₅, -C₃H₇, -C₅H₉, -C₆H₁₃, -OCH₃, -OC₂H₅, -CH₂F, -CHF₂, -CF₃, -C(O)-NH₂, -SCH₃, -SC₂H₅, -NHC(O)CH₃, -N(CH₃)₂, and -N(C₂H₅)₂;

In case the linker -L- is fluorinated, more than two substituents -F are preferred. A perfluorination (i.e. a complete fluorination of the linker, especially of the alkyl linker) is also preferred.

Preferably the linker -L- is selected from: -CH₂-, -(CH₂)₂-, -(CH₂)₃-, -(CH₂)₄-, -(CH₂)₅-, -(CH₂)₆-, -(CH₂)₇-, -(CH₂)₈-, -(CH₂)₉-, -(CH₂)₁₀-, -CF₂-, -(CF₂)₂-, -(CF₂)₃-, -(CF₂)₄-, -(CF₂)₅-₇ -(CF₂)₆-, -(CF₂)₇-, -(CF₂)₈-, -(CF₂)₉-, -(CF₂)₁₀-, -(CH₂)₂-O-(CH₂)₂-, -CH₂-O-(CH₂)₃-, -(CH₂)₃-O-CH₂-, -CH₂-O-(CH₂)₂-, -(CH₂)₂-O-CH₂-, -(CH₂)₃-O-(CH₂)₂-, -(CH₂)₂-O-(CH₂)₃-, -(CH₂)₄-O-CH₂-, -CH₂-O-(CH₂)₄-, -L^{a}-, -L^{a}-L^{e}-, -L^{a}-L^{b}-L^{e}-, -L^{a}-L^{b}-L^{d}-L^{c}-L^{e}-, -L^{a}-L^{d}-L^{e}-;
wherein
-L^{a}- is selected from: -(CH₂)ₒ-, -(CF₂)ₒ-, -(CH₂-CH₂-O)ₒ-C₂H₄-, -(CH₂-CH₂-O)ₒ-CH₂-, -(CR¹⁰R¹¹)ₒ-, 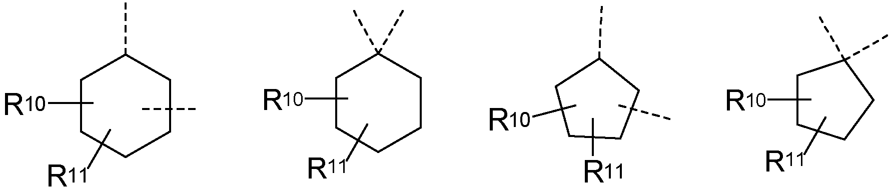 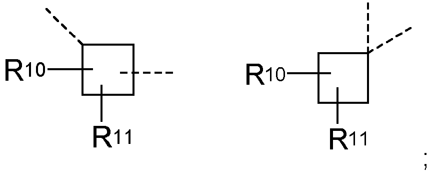
-L^{b}- and -L°- are independently of each other selected from: -O-, -NH-C(O)-NH-, -NH-C(S)-NH-, -NH-C(O)-, -C(O)-NH-, -NH-C(O)-O-, -NR⁹-, -NR¹⁸-, -SO₂-, 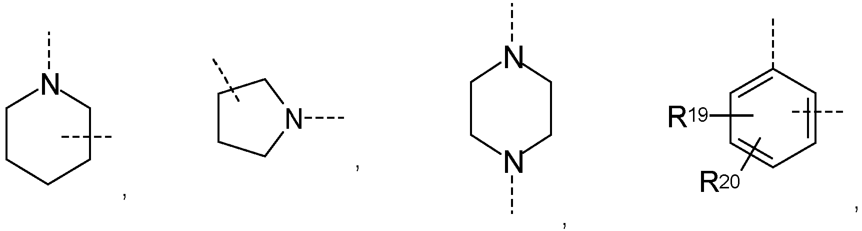 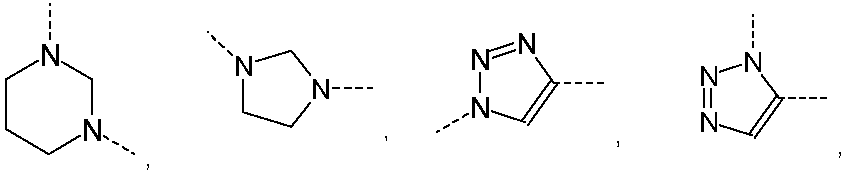 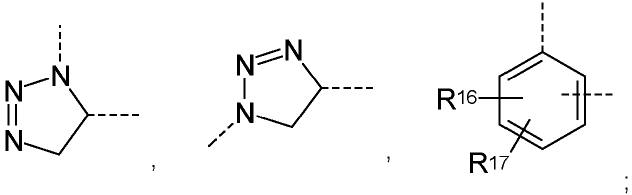
-L^{d}- represents -(CH₂)_{q}-, -(CF₂)_{q}-, -(CR¹²R¹³)_{q}-, -(CH₂-CH₂-O)_{q}-C₂H₄-, -(CH₂-CH₂-O)_{q}-CH₂-, 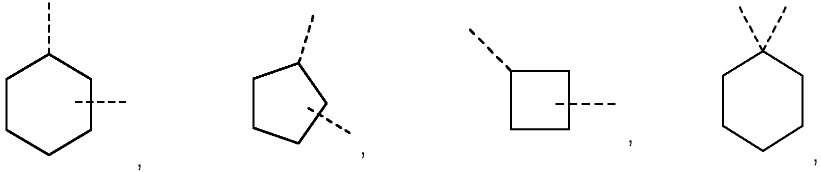 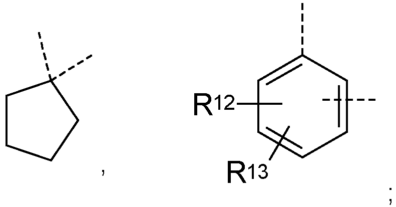
-L^{e}- is selected from: -(CH₂)ₚ₁-, -(CF₂)ₚ₁-, -C₂H₄-(O-CH₂-CH₂)ₚ₁-, -CH₂-(O-CH₂-CH₂)ₚ₁-, -(CH₂)ₚ₁-O-(CH₂)ₚ₂-, -(CR¹⁴R^{1S})ₚ₁-, -(CR¹⁴R¹⁵)ₚ₁-O-(CR²¹R²²)ₚ₂-, 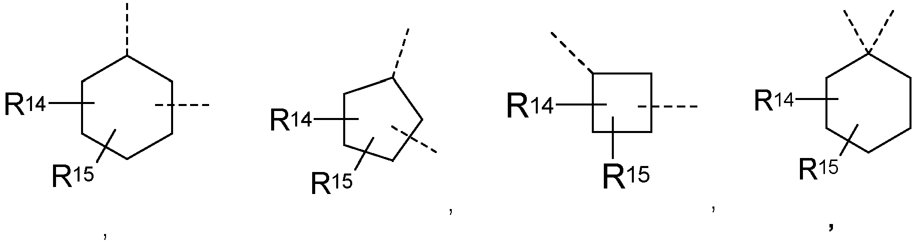 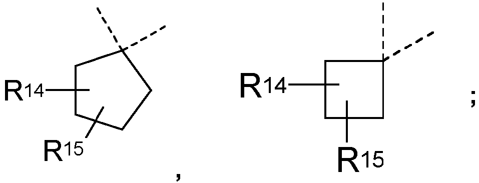
R⁹ and R¹⁸ are independently of each other selected from: -CH₃, -C₂H₅, -C₃H₇, and -C(O)CH₃;
R¹⁰, R¹¹, R¹², R¹³, R¹⁴, R¹⁵, R¹⁶, R¹⁷, R¹⁹, R²⁰, R²¹ and R²² are independently of each other selected from: -H, -F, -Cl, -CH₃, -C₂H₅, -C₃H₇, -C₅H₉, -C₆H₁₃, -OCH₃, -OC₂H₅, -CH₂F, -CHF₂, -CF₃, -C(O)-NH₂, -SCH₃, -SC₂H₅, -NHC(O)CH₃, -N(CH₃)₂ and -N(C₂H₅)₂;
o, q, p1 and p2 are independently of each other an integer selected from 1, 2, 3, 4, 5, 6, 7, 8, 9, and 10.

Preferably, the linker -L- is selected from: -L^{a}-, -L^{a}-L^{e}-, -L^{a}-L^{b}-L^{e}- and -L^{a}-L^{d}-L^{e}-;
-L^{a}- represents -(CH₂)ₒ-, -(CH₂-CH₂-O)ₒ-C₂H₄-, -(CH₂-CH₂-O)ₒ-CH₂-;
-L^{b}- represents -O-; -NH-CO-NH-, -NH-CO-CH₂-NH-, -NH-CO-;
-L^{d}- represents -(CH₂)_{q}-, -(CF₂)_{q}-, -(CH₂-CH₂-O)_{q}-C₂H₄-, or -(CH₂-CH₂-O)_{q}-CH₂-;
-L^{e}- represents -(CH₂)ₚ₁-, -(CF₂)ₚ₁-, -C₂H₄-(O-CH₂-CH₂)ₚ₁-, -CH₂-(O-CH₂-CH₂)ₚ₁- or -(CH₂)ₚ₁-O-(CH₂)ₚ₂-;
o, q, p1 and p2 are independently of each other an integer selected from 1, 2, 3, 4, 5, and 6.

Preferably, the linker -L- represents -(CH₂)ₒ- and o is an integer selected from 1, 2, 3, 4, 5, 6, 7 and 8.

The saccharides of the present invention bear basic and/or acidic substituents and they may form salts with organic or inorganic acids or bases.

Examples of suitable acids for such acid addition salt formation are hydrochloric acid, hydrobromic acid, sulfuric acid, phosphoric acid, acetic acid, citric acid, oxalic acid, malonic acid, salicylic acid, p-aminosalicylic acid, malic acid, fumaric acid, succinic acid, ascorbic acid, maleic acid, sulfonic acid, phosphonic acid, perchloric acid, nitric acid, formic acid, propionic acid, gluconic acid, lactic acid, tartaric acid, hydroxymaleic acid, pyruvic acid, phenylacetic acid, benzoic acid, p-aminobenzoic acid, *p*-hydroxybenzoic acid, methanesulfonic acid, ethanesulfonic acid, nitrous acid, hydroxyethanesulfonic acid, ethylenesulfonic acid, *p*-toluenesulfonic acid, naphthylsulfonic acid, sulfanilic acid, camphorsulfonic acid, china acid, mandelic acid, o-methylmandelic acid, hydrogen-benzenesulfonic acid, picric acid, adipic acid, d-o-tolyltartaric acid, tartronic acid, (*o*, *m*, *p*)-toluic acid, naphthylamine sulfonic acid, and other mineral or carboxylic acids well known to those skilled in the art. The salts are prepared by contacting the free base form with a sufficient amount of the desired acid to produce a salt in the conventional manner.

Examples of suitable inorganic or organic bases are, for example, NaOH, KOH, NH₄OH, tetraalkylammonium hydroxide, lysine or arginine and the like. Salts may be prepared in a conventional manner using methods well known in the art, for example by treatment of a solution of the compound of the general formula (**I**) with a solution of a base, selected out of the group mentioned above.

It is clear for the skilled person in the art of carbohydrate chemistry that the saccharides of general (**I**) are not containing -O-O- bonds and or sugar fragments (Uₓ, Uₓ₊₁, Uₓ₊₂, Uₓ₊₃) connected or bound to each other *via* their anomeric or C-1 carbons. It is also clear for the person skilled in the art that the stereochemistry of the glycosidic bond is the stereochemistry indicated for the anomeric center of the sugar fragment in the general formula. Hence, the stereochemistry of the anomeric center for sugar fragment U₁ and U₄ is α, for sugar fragment U₂ and U₆ is β, for sugar fragment U₃ and U₇ is β and for sugar fragment U₅ is α.

The saccharide of general formula (**I**) contains a protective immunogenic epitope and is able to induce a protective immune response against *F. tularensis* bacteria in a human and/or animal host. The saccharide of general formula (**I**) elicits antibodies that are cross-reacting with the *F. tularensis* strain 15 O-antigen capsular polysaccharide, recognize specifically *F. tularensis* bacteria of type A and type B. Additionally, the inventive saccharides have the advantage that these are pure synthesized compounds, which can be easily manufactured in accordance with GMP regulations.

Thus, the vaccine composition of the present invention contains most preferably only one single compound of the general formula (**I**) bound to an immunogenic carrier, preferably a carrier protein and more preferably CRM₁₉₇. Thus, the compound of the general formula (**I**) is useful for the preparation of well defined, well characterized and pure vaccines containing only one synthetically prepared and well characterized tri-, tetra-, penta-, hexa-, hepta- or octasaccharide preferably linked to an immunogenic carrier, preferably a carrier protein and more preferably CRM₁₉₇. Consequently, the vaccines of the present invention contain only one synthetically synthesized compound of general formulae (**I**) preferably linked to an immunogenic carrier, preferably a carrier protein and more preferably CRM₁₉₇.

Preferred is a saccharide of general formula (**I**)

**H-Uₓ₊₁-Uₓ-T-O-L-NH₂** (**I**)

wherein
x is an integer selected from 1 and 2; 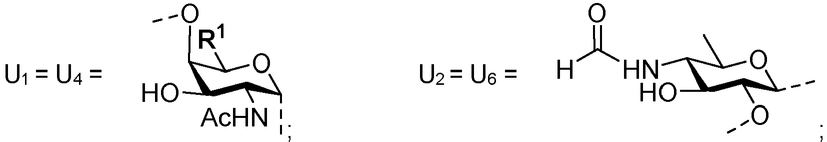 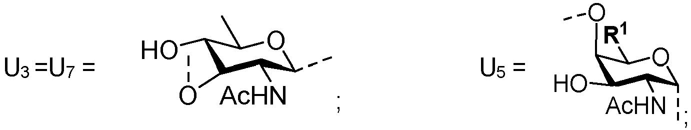
-T- represents a bond, -Uₓ₊₃-, -Uₓ₊₃-Uₓ₊₂-, -Uₓ₊₃-Uₓ₊₂-Uₓ₊₁-, or -[Uₓ₊₃-Uₓ₊₂-Uₓ₊₁-Uₓ]ₙ- with n being an integer selected from 1, 2 and 3;
R¹ represents -CH₂OH or -CONH₂;
L represents a linker; or a pharmaceutically acceptable salt thereof.

Preferred is a saccharide of general formula (I), wherein x represents 1 and a pharmaceutically acceptable salt thereof. Hence, a saccharide of general formula (**I-a**) 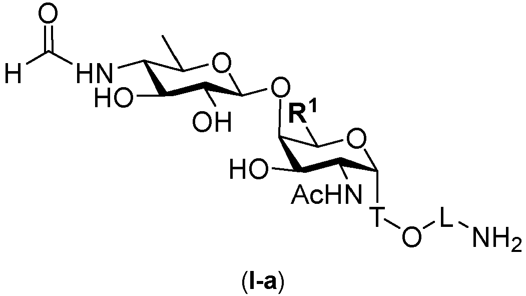 with L, T, and R¹ having the meaning defined herein, or a pharmaceutically acceptable salt thereof is particularly preferred.

Also preferred is a saccharide of general formula (**I**), wherein x represents 2, and a pharmaceutically acceptable salt thereof. Thus, a saccharide of general formula (**I-b**), 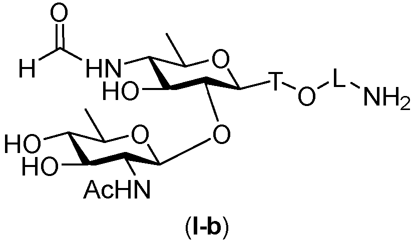 with L, T, and R¹ having the meaning defined herein, or a pharmaceutically acceptable salt thereof is also preferred.

Also preferred is a saccharide of general formula (**I**), wherein x represents 3, and a pharmaceutically acceptable salt thereof. Thus, a saccharide of general formula (**I-c**)**,** 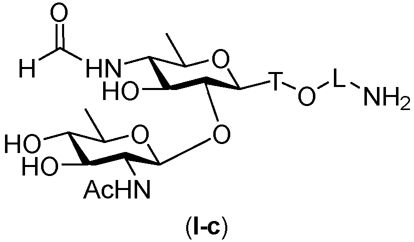 with L, T, and R¹ having the meaning defined herein, or a pharmaceutically acceptable salt thereof is also preferred.

Also preferred is a saccharide of general formula (**I**), wherein x represents 4, and a pharmaceutically acceptable salt thereof. Thus, a saccharide of general formula (**I-d**), 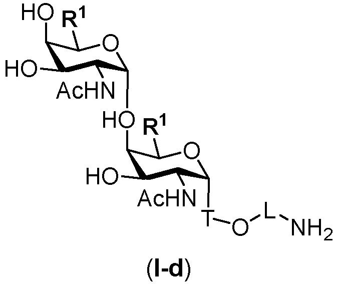 with L, T, and R¹ having the meaning defined herein, or a pharmaceutically acceptable salt thereof is also preferred.

Preferably, T represents a bond, -Uₓ₊₃-, -Uₓ₊₃-Uₓ₊₂-, or -Uₓ₊₃-Uₓ₊₂-Uₓ₊₁-. Thus, a saccharide of general formula (**I**), (**I-a**), (**I-b**), (**I-c**), or (**I-d**), wherein T represents a bond, -Uₓ₊₃-, -Uₓ₊₃-Uₓ₊₂-, or -Uₓ₊₃-Uₓ₊₂-Uₓ₊₁- is preferred.

Particularly preferred is a saccharide of general formula (**I**), wherein T represents -Uₓ₊₃-, and a pharmaceutically acceptable salt thereof. Thus, a saccharide of general formulae (**II-a**), (**II-b**), (**II-c**) and (**II-d**)**,** 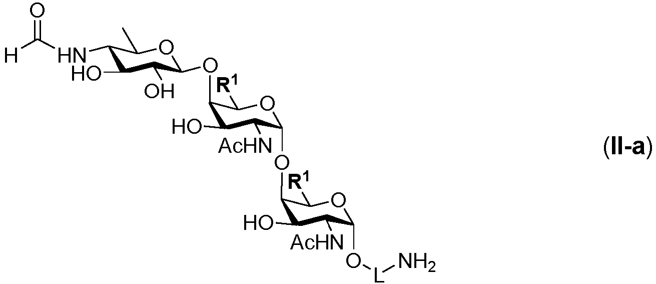 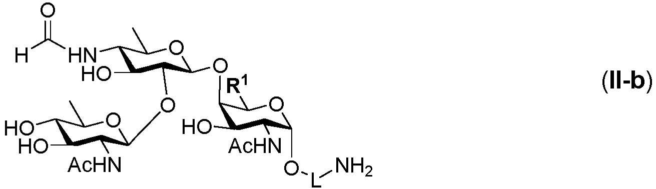 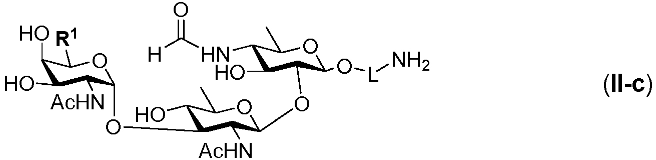 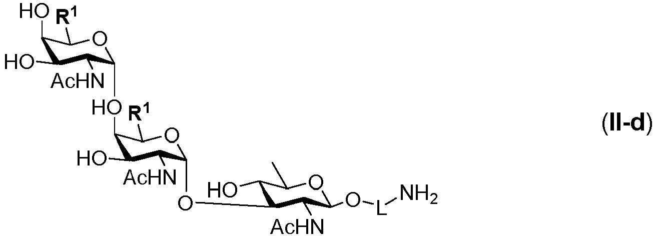 with L and R¹ having the meaning defined herein, or a pharmaceutically acceptable salt thereof is also preferred.

Also preferred is a saccharide of general formula (**I**), wherein T represents -Uₓ₊₃-Uₓ₊₂-, and a pharmaceutically acceptable salt thereof. Thus, a saccharide of general formulae (**III-a**), (**III-b**), (**III-c**) and (**III-d**), 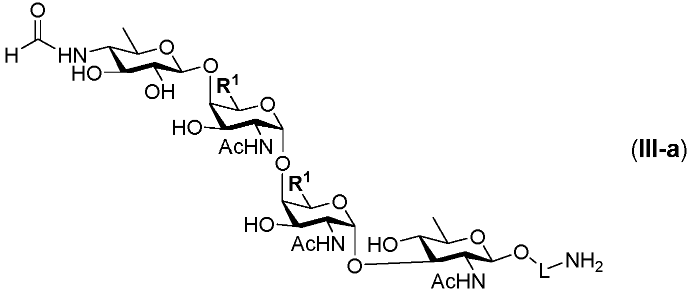 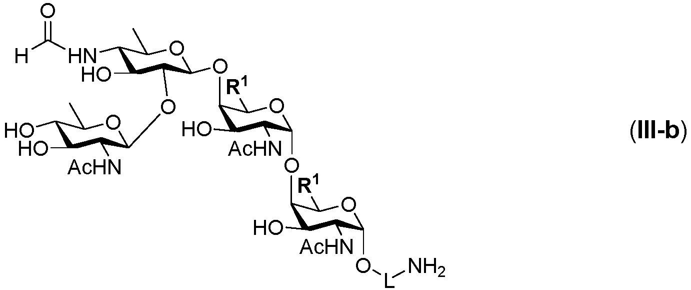 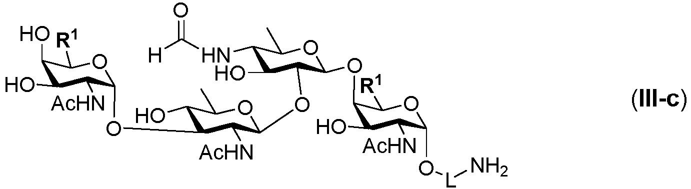 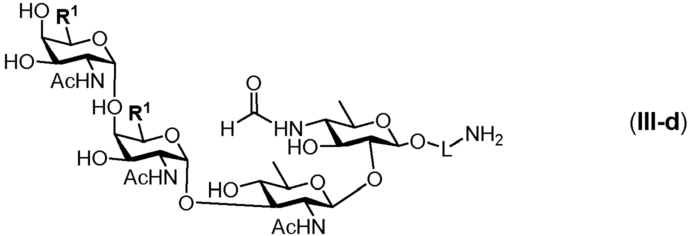 with L and R¹ having the meaning defined herein, or a pharmaceutically acceptable salt thereof is also preferred.

Also preferred is a saccharide of general formula (I), wherein T represents -a bond, and a pharmaceutically acceptable salt thereof. Thus, a saccharide of general formulae (**IV-a**), (**IV-b**), (**IV-c**) and (**IV-d**), 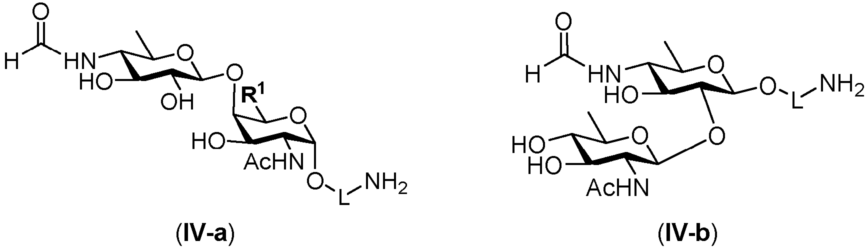 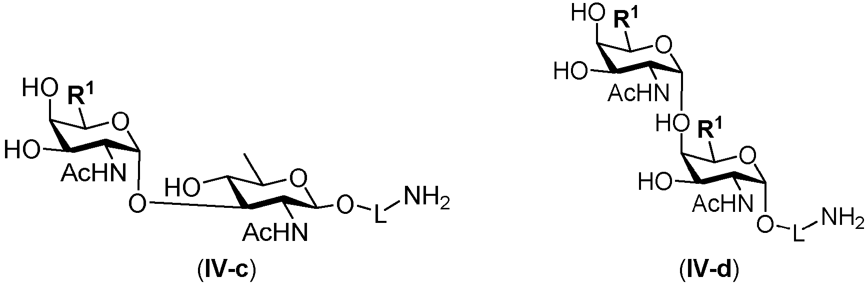 with L and R¹ having the meaning defined herein, or a pharmaceutically acceptable salt thereof is also preferred.

Also, preferred is a saccharide of general formula (V),

**H-Uₓ₊₁-Uₓ-[Uₓ₊₃-Uₓ₊₂-Uₓ₊₁-Uₓ]ₙ-O-L-NH₂** (**V**)

wherein Uₓ, Uₓ₊₁, Uₓ₊₂, Uₓ₊₃, x, L, and n have the meanings as defined herein.

Particularly preferred is a saccharide of general formula (**V**), wherein n represents 1. In a preferred embodiment, R¹ represents -CH₂OH, Thus, a saccharide of general formula (**I**), (**I-a**), (**I-b**), (**I-c**), (**I-d**), (**II-a**)**,** (**II-b**), (**II-c**), (**II-d**), (**III-a**), (**III-b**), (**III-c**), (**III-d**), (**IV-a**), (**IV-b**), (**IV-c**), (**IV-d**), and (**V**) wherein R¹ represents -CH₂OH is also preferred.

In a preferred embodiment, R¹ represents -CONH₂, Thus, a saccharide of general formula (**I**), (**I-a**), (**I-b**), (**I-c**), (**I-d**), (**II-a**)**,** (**II-b**), (**II-c**), (**II-d**), (**III-a**), (**III-b**), (**III-c**), (**III-d**), (**IV-a**), (**IV-b**), (**IV-c**), (**IV-d**), and (**V**) wherein R¹ represents -CH₂OH is also preferred.

Preferably the linker**-L-** represents **-L^{a}-, -L^{a}-L^{e}-, -L^{a}-L^{b}-L^{e}-,** or **-L^{a}-L^{d}-L^{e}-;**
**-L^{a}-** represents -(CH₂)ₒ-, -(CH₂CH₂O)ₒ-C₂H₄-, or -(CH₂-CH₂-O)ₒ-CH₂;
**-L^{b}-** represents -O-, -NH-CO-NH-, -NH-CO-CH₂-NH-, -NH-CO-;
**-L^{d}-** represents -(CH₂)_{q}-, -(CH(OH))_{q}-, -(CF₂)_{q}-, -(CH₂-CH₂-O)_{q}-C₂H₄-, or -(CH₂-CH₂-O)_{q}-CH₂- ;
**-L^{e}-** represents -(CH₂)ₚ₁-, -(CF₂)ₚ₁-, -C₂H₄-(O-CH₂-CH₂)ₚ₁-, -CH₂-(O-CH₂-CH₂)ₚ₁- or -(CH₂)ₚ₁-O-(CH₂)ₚ₂- ;
and o, q, p1 and p2 are independently of each other an integer selected from 1, 2, 3, 4, 5, and 6.

Therefore, a saccharide of general formula (**I**), (**I-a**), (**I-b**), (**I-c**), (**I-d**), (**II-a**), (**II-b**), (**II-c**), (**II-d**), (**III-a**), (**III-b**), (**III-c**), (**III-d**), (**IV-a**), (**IV-b**), (**IV-c**), (**IV-d**), or (**V**), wherein
-L- represents -L^{a}-, -L^{a}-L^{e}-, -L^{a}-L^{b}-L^{e}-, or -L^{a}-L^{d}-L^{e}- ;
-L^{a}- represents -(CH₂)ₒ-, -(CH₂-CH₂-O)ₒ-C₂H₄-, or -(CH₂-CH₂-O)ₒ-CH₂;
-L^{b}- represents -O-, -NH-CO-NH-, -NH-CO-CH₂-NH-, -NH-CO-;
-L^{d}- represents -(CH₂)_{q}-, -(CH(OH))_{q}-, -(CF₂)_{q}-, -(CH₂-CH₂-O)_{q}-C₂H₄-, or -(CH₂-CH₂-O)_{q}-CH₂- ;
-L^{e}- represents -(CH₂)ₚ₁-, -(CF₂)ₚ₁-, -C₂H₄-(O-CH₂-CH₂)ₚ₁-, -CH₂-(O-CH₂-CH₂)ₚ₁- or -(CH₂)ₚ₁-O-(CH₂)ₚ₂- ;
and o, q, p1 and p2 are independently of each other an integer selected from 1, 2, 3, 4, 5, and 6 is especially preferred.

It is particularly preferred that -L- represents -(CH₂)ₒ- and o is an integer selected from 2, 3, 4, 5, 6, 7 and 8. Thus, a particularly preferred saccharide is a saccharide of general formula (**I**), (**I-a**), (**I-b**), (**I-c**), (**I-d**), (**II-a**), (**II-b**), (**II-c**), (**II-d**), (**III-a**), (**III-b**), (**III-c**), (**III-d**), (**IV-a**), (**IV-b**), (**IV-c**), (**IV-d**), or (**V**), wherein-L- represents -(CH₂)ₒ- and o is an integer selected from 2, 3, 4, 5, 6, 7 and 8.

Preferably, the inventive saccharide is selected from: 4-amino butyl 4-formamido-*β*-D-quinovopyranosyl-(1→4)-*α*-*N*-acetylgalactopyranoside; 5-amino pentyl 4-formamido-*β*-D-quinovopyranosyl-(1→4)-*α*-D-*N*-acetylgalactopyranoside; 6-amino hexyl 4-formamido-*β*-D-quinovopyranosyl-(1→4)-*α*-D-*N*-acetylgalactopyranoside; 7-amino heptyl 4-formamido-*β*-D-quinovopyranosyl-(1→4)-*α*-D-*N*-acetylgalacto-pyranoside; 4-amino butyl *β*-D-*N*-acetylquinovopyranoside-(1→2)-4-formamido-*β*-D-quinovopyranoside; 5-amino pentyl *β*-D-*N*-acetylquinovopyranoside-(1→2)-4-formamido-*β*-D-quinovopyranoside; 6-amino hexyl *β*-D-*N*-acetyl-quinovosamine-pyranoside-(1→2)-4-formamido-*β*-D-quinovopyranoside; 7-amino pentyl *β*-D-N-acetylquinovosaminepyranoside-(1→2)-4-formamido-*β*-D-quinovo-pyranoside.

### Conjugate

Another aspect of the present invention is directed to a conjugate comprising a synthetic saccharide of general formula (**I**) covalently bound or covalently linked to an immunogenic carrier through the nitrogen atom of the -O-L-NH₂ group. In other words, another aspect of the present invention is directed to a saccharide of any of the general formulae (**I**), (**I-a**), (**I-b**), (**I-c**), (**I-d**), (**II-a**), (**II-b**), (**II-c**), (**II-d**), (**III-a**), (**III-b**), (**III-c**), (**III-d**), (**IV-a**), (**IV-b**), (**IV-c**), (**IV-d**), or **(V)** conjugated with an immunogenic carrier through the nitrogen atom of the -O-L-NH₂ group. The inventive conjugate comprising a synthetic saccharide of the general formula (**I**), (**I-a**), (**I-b**), (**I-c**), (**I-d**), (**II-a**), (**II-b**), (**II-c**), (**II-d**), (**III-a**), (**III-b**), (**III-c**), (**III-d**), (**IV-a**), (**IV-b**), (**IV-c**), (**IV-d**), or (**V**) covalently bound or covalently linked to an immunogenic carrier through the nitrogen atom of the -O-L-NH₂ group is also defined as a conjugate obtained by reacting a saccharide of any of the general formulae (**I**), (**I-a**), (**I-b**), (**I-c**), (**I-d**), (**II-a**), (**II-b**), (**II-c**), (**II-d**), (**III-a**), (**III-b**), (**III-c**), (**III-d**), (**IV-a**), (**IV-b**), (**IV-c**), (**IV-d**), or **(V)** with an immunogenic carrier. Said conjugate proved to be efficient as a vaccine for immunization against diseases associated with *Francisella tularensis* bacteria.

Saccharides are known by the person skilled in the art as generally TI-2 (T cell independent-2) antigens and poor immunogens. TI-2 antigens are antigens, which are recognized only by mature B cells through the cross linking of surface exposed immunoglobulin receptors. Without T cell help, no immunological memory is generated and neither isotype switching from IgM to other IgG subclasses, nor B cells affinity maturation occurs. Moreover, saccharides are known as poor immunogens in humans due to the structural homology to human glycolipids and glycoproteins. Due to their poor immunogenic properties, saccharides manifest poor ability to produce both antibody production by B cells, as well as the formation of memory cells, features which are essential for the production of potent vaccines.

Therefore, to produce a potent saccharide-based vaccine, a saccharide of general formula (**I**), (**I-a**), (**I-b**), (**I-c**), (**I-d**), (**II-a**), (**II-b**), (**II-c**), (**II-d**), (**III-a**), (**III-b**), (**III-c**), (**III-d**), (**IV-a**), (**IV-b**), (**IV-c**), (**IV-d**), or (**V**) is conjugated to an immunogenic carrier to provide a conjugate presenting increased immunogenicity in comparison with the saccharide.

Said conjugate consists of at least one synthetic saccharide of the general (**I**), (**I-a**), (**I-b**), (**I-c**), (**I-d**), (**II-a**), (**II-b**), (**II-c**), (**II-d**), (**III-a**), (**III-b**), (**III-c**), (**III-d**), (**IV-a**), (**IV-b**), (**IV-c**), (**IV-d**), or (**V**) and an immunogenic carrier to which the at least one saccharide of the general formula (**I**), (**I-a**), (**I-b**), (**I-c**), (**I-d**), (**II-a**), (**II-b**), (**II-c**), (**II-d**), (**III-a**), (**III-b**), (**III-c**), (**III-d**), (**IV-a**), (**IV-b**), (**IV-c**), (**IV-d**), or (**V**) is covalently bound.

Surprisingly, it was found that immunization with a conjugate according to the present invention results in the production of high titers of antibodies specific to the carbohydrate part of the saccharide according to the present invention. Said antibodies are cross-reacting with the natural *Francisella tularensis* O-antigen capsular polysaccharide, thus conferring protection against *Francisella tularensis* bacteria of type A and type B.

In this context the term "immunogenic carrier" is defined as a structure, which is conjugated to the saccharide to form a conjugate that presents an increased immunogenicity in comparison with the saccharide *per se*. Thus, the conjugation of a saccharide of the general formula (**I**), (**I-a**), (**I-b**), (**I-c**), (**I-d**), (**II-a**), (**II-b**), (**II-c**), (**II-d**), (**III-a**), (**III-b**), (**III-c**), (**III-d**), (**VI-a**), (**VI-b**), (**VI-c**), (**VI-d**), or (**V**) to the immunogenic carrier has as effect the stimulation of the immune response against the saccharide of the general formula (**I**), (**I-a**), (**I-b**), (**I-c**), (**I-d**), (**II-a**), (**II-b**), (**II-c**), (**II-d**), (**III-a**), (**III-b**), (**III-c**), (**III-d**), (**IV-a**), (**IV-b**), (**IV-c**), (**IV-d**), or (**V**) without inducing an immune response against the said immunogenic carrier.

Preferred immunogenic carriers are carrier proteins or glycosphingolipids with immunomodulatory properties. For the person skilled in the art, a carrier protein is a protein selected from the group comprising or consisting of: a diphtheria toxoid, a mutated diphtheria toxoid, a modified diphtheria toxoid, a mutated and modified diphtheria toxoid, a tetanus toxoid, a modified tetanus toxoid, a mutated tetanus toxoid, outer membrane protein (OMP), bovine serum albumin (BSA), keyhole limpet hemocyanine (KLH), cholera toxoid (CT) and protein D (a non-typeable *Haemophilus influenzae* protein).

The term "toxoid" as used herein refers to a bacterial toxin (usually an exotoxin), whose toxicity has been inactivated or suppressed either by chemical (formalin) or heat treatment, while other properties, typically immunogenicity, are maintained. A mutated toxoid as used herein is a recombinant bacterial toxin, which has been amended to be less toxic or even non-toxic by amending the wild-type amino acid sequence. Such a mutation could be a substitution of one or more amino acids. Such a mutated toxoid presents on its surface a functionality that can react with the functional group Y of the interconnecting molecule to provide a modified toxoid. Said functionality is known to the person skilled in the art and includes but is not restricted to the primary amino functionality of a lysine residue that can react with activated esters, an isocyanate group or an aldehyde in presence of a reducing agent, to the carboxylate functionality of a glutamate or aspartate residue that can be activated by carbodiimides or to the thiol functionality of a cysteine residue.

Activated esters include, but are not restricted to N-(γ-maleimidobutyryloxy) sulfosuccinimide ester (sulfo-GMBS), succinimidyl (4-iodoacetyl) aminobenzoate (sulfo-SIAB), succinimidyl-3-(bromoacetamido)propionate (SBAP), disuccinimidyl glutarate (DSG), disuccinimidyl adipate (DSA), 2-pyridyldithiol-tetraoxatetradecane-N-hydroxysuccinimide (PEG-4-SPDP), bis-(4-nitrophenyl) adipate and bis-(4-nitrophenyl) succinate (see **Figure 2**). Preferred activated esters are disuccinimidyl adipate (DSA), disuccinimidyl glutarate (DSG), bis-(4-nitrophenyl) adipate and bis-(4-nitrophenyl) succinate.

Preferred is a conjugate of general formula (**IV**)

**[H-Uₓ₊₁-Uₓ-T-O-L-NH-W]ₘ-IM** **(VI)**

wherein m is comprised between about 2 and about 18;
-W- is selected from: 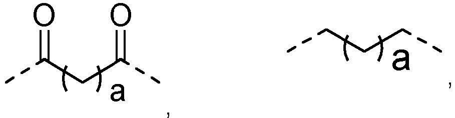 and 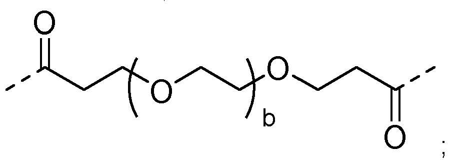
a represents an integer from 1 to 10; b represents an integer from 1 to 4;
**IM** represents an immunogenic carrier, and
Uₓ, Uₓ₊₁, T, x, n and L have the meanings defined herein.

As well known to the skilled person, "m" in structure **VI** corresponds to the average load of saccharide units per unit of immunogenic carrier as determined by MALDI-TOF MS method using the molecular weight of the immunogenic carrier as reference. By varying the reaction conditions for the coupling of the saccharide according to the present invention to the immunogenic carrier any conjugate of structure **VI** with m being comprised between about 2 and about 18 can be obtained.

Preferably, the linker -L- is selected from: -L^{a}-, -L^{a}-L^{e}-, -L^{a}-L^{b}-L^{e}-, and -L^{a}-L^{d}-L^{e}-;
-L^{a}- represents -(CH₂)ₒ-, -(CH₂-CH₂-O)ₒ-C₂H₄-, or -(CH₂-CH₂-O)ₒ-CH₂ ;
-L^{b}- represents -O-, -NH-CO-NH-, -NH-CO-CH₂-NH-, -NH-CO-;
-L^{d}- represents -(CH₂)_{q}-, -(CH(OH))_{q}-, -(CF₂)_{q}-, -(CH₂-CH₂-O)_{q}-C₂H₄-, or -(CH₂-CH₂-O)_{q}-CH₂- ;
**-L^{e}-** represents -(CH₂)ₚ₁-, -(CF₂)ₚ₁-, -C₂H₄-(O-CH₂-CH₂)ₚ₁-, -CH₂-(O-CH₂-CH₂)ₚ₁- or -(CH₂)ₚ₁-O-(CH₂)ₚ₂- ;
and o, q, p1 and p2 are independently of each other an integer selected from 1, 2, 3, 4, 5, and 6.
It is also preferred that -W- represents 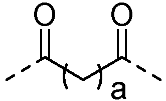 and a is an integer selected from 2, 3, 4, 5 and 6 is especially preferred.

Hence, a conjugate of general formula (**VI**), wherein
the linker -L- is selected from: -L^{a}-, -L^{a}-L^{e}-, -L^{a}-L^{b}-L^{e}-, and -L^{a}-L^{d}-L^{e}-;
-L^{a}- represents -(CH₂)ₒ-, -(CH₂-CH₂-O)ₒ-C₂H₄-, or -(CH₂-CH₂-O)ₒ-CH₂ ;
-L^{b}- represents -O-, -NH-CO-NH-, -NH-CO-CH₂-NH-, -NH-CO-;
-L^{d}- represents -(CH₂)_{q}-, -(CH(OH))_{q}-, -(CF₂)_{q}-, -(CH₂-CH₂-O)_{q}-C₂H₄-, or -(CH₂-CH₂-O)_{q}-CH₂- ;
**-L^{e-}** represents -(CH₂)ₚ₁-, -(CF₂)ₚ₁-, -C₂H₄-(O-CH₂-CH₂)ₚ₁-, -CH₂-(O-CH₂-CH₂)ₚ₁- or -(CH₂)ₚ₁-O-(CH₂)ₚ₂- ;
o, q, p1 and p2 are independently of each other an integer selected from 1, 2, 3, 4, 5, and 6;
-W- represents 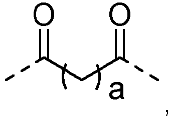 and a is an integer selected from 2, 3, 4, 5 and 6 is especially preferred.

Preferred is also conjugate of general formula (**VIa**)

**[H-Uₓ₊₁-Uₓ-T-O-L-NH-W]ₘ-CP** **(VIa)**

wherein m is comprised between about 2 and about 18;
-W- is selected from: 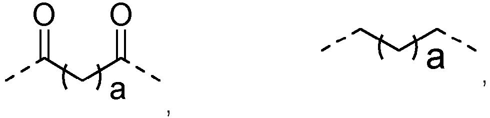 and 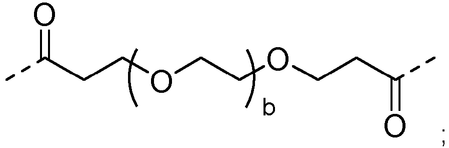
a represents an integer from 1 to 10;
b represents an integer from 1 to 4;
**CP** represents a carrier protein, and
Uₓ, Uₓ₊₁, T, x, n and L have the meanings defined herein.

As well known to the skilled person, "m" in structure **VIa** corresponds to the average load of saccharide units per unit of carrier protein as determined by MALDI-TOF MS method using the molecular weight of carrier protein as reference. By varying the reaction conditions for the coupling of the saccharide according to the present invention to the carrier protein any conjugate of structure **VIa** with m being comprised between about 2 and about 18 can be obtained.

Preferably, the linker -L- is selected from: -L^{a}-, -L^{a}-L^{e}-, -L^{a}-L^{b}-L^{e}-, and -L^{a}-L^{d}-L^{e}-;
-L^{a}- represents -(CH₂)ₒ-, -(CH₂-CH₂-O)ₒ-C₂H₄-, or -(CH₂-CH₂-O)ₒ-CH₂ ;
-L^{b}- represents -O-, -NH-CO-NH-, -NH-CO-CH₂-NH-, -NH-CO-;
-L^{d}- represents -(CH₂)_{q}-, -(CH(OH))_{q}-, -(CF₂)_{q}-, -(CH₂-CH₂-O)_{q}-C₂H₄-, or -(CH₂-CH₂-O)_{q}-CH₂- ;
-L^{e}- represents -(CH₂)ₚ₁-, -(CF₂)ₚ₁-, -C₂H₄-(O-CH₂-CH₂)ₚ₁-, -CH₂-(O-CH₂-CH₂)ₚ₁- or -(CH₂)ₚ₁-O-(CH₂)ₚ₂- ;
and o, q, p1 and p2 are independently of each other an integer selected from 1, 2, 3, 4, 5, and 6.

It is also preferred that -W- represents 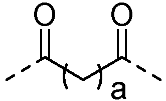 and a is an integer selected from 2, 3, 4, 5 and 6 is especially preferred.

Hence, a conjugate of general formula (VI), wherein
the linker -L- is selected from: -L^{a}-, -L^{a}-L^{e}-, -L^{a}-L^{b}-L^{e}-, and -L^{a}-L^{d}-L^{e}-;
-L^{a}- represents -(CH₂)ₒ-, -(CH₂-CH₂-O)ₒ-C₂H₄-, or -(CH₂-CH₂-O)ₒ-CH₂ ;
-L^{b}- represents -O-, -NH-CO-NH-, -NH-CO-CH₂-NH-, -NH-CO-;
-L^{d}- represents -(CH₂)_{q}-, -(CH(OH))_{q}-, -(CF₂)_{q}-, -(CH₂-CH₂-O)_{q}-C₂H₄-, or -(CH₂-CH₂-O)_{q}-CH₂- ;
-L^{e}- represents -(CH₂)ₚ₁-, -(CF₂)ₚ₁-, -C₂H₄-(O-CH₂-CH₂)ₚ₁-, -CH₂-(O-CH₂-CH₂)ₚ₁- or -(CH₂)ₚ₁-O-(CH₂)ₚ₂- ;
o, q, p1 and p2 are independently of each other an integer selected from 1, 2, 3, 4, 5, and 6;
-W- represents 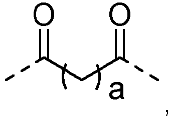 and a is an integer selected from 2, 3, 4, 5 and 6 is especially preferred.

The cysteine residue on the carrier protein can be converted to the corresponding dehydroalanine that can be further reacted with a suitable interconnecting molecule to provide modified carrier protein having on their surface the functional group X of the interconnecting molecule.

It is especially preferred that the inventive saccharides described herein are conjugated to the non-toxic mutated diphtheria toxin CRM₁₉₇ presenting as a functionality a primary amine functionality of a lysine residue.

CRM₁₉₇ like wild-type diphtheria toxin is a single polypeptide chain of 535 amino acids (58 kD) consisting of two subunits linked by disulfide bridges having a single amino acid substitution of glutamic acid for glycine. It is utilized as a carrier protein in a number of approved conjugate vaccines for diseases such as Prevnar^{®}.

Thus, in a preferred embodiment of the present invention the carrier protein presents on its surface primary amino functionalities of lysine residues that are able to react with the functional group Y of the interconnecting molecule to provide modified carrier protein having on their surface said functional group X of the interconnecting molecule, which is able to react with the terminal amino group of the linker functionalizing the inventive saccharides.

Said functional group X of the interconnecting molecules is selected from the group comprising or consisting of maleimide; α-iodoacetyl; α-bromoacetyl; and *N-*hydroxysuccinimide ester (NHS), aldehyde, imidoester, carboxylic acid, alkyl sulfonate, sulfonyl chloride, epoxide, anhydride, carbonate (see **Figure 3**).

Even more preferred is a conjugate of general formula (VII),

**[H-Uₓ₊₁-Uₓ-T-O-L-NH-W]ₘ-CRM₁₉₇** **(VII)**

wherein m is comprised between about 2 and about 18;
-W- is selected from: 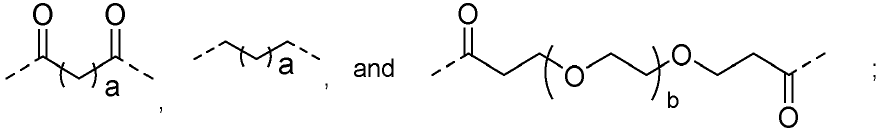
a represents an integer from 1 to 10;
b represents an integer from 1 to 4, and
Uₓ, Uₓ₊₁, T, x, n and L have the meanings defined herein.

Also preferred is a conjugate of general formula (**VII**), wherein x represents 1, 2, 3 or 4. Thus, a conjugate of general formula (**VII-a**), (**VII-b**), (**VII-c**), or (**VII-d**), 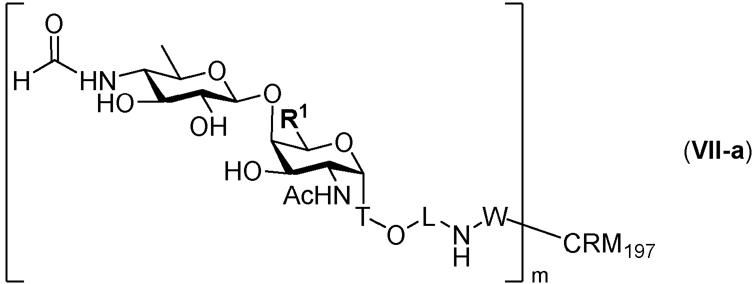 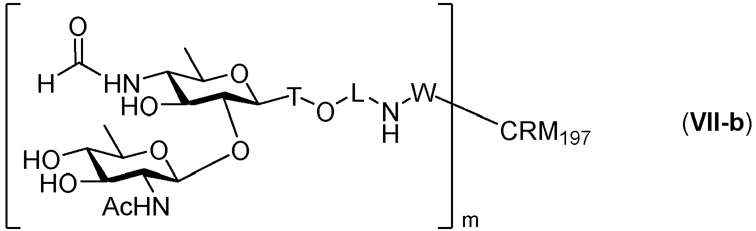 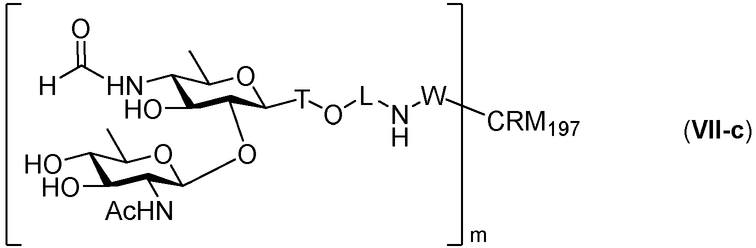 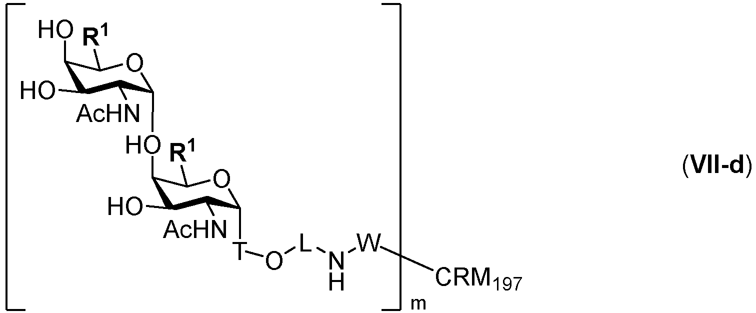
wherein m is comprised between about 2 and about 18;
-W- is selected from: 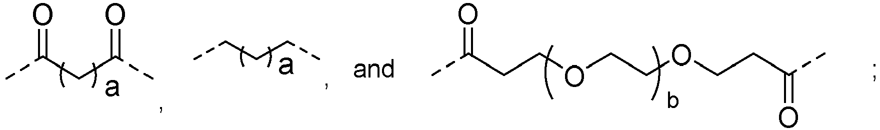
a represents an integer from 1 to 10;
b represents an integer from 1 to 4;
T, L, and R¹ have the meaning defined herein, is also preferred.

Also preferred is a conjugate of general formula (**VII**), wherein T represents a bond, - Uₓ₊₃-, or -Uₓ₊₃-Uₓ₊₂-. Thus, a conjugate of general formula (**VIII-a**), (**VIII-b**), (**VIII-c**), (**VIII-d**), (**IX-a**), (**IX-b**), (**IX-c**), (**IX-d**), (**X-a**), (**X-b**), (**X-c**), or (**X-d**) 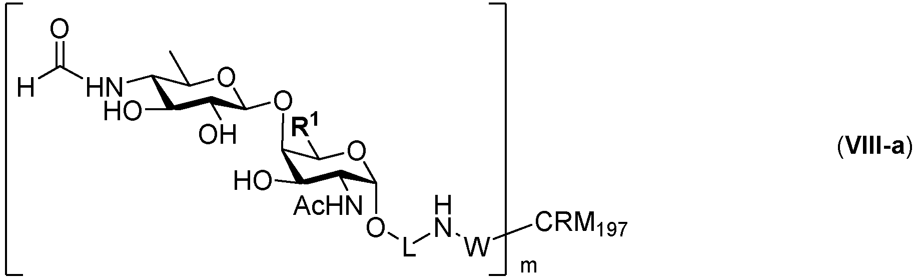 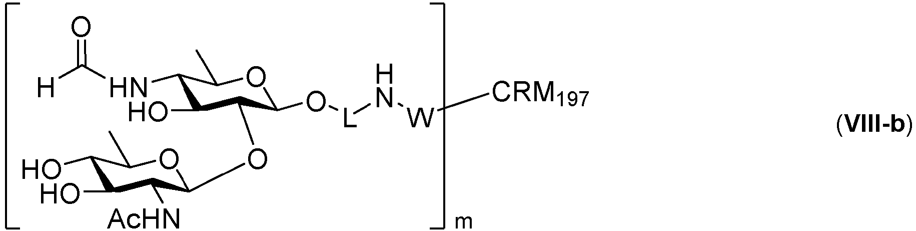 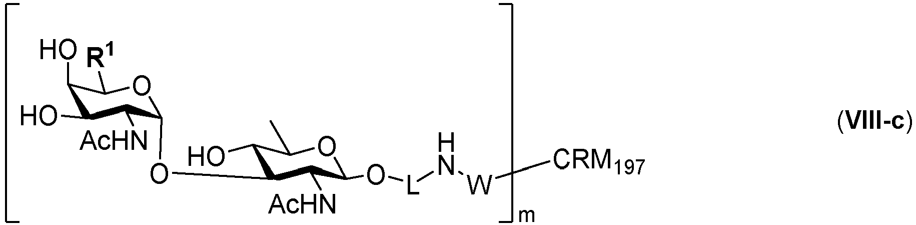 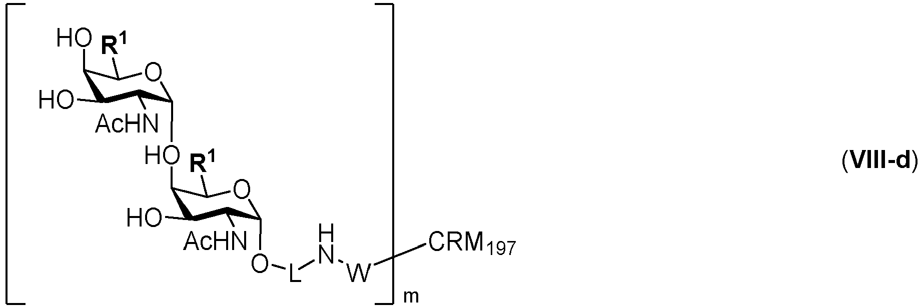 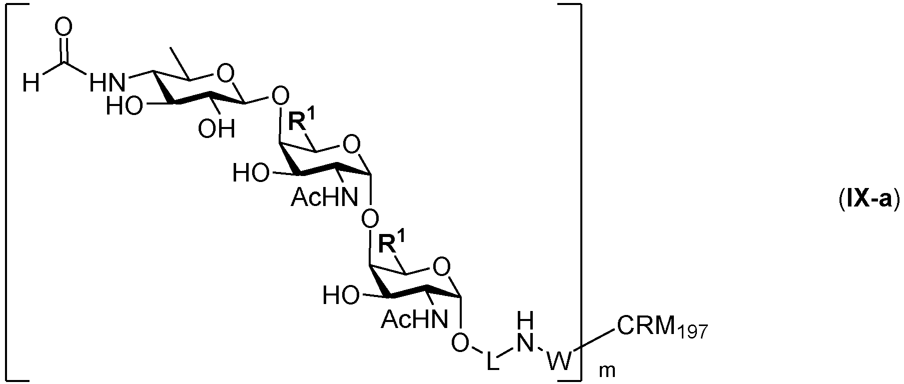 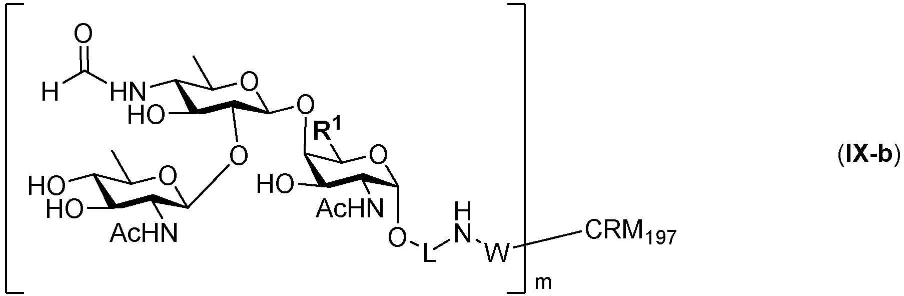 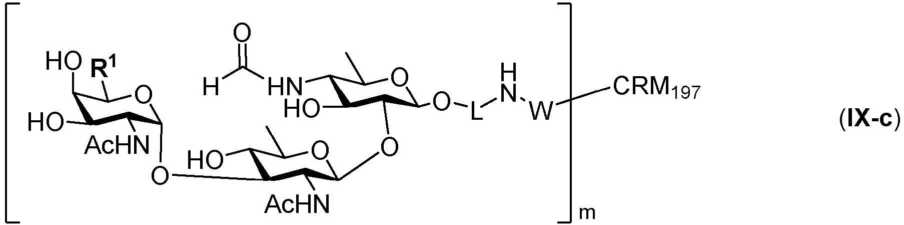 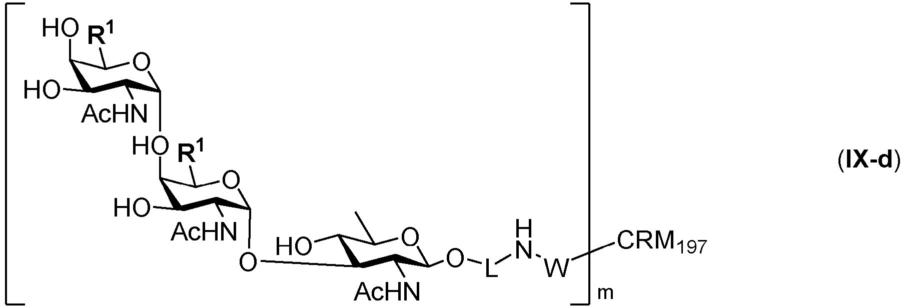 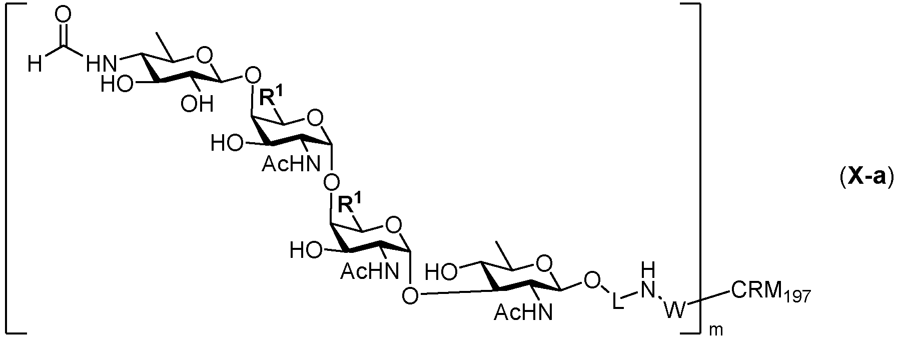 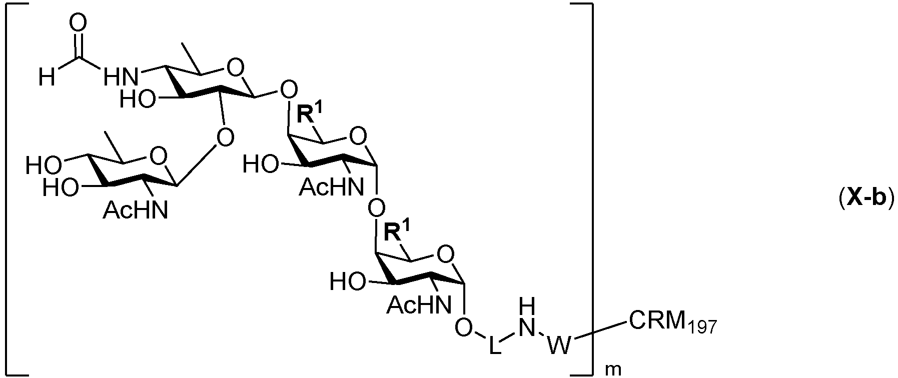 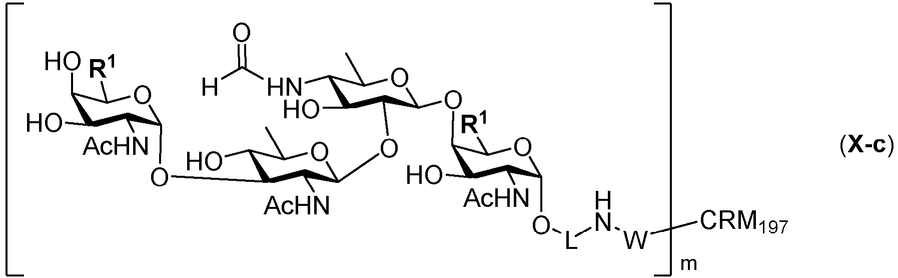 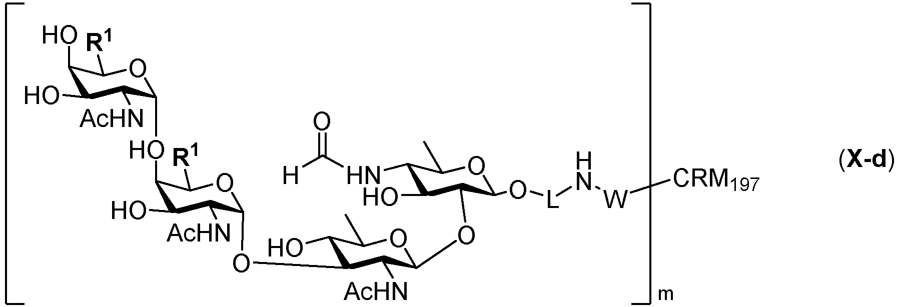
wherein m is comprised between about 2 and about 18;
-W- is selected from: 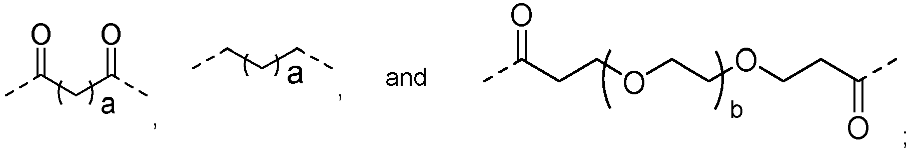
a represents an integer from 1 to 10;
b represents an integer from 1 to 4;
L, and R¹ have the meaning defined herein, is also preferred.

Preferably, in general formulae (**VII**), (**VII-a**), (**VII-b**), (**VII-c**), (**VII-d**), (**VIII-a**), (**VIII-b**), (**VIII-c**), (**VIII-d**), (**IX-a**), (**IX-b**), (**IX-c**), (**IX-d**), (**X-a**), (**X-b**), (**X-c**), and (**X-d**) the linker
-L- is selected from: -L^{a}-, -L^{a}-L^{e}-, -L^{a}-L^{b}-L^{e}-, and -L^{a}-L^{d}-L^{e}-;
-L^{a}- represents -(CH₂)ₒ-, -(CH₂-CH₂-O)_{q}-C₂H₄-, or -(CH₂₋CH₂₋O)_{q}-CH₂;
-L^{b}- represents -O-, -NH-CO-NH-, -NH-CO-CH₂-NH-, -NH-CO-;
-L^{d}- represents -(CH₂)_{q}-, -(CH(OH))_{q}-, -(CF₂)_{q}-, -(CH₂-CH₂-O)_{q}-C₂H₄-, or -(CH₂-CH₂-0)_{q}-CH₂- ;
-L^{e}- represents -(CH₂)ₚ₁-, -(CF₂)ₚ₁-, -C₂H₄-(O-CH₂-CH₂)ₚ₁-, -CH₂-(O-CH₂-CH₂)ₚ₁- or -(CH₂)ₚ₁-O-(CH₂)ₚ₂- ;
o, q, p1 and p2 are independently of each other an integer selected from 1, 2, 3, 4, 5, and 6;
-W- represents 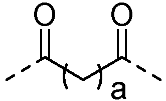 and a is an integer selected from 2, 3, 4, 5 and 6.

Especially preferred is a conjugate of general formula (**VII**), (**VII-a**), (**VII-b**), (**VII-c**), (**VII-d**), (**VIII-a**), (**VIII-b**), (**VIII-c**), (**VIII-d**), (**IX-a**), (**IX-b**), (**IX-c**), (**IX-d**), (**X-a**), (**X-b**), (**X-c**), or (**X-d**), wherein the linker -L- represents -(CH₂)ₒ- ,
o is an integer selected from 2, 3, 4, 5, 6, 7 and 8;
-W- represents 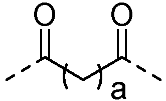 and a is an integer selected from 2, 3, 4, 5 and 6.

Preferably m is comprised between about 2 and about 18, more preferably between about 5 and about 15, even more preferably between about 8 and about 12.

In another embodiment, said immunogenic carrier is preferably a glycosphingolipid with immunomodulatory properties, and more preferably (2*S*,3*S*,4*R*)-1-(α-D-galactopyranosyl)-2-hexacosanoylaminooctadecane-3,4-diol. The term glycosphingolipid with immunomodulatory properties, as used herein, refers to a suitable glycosphingolipid capable of stimulating the immune system's response to a target antigen, but which does not in itself confer immunity as defined above.

Glycosphingolipids as used herein are compounds containing a carbohydrate moiety α-linked to a sphingolipid. Preferably, the carbohydrate moiety is a hexopyranose and most preferably is α-D-galactopyranose. For the person skilled in the art, sphingolipids are a class of lipids containing a C18 amino alcohol connected *via* an amide bond to a fatty acid. The C18 amino alcohol is preferably mono-, di- or polysubstituted with hydroxyl groups. Especially preferred, the C18 amino alcohol is phytosphingosine. The fatty acid is preferably a monocarboxylic acid having a saturated alkyl chain of a number of carbons ranging from 16 to 28 and more preferably from 18 to 26. Glycosphingolipids with immunomodulatory properties include, but they are not restricted to (2*S*,3*S*,4*R*)-1-(α-D-galactopyranosyl)-2-hexacosanoylaminooctadecane-3,4-diol, which can stimulate natural killer (NK) activity and cytokine production by natural killer T (NKT) cells and exhibits potent antitumor activity *in vivo* (Proc. Natl Acad. Sci. USA, 1998, 95, 5690).

The conjugates of the inventive saccharides with a glycosphingolipid with immunomodulatory properties have the advantage of being heat stable. To be suitable for conjugation, on the glycosphingolipid with immunomodulatory properties a functionality is introduced. Said functionality is prone to react directly with the terminal amino group of the linker of the inventive to provide conjugates of the saccharides or with the functional group Y of the interconnecting molecule to provide the modified glycosphingolipid with immunomodulatory properties.

Preferably, said functionality is introduced at the C6 of the carbohydrate moiety of the glycosphingolipid with immunomodulatory properties. Thus, the glycosphingolipid with immunomodulatory properties is functionalized with a functionality, which is prone of reacting with the terminal amino group of the saccharides or with the functional group Y of the interconnecting molecule. A functionality prone to react with an amino group includes, but it is not restricted to activated ester, isocyanate group, aldehyde, epoxide, imidoester, carboxylic acid, alkyl sulfonate and sulfonyl chloride. A functionality prone to react with the functional group Y of the interconnecting molecule so that to provide the modified glycosphingolipid with immunomodulatory properties presenting the functional group X of the interconnecting molecule includes, but it is not restricted to amine, alcohol, thiol, activated ester, isocyanate group, aldehyde, epoxide, vinyl, imidoester, carboxylic acid, alkyl sulfonate, sulfonyl chloride, vinyl group, alkynyl group and azido group.

Preferably, the functionality introduced at the C-6 position of the carbohydrate moiety of the glycosphingolipid with immunomodulatory properties is selected from the group comprising or containing an amine, a thiol, an alcohol, a carboxylic acid, a vinyl, maleimide, α-iodoacetyl, α-bromoacetyl, *N*-hydroxysuccinimide ester (NHS), 2-pyridyldithiols.

Said functional group X of the interconnecting molecules is selected from the group comprising or consisting of maleimide, α-iodoacetyl, α-bromoacetyl, *N-*hydroxysuccinimide ester (NHS), aldehyde, carboxylic acid, epoxyde, alkyl sulfonate, sulfonyl chloride, anhydride, carbonate.

Preferably, di(*N*-succinimidyl) adipate or bis(4-nitrophenyl) adipate is first reacted with a synthetic saccharide having a primary amino group. Activated saccharide is subsequently condensed with a glycosphingolipid, which is modified at C-6 position by an interconnecting molecule having a terminal amino functionality in order to afford the conjugate.

As used herein, the term "interconnecting molecule" refers to a bifunctional molecule containing functional group X and functional group Y, wherein functional group X is capable of reacting with the terminal amino group on the linker -L- and the functional group Y is capable of reacting with a functionality present on the immunogenic carrier.

As used herein, the term "interconnecting molecule" refers to a bifunctional molecule containing functional group X and functional group Y, wherein functional group X is capable of reacting with the terminal amino group on the linker -L- and the functional group Y is capable of reacting with a functionality present on the immunogenic carrier.

Thus, in another preferred embodiment the conjugate has the structure of general formula (XI)

**H-Uₓ₊₁-Uₓ-T-O-L-NH-W'-GSL** **(XI)**

-W'- represents -L²-NH-L³- ;
L³ represents -L ^{3'}-L^{3"}-L^{3‴}- - or -L^{3'}-L^{3‴}- or -L^{3'}-; and
L^{3'}, L^{3"}, and L^{3‴} are independently of each other selected from: -CH₂-,-C₂H₄-, -C₃H₆-, -C₄H₈-, -C₅H₁₀-, -C₆H₁₂-, -C₇H₁₄-, -C₈H₁₆-, -C₉H₁₈-,-C₁₀H₂₀-, -CR²³R²⁴-, -CR²⁵R²⁶-, -CR²⁷R²⁸-, -CR²⁹R³⁰- , -CR³¹ R³²-,-CR³³R³⁴-, -(CH₂-CH₂-O)ₒ-; -*o*-C₆H₄-, -*m*-C₆H₄-, -*p*-C₆H₄-, -CH₂-S-CH₂--CH₂-O-CH₂-, -S-, -O-;
L² is selected from: -C(O)-, -E-, -C(O)-NH-NH-C(O)- 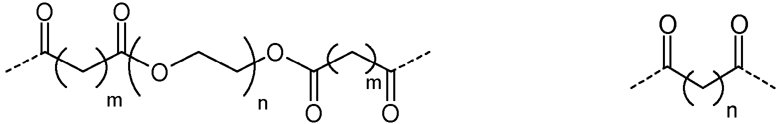 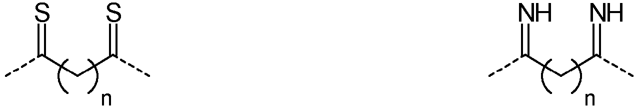 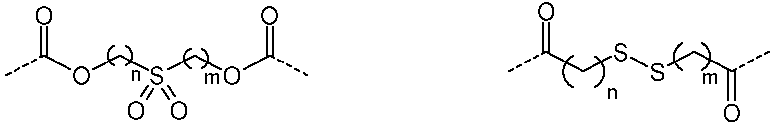 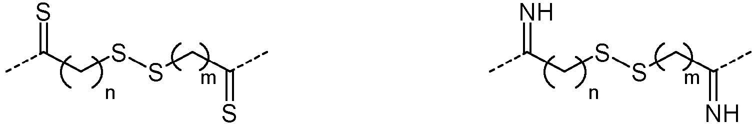 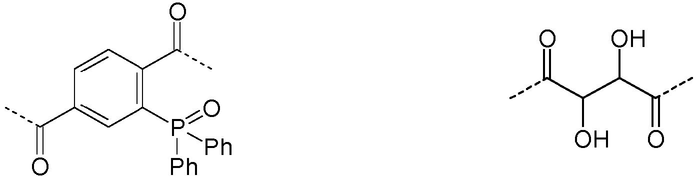 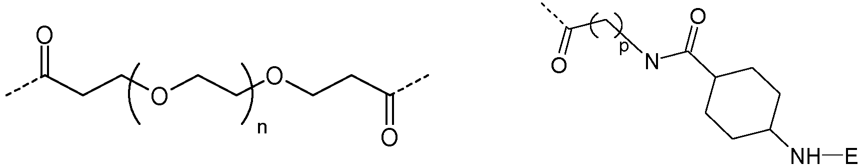 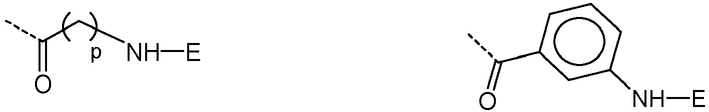 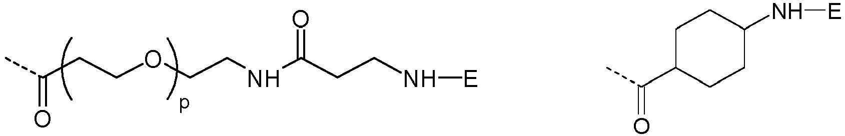 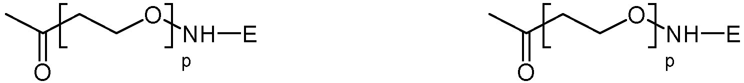
E is selected from 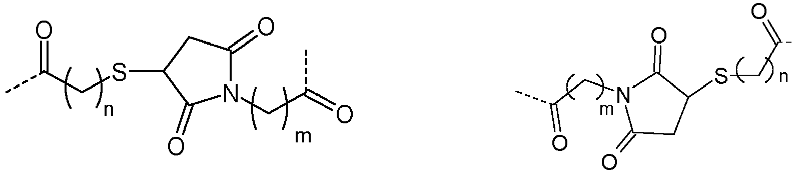 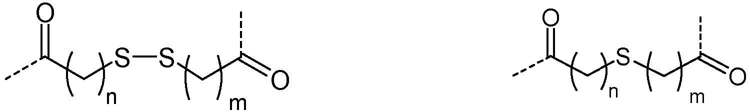 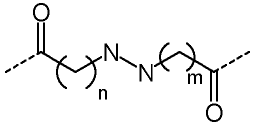
m, n, p represent independently of each other an integer from 0 to 30; **GSL** represents a glycosphingolipid,
R²³ - R³⁴ are independently of each other selected from: -H, -F, -Cl, -CH₃, -C₂H₅, -C₃H₇, -C₅H₉, -C₆H₁₃, -OCH₃, -OC₂H₅, -CH₂F, -CHF₂, -CF₃, -C(O)-NH₂, -SCH₃, -SC₂H₅, -NHC(O)CH₃, -N(CH₃)₂, and -N(C₂H₅)₂; and Uₓ, Uₓ₊₁, Uₓ₊₂, Uₓ₊₃, x, and L have the meanings defined herein.

In a preferred embodiment of the conjugate of general formula (**XI**), L² is selected from: 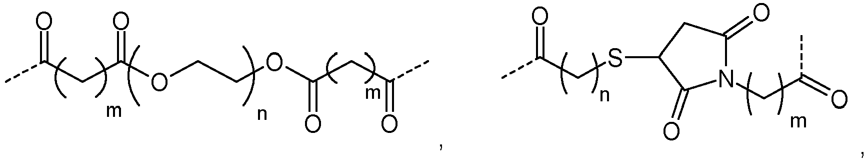 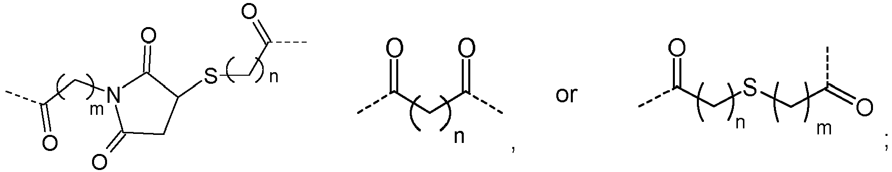 and m and n represent independently of each other an integer from 0 to 30.

In a further preferred embodiment the glycosphingolipid **GSL** has the following structure: 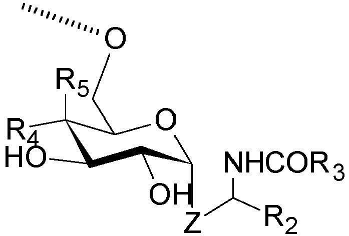
wherein R² is selected from 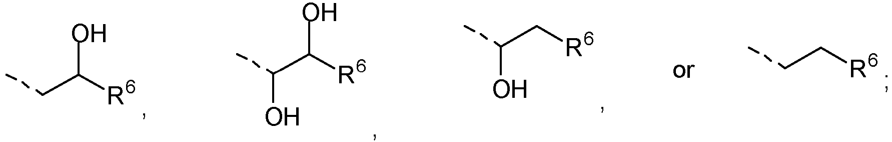
R³ is -(X¹)ₚ₁-(X²)ₚ₂-(X³)ₚ₃-X⁴;
R⁴ and R⁵ are selected from -H and -OH and cannot be simultaneously the same;
R⁶ is -(Y¹)ₘ₁-(Y²)ₘ₂-(Y³)ₘ₃-Y⁴;
X¹, X², X³, Y¹, Y², and Y³ are independently of each other selected from:
-CH₂-, -CH(OH)-, -CH(CH₃)-, -CH(C₂H₅)-, -CH(C₃H₇)-, -CH(C₄H₉)-, 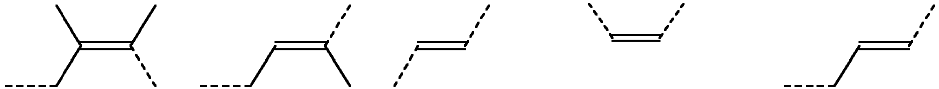 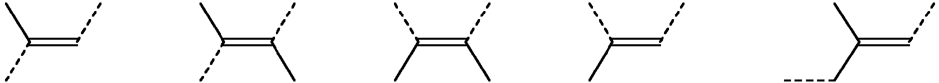 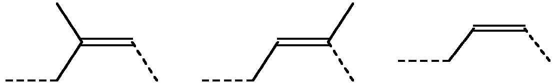
X⁴ represents: -H, -/Pr, -tBu, or -sBu, 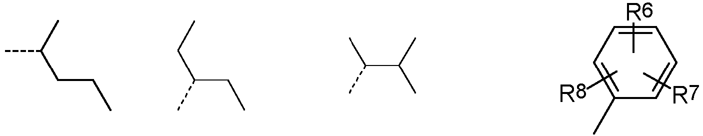 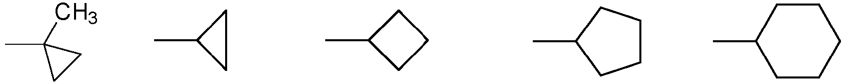 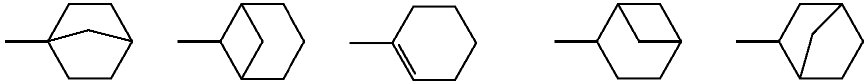 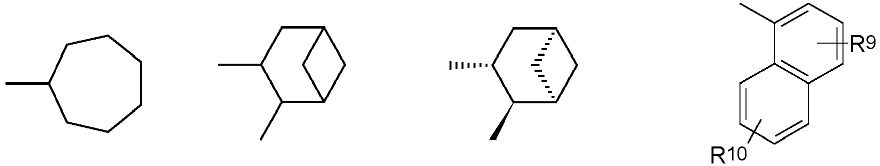 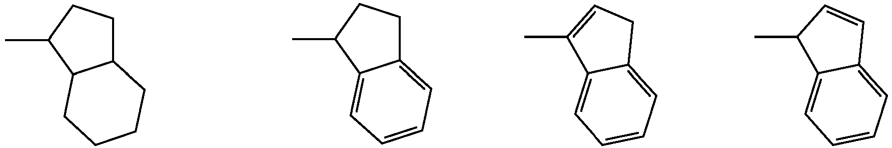 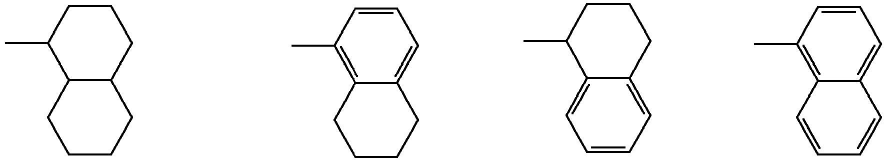
Y⁴ is selected from: -H, -/Pr, -tBu, -Ph, sBu, 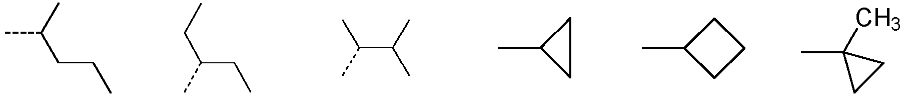 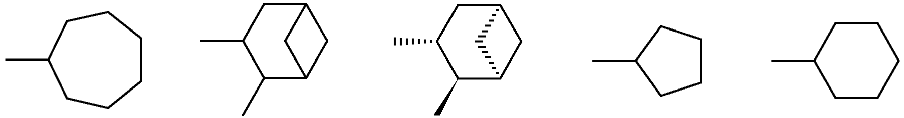 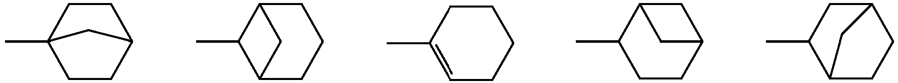 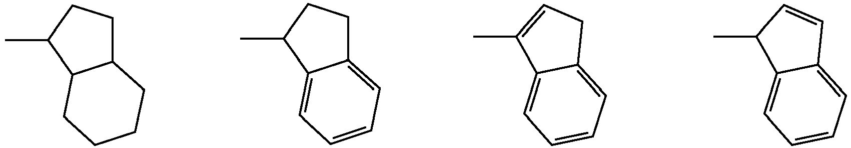 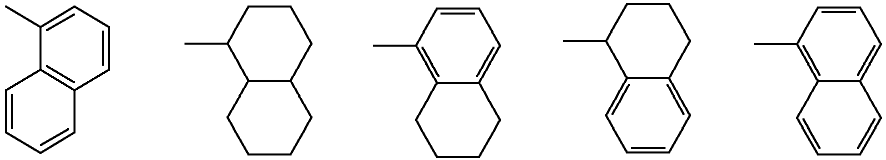
p1, p2, p3, m1, m2 and m3 represent independently of each other an integer from 0 to 30;

In a particularly preferred embodiment, the glycosphingolipid is (2*S*,3*S*,4*R*)-1-(α-D-galactopyranosyl)-2-hexacosanoylaminooctadecane-3,4-diol (α-galactosylceramide).

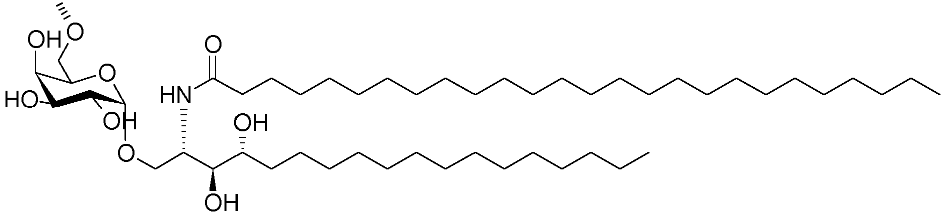

Also preferred is a conjugate of general of general formula (**XI-a**), (**XI-b**), (**XI-c**), (**XI-d**), (**XII-a**), (**XII-b**), (**XII-c**), (**XII-d**), (**XIII-a**), (**XIII-b**), (**XIII-c**), (**XIII-d**), wherein **GSL** represents a glycosphingolipid as defined herein 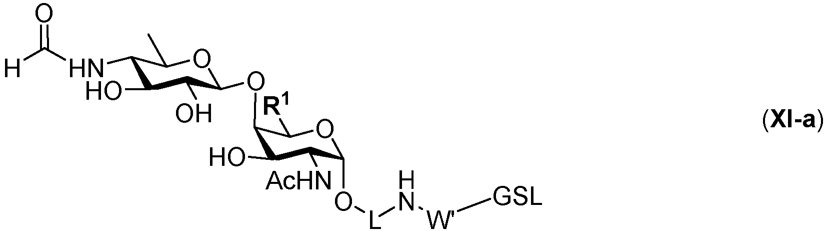 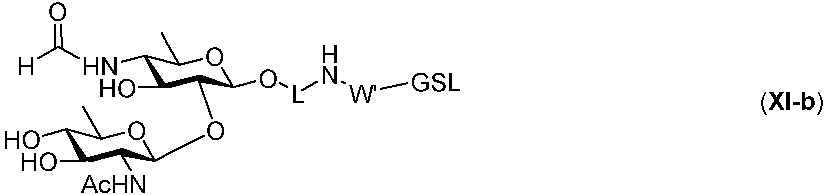 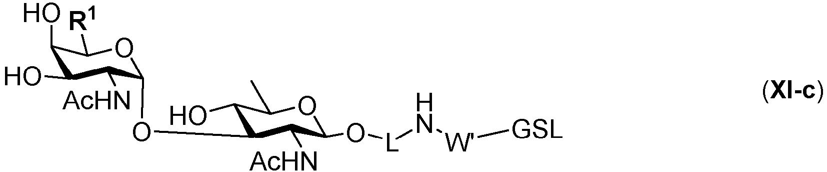 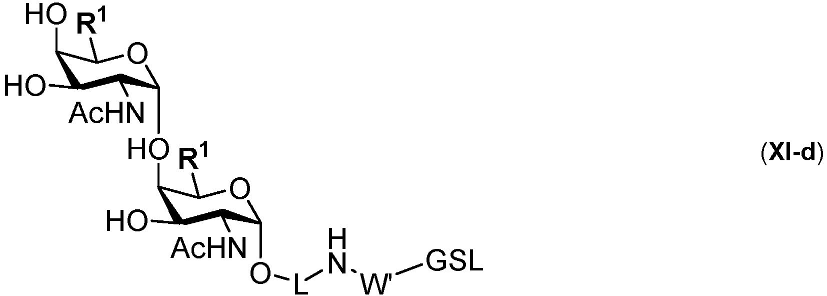 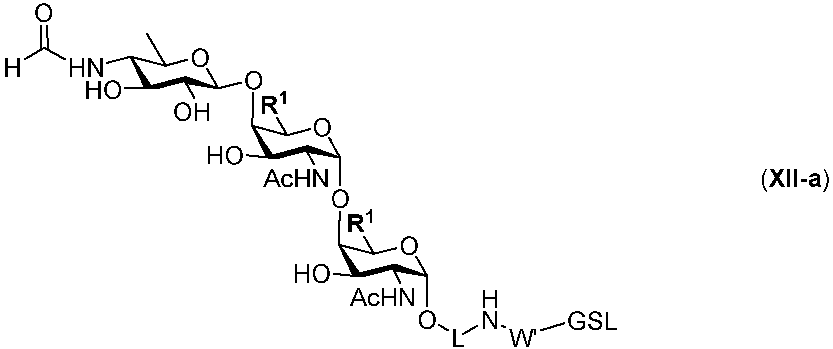 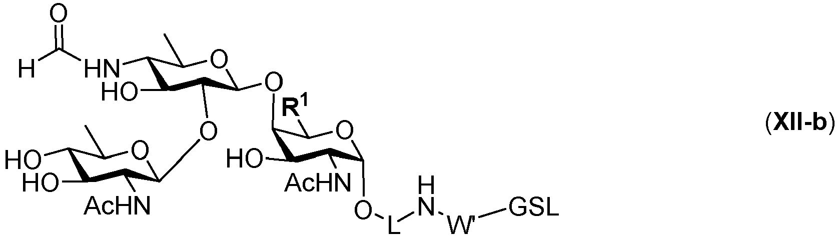 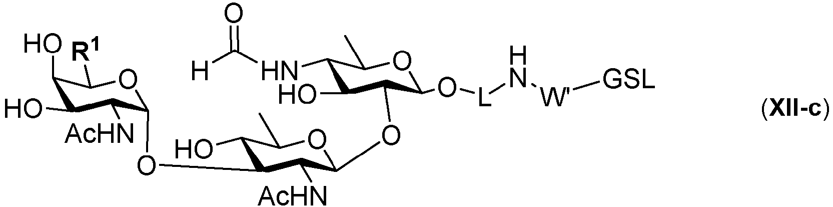 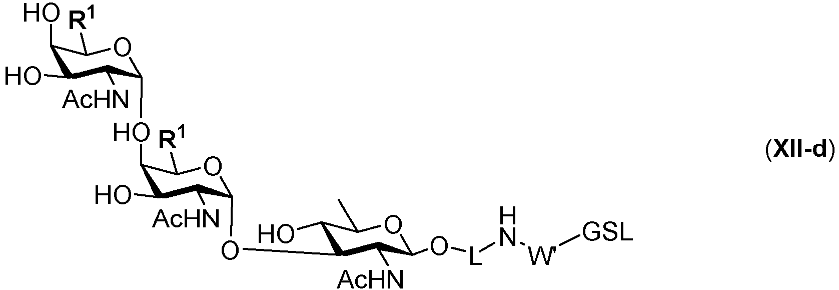 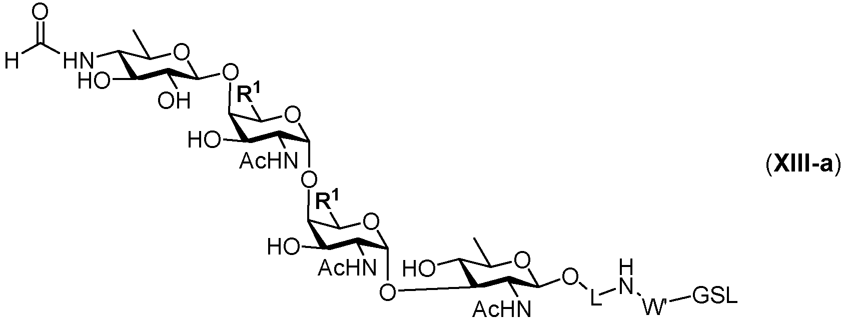 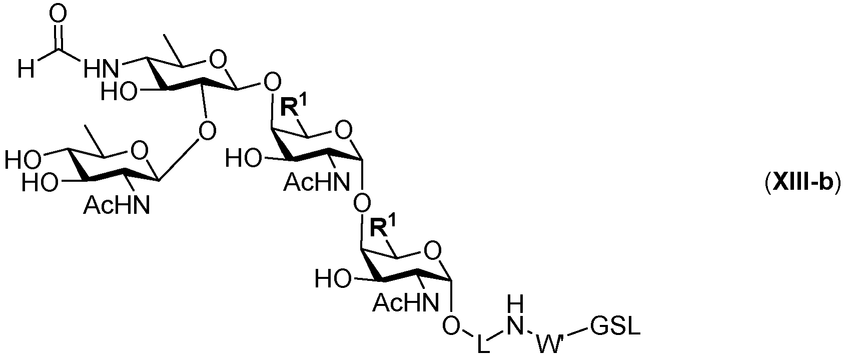 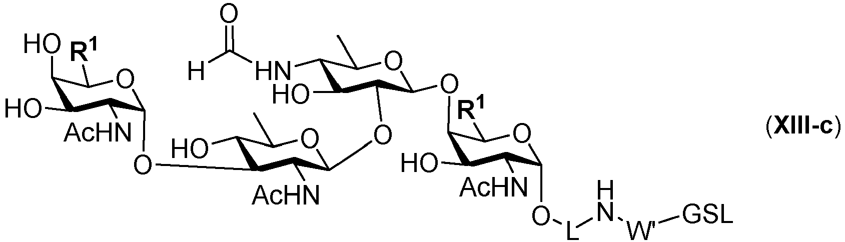 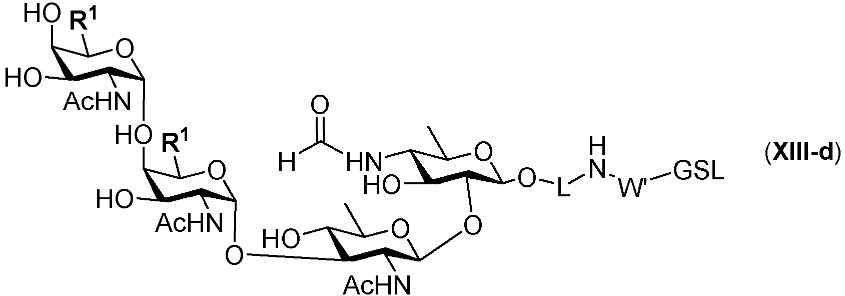 wherein R¹, W, L and **GSL** have the meanings as defined herein.

It was found that the saccharide of general formula (**I**), (**I-a**), (**I-b**), (**I-c**), (**I-d**), (**II-a**), (**II-b**), (**II-c**), (**II-d**), (**III-a**), (**III-b**), (**III-c**), (**III-d**), (**IV-a**), (**IV-b**), (**IV-c**), (**IV-d**), or (**V**) covalently linked or covalently bound to an immunogenic carrier through the nitrogen atom of the -O-L-NH₂ group or in other words the conjugate obtained by reacting a saccharide of general formula (**I**), (**I-a**), (**I-b**), (**I-c**), (**I-d**), (**II-a**), (**II-b**), (**II-c**), (**II-d**), (**III-a**), (**III-b**), (**III-c**), (**III-d**), (**IV-a**), (**IV-b**), (**IV-c**), (**IV-d**), or (**V**) with an immunogenic carrier, and especially a conjugate of general formula (**VI**), (**VIa**), (**VII**), (**VII-a**), (**VII-b**), (**VII-c**), (**VII-d**), (**VIII-a**), (**VIII-b**), (**VIII-c**), (**VIII-d**), (**IX-a**), (**IX-b**), (**IX-c**), (**IX-d**), (**X-a**), (**X-b**), (**X-c**), (**X-d**), (**XI**), (**XI-a**), (**XI-b**), (**XI-c**), (**XI-d**), (**XII-a**), (**XII-b**), (**XII-c**), (**XII-d**), (**XIII-a**), (**XIII-b**), (**XIII-c**), or (**XIII-d**), elicits a protective immune response in a human and/or animal host, and therefore is useful in the prevention and/or treatment of a disease caused by *F. tularensis.* Such disease includes tularemia, particularly ulceroglandular tularemia, glandular tularemia, oculoglandular tularemia, oropharyngeal tularemia, pneumonic tularemia, or typhoidal tularemia.

It was also found that the saccharide of general formula (**I**), (**I-a**), (**I-b**), (**I-c**), (**I-d**), (**II-a**), (**II-b**), (**II-c**), (**II-d**), (**III-a**), (**III-b**), (**III-c**), (**III-d**), (**IV-a**), (**IV-b**), (**IV-c**), (**IV-d**), or (**V**) covalently linked or covalently bound to an immunogenic carrier through the nitrogen atom of the -O-L-NH₂ group or in other words the conjugate obtained by reacting a saccharide of general formula (**I**), (**I-a**), (**I-b**), (**I-c**), (**I-d**), (**II-a**), (**II-b**), (**II-c**), (**II-d**), (**III-a**), (**III-b**), (**III-c**), (**III-d**), (**IV-a**), (**IV-b**), (**IV-c**), (**IV-d**), or (**V**) with an immunogenic carrier, and especially a conjugate of general formula (**VI**), (**VIa**), (**VII**), (**VII-a**), (**VII-b**), (**VII-c**), (**VII-d**), (**VIII-a**), (**VIII-b**), (**VIII-c**), (**VIII-d**), (**IX-a**), (**IX-b**), (**IX-c**), (**IX-d**), (**X-a**), (**X-b**), (**X-c**), (**X-d**), (**XI**), (**XI-a**), (**XI-b**), (**XI-c**), (**XI-d**), (**XII-a**), (**XII-b**), (**XII-c**), (**XII-d**), (**XIII-a**), (**XIII-b**), (**XIII-c**), or (**XIII-d**), elicits a protective immune response in an animal host susceptible to a *Francisella tularensis* infection, and therefore is useful in the prevention and/or treatment of a disease in an animal caused by *F. tularensis,* particularly tularemia.

Animals susceptible to a *Francisella tularensis* infection include, but are not limited to, rodents, rabbits, hares, horses, cats, cheetahs, and dogs.

### Pharmaceutical composition

A further aspect of the present invention relates to a pharmaceutical composition or a vaccine containing at least one synthetic saccharide and/or a pharmaceutically acceptable salt thereof according to the present invention and/or a conjugate comprising a saccharide according to the present invention covalently linked to an immunogenic carrier, preferably to CRM₁₉₇ carrier protein, through the nitrogen atom of the -O-L-NH₂ group together with at least one pharmaceutically acceptable adjuvant and/or excipient.

The term "adjuvant" as used herein refers to an immunological adjuvant i.e. a material used in a pharmaceutical composition or vaccine that modifies or augments the effects of said pharmaceutical composition or said vaccine by enhancing the immune response to a given antigen contained in the pharmaceutical composition or vaccine without being antigenically related to it. For the persons skilled in the art, classically recognized examples of immunological adjuvants include, but are not restricted to oil emulsions (e.g. Freund's adjuvant), saponins, aluminium or calcium salts (e.g. alum), non-ionic block polymer surfactants, and many others.

Pharmaceutical compositions or vaccines are preferably in aqueous form, particularly at the point of administration, but they can also be presented in non-aqueous liquid forms or in dried forms e.g. as gelatin capsules, or as lyophilisates, etc.. Pharmaceutical compositions or vaccines may include one or more preservatives, such as thiomersal or 2-phenoxyethanol. Mercury-free compositions are preferred, and preservative-free vaccines can be prepared.

Pharmaceutical compositions or vaccines may include a physiological salt, such as a sodium salt e.g. to control tonicity. Sodium chloride (NaCl) is typical and may be present at between 1 and 20 mg/ml. Other salts that may be present include potassium chloride, potassium dihydrogen phosphate, disodium phosphate dehydrate, magnesium chloride, calcium chloride, etc.

Pharmaceutical compositions or vaccines can have an osmolality of between 200 mOsm/kg and 400 mOsm/kg.

Pharmaceutical compositions or vaccines may include compounds (with or without an insoluble metal salt) in plain water (e.g. w.f.i.), but will usually include one or more buffers. Typical buffers include: a phosphate buffer; a Tris buffer; a borate buffer; a succinate buffer; a histidine buffer (particularly with an aluminium hydroxide adjuvant); or a citrate buffer. Buffer salts will typically be included in the 5-20 mM range.

Pharmaceutical compositions or vaccines typically have a pH between 5.0 and 9.5, e.g. between 6.0 and 8.0.

Pharmaceutical compositions or vaccines are preferably sterile and gluten free.

Pharmaceutical compositions or vaccines are suitable for administration to animal (and, in particular, human) patients, and thus include both human and veterinary uses. They may be used in a method of raising an immune response in a patient, comprising the step of administering the composition to the patient.

The pharmaceutical compositions or vaccines of the present invention may be administered before a subject is exposed to *Francisella tularensis* and/or after a subject is exposed to *Francisella tularensis.*

Pharmaceutical compositions or vaccines may be prepared in unit dose form. In some embodiments a unit dose may have a volume of between 0.1-1.0 mL e.g. about 0.5 mL.

Pharmaceutical compositions or vaccines of the invention may be prepared in various forms. For example, the pharmaceutical compositions or vaccines may be prepared as injectables, either as liquid solutions or suspensions. Solid forms suitable for solution in, or suspension in, liquid vehicles prior to injection can also be prepared (e.g. a lyophilized composition or a spray-freeze dried composition). The composition may be prepared for topical administration e.g. as an ointment, cream or powder. The composition may be prepared for oral administration e.g. as a tablet or capsule, as a spray, or as a syrup (optionally flavored). The composition may be prepared for pulmonary administration e.g. by an inhaler, using a fine powder or a spray. The composition may be prepared as a suppository. The composition may be prepared for nasal, aural or ocular administration e.g. as a spray or drops. Injectables for intramuscular administration are typical.

A therapeutically effective dosage of one conjugate according to the present invention or of one saccharide of general formula (**I**) refers to that amount of the compound that results in an at least a partial immunization against a disease. Toxicity and therapeutic efficacy of such compounds can be determined by standard pharmaceutical, pharmacological, and toxicological procedures in cell cultures or experimental animals. The dose ratio between toxic and therapeutic effect is the therapeutic index. The actual amount of the composition administered will be dependent on the subject being treated, on the subject's weight, the severity of the affliction, the manner of administration and the judgement of the prescribing physician.

### Antibody

A further embodiment of the present invention relates to a monoclonal antibody having specificity for the saccharide of general formula (**I**) or a fragment thereof. Said antibody is able to prevent and treat diseases caused by *Francisella tularensis.* Thus, the monoclonal antibody is useful for passive immunization against *Francisella tularensis* infections by providing a fast immune response in any subject. The monoclonal antibody is particularly useful for passive immunization against *Francisella tularensis* infections in a subject having a deficient immune response and who does not respond to active immunization. Thus, the present invention also relates to a monoclonal antibody recognizing the *O*-antigen capsular polysaccharide of *Francisella tularensis* type A and type B.

The term fragment of a saccharide of general formula (**I**) refers to constituting subunits of a saccharide of general formula (**I**). For example, if the saccharide of general formula (**I**) is a hexasaccharide, then a fragment thereof includes penta-, tetra-, tri-, di- and monosaccharides, which are the constituting units of the hexasaccharide.

The term "antibody" as used herein encompasses polyclonal and monoclonal antibody preparations, as well as preparations including hybrid antibodies, F(ab')₂ fragments, F(ab) molecules, single domain antibodies and functional fragments thereof, which exhibit immunological binding properties of the parent antibody molecule. The antibody disclosed herein may be a monoclonal antibody.

Preferably, the monoclonal antibody has been raised against a conjugate according to the present invention.

Also disclosed herein is a vaccine composition comprising a monoclonal antibody having specificity for the saccharide of general formula (**I**) or a fragment thereof, and a pharmaceutically acceptable carrier.

The vaccine composition typically includes one or more pharmaceutically acceptable carriers (e.g., sterile liquids, such as water and oils, including those of petroleum, animal, vegetable or synthetic origin, such as peanut oil, soybean oil, mineral oil, sesame oil and the like). Water is a more typical carrier when the pharmaceutical composition is administered intravenously. Saline solutions and aqueous dextrose and glycerol solutions can also be employed as liquid carriers, particularly for injectable solutions. Suitable pharmaceutical excipients include, for example, starch, glucose, lactose, sucrose, gelatine, malt, rice, flour, chalk, silica gel, sodium stearate, glycerol monostearate, talc, sodium chloride, dried skimmilk, glycerol, propylene glycol, water, ethanol, and the like. The vaccine composition, if desired, can also contain minor amounts of wetting or emulsifying agents, or pH buffering agents. These compositions can take the form of solutions, suspensions, emulsion, tablets, pills, capsules, powders, sustained-release formulations and the like.

For prophylactic treatment against *Francisella tularensis* infection, the monoclonal antibody or the vaccine composition can be administered prior to exposure of a subject to the bacteria so that the resulting immune response can inhibit or reduce the severity of the bacterial infection such that the bacteria can be eliminated from said subject.

Also, the monoclonal antibody or the vaccine composition can be administered post infection or after presumed infection, exposure or manifestation of clinical symptoms. In an aspect thereof, the monoclonal antibody or the vaccine composition can be administered in a time period up to 8 hours post infection. Alternatively, the monoclonal antibody combination is administered in a time period up to 24 hours post infection. In a further alternative, the monoclonal antibody combination is administered in a time period up to 48 hours post infection.

The monoclonal antibody or the vaccine composition may be administered, including as a single dose or in multiple sequential doses, up to 8 hours post infection (8hpi), 12hpi, 18hpi, 24hpi, 36hpi, 48hpi, 72hpi, 1 day post infection, 2 days post infection, 3 days post infection, 4 days post infection, 5 days post infection, 6 days post infection 7 days post infection, a week post infection, 10 days post infection, 2 weeks post infection, 3 weeks post infection, 4 weeks post infection, a month post infection, months post infection.

Various delivery systems are known and can be used to administer the monoclonal antibody. Methods of introduction include but are not limited to intradermal, intramuscular, intraperitoneal, intravenous, subcutaneous, intranasal, epidural, intrarectal, and oral routes. The therapeutic agent can be administered, for example by infusion or bolus injection, by absorption through epithelial or mucocutaneous linings (e.g., oral mucosa, rectal and intestinal mucosa, and the like).

### Immunological assay

A further embodiment of the present invention is directed to a saccharide according to the present invention for use as marker in immunological assays for detection of antibodies against *Francisella tularensis.* Such assays comprise, for instance, microarray and ELISA.

Hence, the inventive saccharide can be used for diagnosis of diseases caused by *Francisella tularensis.* An assay conducted for diagnostic purposes according to the invention may be an immune assay like a solid-phase enzyme immunoassay (EIA), an enzyme linked immunosorbent assay (ELISA), especially an "indirect" ELISA or a radioimmune assay (RIA). Preferably, the saccharide according to the present invention is covalently linked on the solid support through an interconnecting molecule. Thus, the saccharide according to the present invention can be covalently linked on the solid support directly or indirectly through the nitrogen atom of the -O-L-NH₂ group. The solid support is preferably selected from the group comprising or consisting of: a glass slide, a microtitre plate, test tubes, microspheres, nanoparticle or beads.

Thus, the saccharides of the present invention can be easily conjugated to solid supports for providing immunological assays useful for detection of antibodies against *Francisella tularensis.* Said solid supports present on their surface a functionality that is prone to react with the amino group of saccharides of general formula (**I**) or with the functional group Y of the interconnecting molecule to provide modified solid supports, presenting on their surface the functional group X of the interconnecting molecule that can further react with the amino group of saccharides of general formula (**I**). In an embodiment according to the present invention the solid supports are microarray slides, which present on their surface a functionality that is prone to react with the functional group Y of the interconnecting molecule to provide modified microarray slides, presenting of their surface the functional group X of the interconnecting molecule. Examples of such microarray slides include, but are not restricted to Corning^{®} epoxide coated slides or Corning^{®} GAPS^{™} II coated slides.

It is particularly preferred that the solid support is a glass slide or a microtitre plate. A microtitre plate or microplate or microwell plate is a flat plate with multiple "wells" used as small test tubes. Typically, a microtitre plate having 6, 24, 96, 384 or even 1536 sample wells can be used. Microplates are produced from many different materials, like polycarbonate for microtitre plate used for PCR. The most common is polystyrene as used for most optical detection microplates. It can be colored white by the addition of titanium dioxide for optical absorbance or luminescence detection or black by the addition of carbon for fluorescent biological assays.

Thus, another aspect of the present invention is directed to a diagnostic test kit for conducting an immunological assay for detection of antibodies against *Francisella tularensis* comprising at least one saccharide of formula (**I**) immobilized on a solid support.

A further embodiment of the present invention is directed to a monoclonal antibody having specificity for the saccharide of general formula (**I**) or a fragment thereof for use as specific binder in immunological assays for detection of *Francisella tularensis* bacteria in a specimen. Such assays comprise, for instance, ELISA (direct ELISA, indirect ELISA, and Sandwich ELISA), lateral flow immunoassay, Western Blot, immunofluorescence assay, immunohistochemistry, flow cytometry, immunomagnetic separation, chemiluminescence immunoassay, and surface plasmon resonance.

Hence, the inventive monoclonal antibody can be used for detection of the presence of *Francisella tularensis* in a specimen. An assay conducted for detection purposes according to the invention may be an immune assay like a solid-phase enzyme immunoassay (EIA), an enzyme linked immunosorbent assay (ELISA), especially an "indirect" ELISA or a radioimmune assay (RIA). Preferably, the monoclonal antibody according to the present invention is covalently linked on the solid support. Preferably, the monoclonal antibody according to the present invention is present in a solution. The solid support is preferably selected from the group comprising or consisting of: a glass slide, a microtitre plate, test tubes, microspheres, nanoparticle or beads.

Thus, another aspect of the present invention is directed to a diagnostic kit for detection of *Francisella tularensis* bacteria in a specimen comprising a monoclonal antibody having specificity for the saccharide of general formula (**I**) or a fragment thereof.

In a preferred embodiment, the diagnostic kit for detection of *Francisella tularensis* bacteria in a specimen comprises a monoclonal antibody having specificity for the saccharide of general formula (**I**) or a fragment thereof on a solid support.

In a preferred embodiment, the diagnostic kit further comprises a detection antibody for visualizing the antigen-antibody interaction. To this extent, the detection antibody has a detection label for labeling the *Francisella tularensis O*-antigen capsular polysaccharide in the specimen. Thus, in a preferred embodiment, the diagnostic kit for detection of *Francisella tularensis* bacteria in a specimen comprises a monoclonal antibody having specificity for the saccharide of general formula (**I**) or a fragment thereof, and a detection antibody having a detection label for labeling the *Francisella tularensis O*-antigen capsular polysaccharide in the specimen.

### Description of the Figures

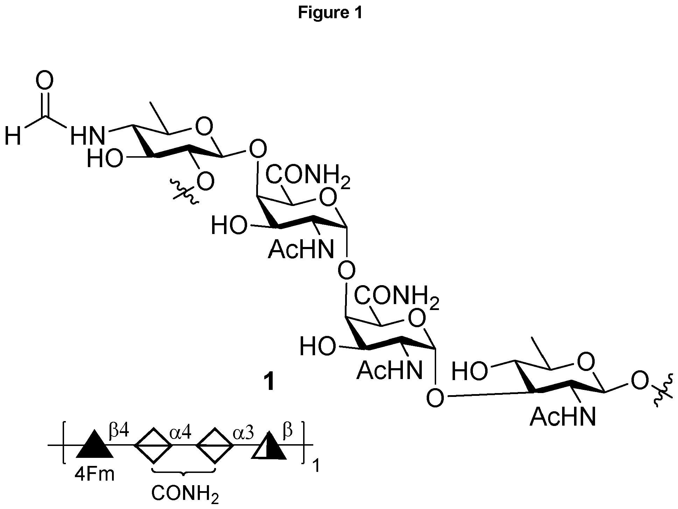

- **Figure 1:**: shows a repeating unit of the O-antigen of *Francisella tularensis* strain 15 O-antigen capsular polysaccharide.
- **Figure 2:**: Commercially available interconnecting molecules .
- **Figure 3:**: Examples of functional group X of the interconnecting molecule.

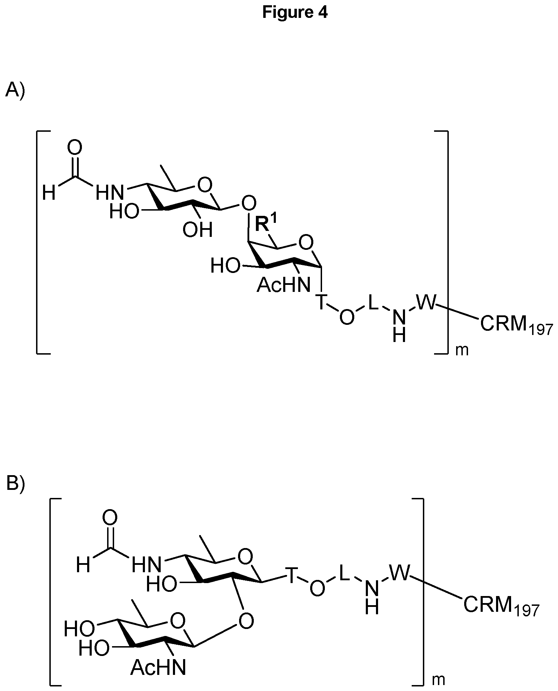

- **Figure 4:**: shows two CRM₁₉₇ conjugates of the present invention.
- **Figure 5:**: shows structures of synthetic *F. tularensis* related glycans tested in glycan microarray analysis
- **Figure 6:**: shows (**A**) printing pattern of glycan microarray and (**B**) exemplary binding pattern of pig serum to immobilized synthetic glycans,
- **Figure 7:**: Mean Fluorescence Intensity of IgG antibody binding to synthetic glycans. The sera are derived from tularemia positive (pos.; dark grey) and negative (neg.; light grey) pigs. A serum dilution of 1:100 was used. Values represent mean ± SEM. Differences were tested for significance to tularemia negative animals using multiple Mann-Whitney test with (***) p < 0.001, (**) p < 0.01 and (*) p < 0.05.
- **Figure 8:**: Glycan microarray analysis of *Francisella tularensis* related glycans. Mean Fluorescence Intensity of IgG antibody binding to synthetic glycans. The sera are derived from tularemia positive (pos.; dark grey) and negative (neg.; light grey) horses (**A**), cats/cheetahs (**B**), dogs (**C**) and rabbits (**D**). A serum dilution of 1:100 was used. Values represent mean ± SEM. Differences were tested for significance to tularemia negative animals using multiple Mann-Whitney test with (****) p < 0.0001 and (*) p < 0.05.
- **Figure 9:**: shows (**A**) MALDI-TOF-MS spectra of **13**-CRM₁₉₇ glycoconjugate and (**B**) MALDI-TOF-MS spectra of **18**-CRM₁₉₇ glycoconjugate,

The following examples are included to demonstrate preferred embodiments of the invention. It should be appreciated by those skilled in the art that the techniques disclosed in the examples, which follow represent techniques discovered by the inventor to function well in the practice of the invention, and thus can be considered to constitute preferred modes for its practice. However, those skilled in the art should, in light of the present disclosure, appreciate that many changes can be made in the specific embodiments, which are disclosed and still obtain a like or similar result.

Further modifications and alternative embodiments of various aspects of the invention will be apparent to those skilled in the art in view of this description. Accordingly, this description is to be construed as illustrative only and is for the purpose of teaching those skilled in the art the general manner of carrying out the invention. It is to be understood that the forms of the invention shown and described herein are to be taken as examples of embodiments. Elements and materials may be substituted for those illustrated and described herein, parts and processes may be reversed, and certain features of the invention may be utilized independently, all as would be apparent to one skilled in the art after having the benefit of this description of the invention.

### Examples

### A. Chemical synthesis

### General Information

All the glassware were dried in the oven prior to reaction. Commercial grade solvents and reagents were used without further purification. Reactions sensitive to moisture were carried out under an atmosphere of nitrogen. Sodium iodide (Nal) used in the reaction was dried at 80 °C under *vacuum.* Anhydrous solvents were obtained from a solvent drying system (JCMeyer) or dried according to reported procedures. Analytical TLC was performed on Kieselgel 60 F254 glass (Macherey-Nagel). Spots were visualized with UV light (λ: 254 nm), sulphuric acid stain [1 mL of 3-methoxyphenol in 1 L of EtOH and 30 mL H₂SO₄] or ceric ammonium molybdate stain [0.5 g Ce(NH₄)₄(SO₄)₄•2H₂O, 12 g (NH₄)₆Mo₇O₂₄•4H₂O and 15 mL H₂SO₄ in 235 mL H₂O]. Flash chromatography was performed on Kieselgel 60 230-400 mesh (Sigma-Aldrich). Preparative HPLC purifications were performed with an Agilent 1200 Series or Agilent 1260 Infinity II. NMR spectra were recorded on a Varian 400 MHz spectrometer (Agilent), Ascend 400 MHz (cryoprobe, Bruker), Ascend 700 MHz (cryoprobe, Bruker) or Varian 600 MHz (Agilent) at 25 °C unless indicated otherwise. Chemical shifts (*δ*) are reported in parts per million (ppm) relative to the respective residual solvent peaks (CDCl₃: *δ* 7.26 in ¹H and 77.16 in ¹³C; D₂O *δ* 4.79 in ¹H). Bidimensional and non-decoupled experiments were performed to assign identities of peaks showing relevant structural features. The following abbreviations are used to indicate peak multiplicities: s (singlet), d (doublet) dd (doublet of doublets), t (triplet), dt (doublet of triplets), td (triplet of doublets), q (quartet), p (pentet), m (multiplet). Additional descriptors b (broad signal) and app (apparent first-order multiplet) are also employed when required. Coupling constants (*J*) are reported in Hertz (Hz). NMR spectra were processed using MestreNova 14.1 (MestreLab Research). High-resolution mass spectra (ESI-HRMS) were recorded with a Xevo G2-XS Q-Tof (Waters).

### Abbreviations

| | |
|---|---|
| Ac | Acetyl |
| AcOH | Acetic acid |
| Ac₂O | Acetic anhydride |
| BAIB | Bisacetyliodobenzene |
| Bn | Benzyl |
| *^{t}*BuOH | *t*-Butanol |
| Bz | Benzoyl |
| CAN | Cericammonium nitrate |
| Cbz | Benzyloxycarbonyl |
| Cu(OAc)₂ | Copper(II) acetate |
| DBU | 1,8-Diazabicyclo[5.4.0]undec-7-ene |
| DCC | *N,N*'-Dicyclohexylcarbodiimide |
| DCM | Dichloromethane |
| DMAP | *N,N*-Dimethylaminopyridine |
| DMF | *N,N*'-Dimethylformamide |
| ESI | Electrosprayionization |
| Et₃N | Triethylamine |
| Et | Ethyl |
| EtOAc | Ethyl acetate |
| FmocCl | 9-Fluorenylmethylchloroformate |
| g | Grams |
| h | Hours |
| HRMS | High resolution mass spectrometry |
| Lev | Levulinyl |
| min | Minute |
| mL | Millilitre |
| Me | Methyl |
| Mel | Methyl iodide |
| MeOH | Methanol |
| MP | *p*-Methoxy phenyl |
| MS | Molecular sieves |
| NaHCO₃ | Sodium bicarbonate |
| NaOH | Sodium hydroxide |
| NaOMe | Sodium methoxide |
| NIS | *N*-Iodo succinimide |
| NMR | Nuclear magnetic resonance |
| Pd/C | Palladium on charcoal |
| Ph | Phenyl |
| Pico | Picoloyl |
| CPS | Capsular polysaccharide |
| Py | Pyridine |
| RT | Room temperature |
| TCA | Trichloroacetamide |
| TEMPO | 2,2,6,6-Tetramethylpiperidinyloxy |
| TfOH | Trifluromethanesulfonic acid |
| TMSOTf | Trimethylsilyltrifluromethanesulfonate |
| THF | Tetrahydrofuran |
| Tol | *p*-Tolyl |

### Example A: Chemical Synthesis of glycans related to F. tularensis

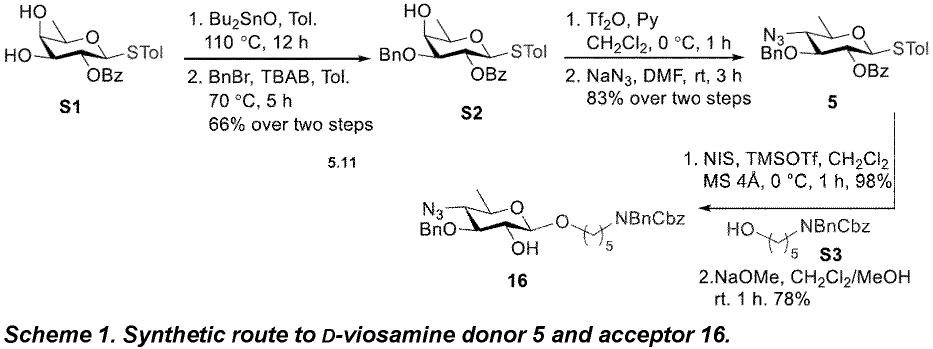

### 4-Methyl phenyl 3-O-benzyl-2-O-benzoyl-6-deoxy-1-thio-β-D-galactopyranoside (S2)

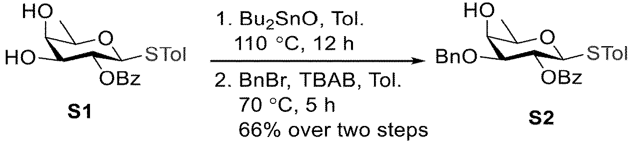

Dibutyltinoxide (Bu₂SnO, 2.7 g, 10.81 mmol) was added to the solution of known compound **S1** (2.7 g, 7.21 mmol) in anhydrous toluene (55 mL) and the reaction was kept stirring at 110 °C for 12 h. After that, the reaction mixture was concentrated under reduced pressure and dried under high *vacuum* for the next step.

The so formed tin acetal compound was dissolved in anhydrous toluene (50 mL). To this solution, tetrabutylammonium bromide (TBAB, 3.48 g, 10.81 mmol) and benzyl bromide (BnBr, 1.3 mL, 10.81 mmol) were added sequentially at room temperature. The reaction was then allowed to stir at 70 °C for 5 h. After completion of the reaction (indicated by TLC), the reaction mixture was diluted with EtOAc (200 mL) and washed with brine solution. The separated organic layer was dried over anhydrous Na2SO4, concentrated and purified by silica gel chromatography (3:7 Ethyl acetate: n-Hexane) to afford compound **S2** (2.21 g, 66% over two steps) as a colorless sticky solid.

¹H NMR (400 MHz, CDCl₃) *δ* 7.83 (dd, *J* = 8.4, 1.3 Hz, 2H, -ArH), 7.38 (ddd, *J* = 7.5, 5.5, 1.3 Hz, 1H, -ArH), 7.24 (t, *J* = 7.7 Hz, 2H, -ArH), 7.16 (d, *J* = 8.2 Hz, 2H, -ArH), 7.00 - 6.87 (m, 5H, -ArH), 6.84 (d, *J* = 8.3 Hz, 2H, -ArH), 5.28 (t, *J* = 9.7 Hz, 1H, H-2), 4.53 - 4.40 (m, 2H, H-1, -*CH*Ph), 4.30 (d, *J* = 12.3 Hz, 1H, -*CH*Ph), 3.73 (d, *J* = 3.9 Hz, 1H, H-4), 3.53 - 3.40 (m, 2H, H-3, H-5), 2.44 (s, 1H, -*OH*), 2.07 (s, 3H, - C*H*₃(STol)), 1.22 (d, *J* = 6.5 Hz, 3H, H-6).

¹³C NMR (101 MHz, CDCl₃) *δ* 165.31, 137.75, 137.06, 133.06, 132.80, 129.90, 129.76, 129.48, 129.41, 128.30, 127.81, 127.74, 86.69 (C-1), 79.56, 77.48, 77.16, 76.84, 74.43, 71.12, 69.62, 68.70, 21.05, 16.71.

HRMS (ESI): calculated for C₂₇H₂₈O₅SNa [M+Na]⁺: 487.1549, found: 487.1550..

### 4-Methyl phenyl 4-azido-3-O-benzyl-2-O-benzoyl-4,6-dideoxy-1-thio-β-D-gluco-pyranoside (5)

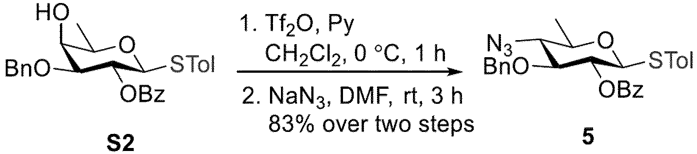

Trifluoromethanesulfonic anhydride (Tf₂O, 1.14 mL, 6.78 mmol) and anhydrous pyridine (1.1 mL, 13.56 mmol) were added dropwise to the stirred solution of compound **S2** (2.1 g, 4.52 mmol) in anhydrous CH₂Cl₂ (21 mL) at 0 °C. The reaction was kept stirring at same temperature for 1 h. After completion of the reaction (monitored by TLC), the reaction mixture was diluted with CH₂Cl₂ (2×30 mL) washed with 1M HCl and aq. NaHCOs solution. The separated organic layer was dried over anhydrous Na₂SO₄ and concentrated under reduced pressure to get triflated compound which was further treated with sodium azide (NaN₃, 1.5 g, 22.6 mmol) in anhydrous DMF (27 mL) at room temperature for 3 h. After completion of reaction (indicated by TLC), the reaction mixture was diluted with EtOAc (2×30 mL) and washed with brine solution. Separated organic layer was dried over anhydrous Na₂SO₄, concentrated and purified by silica gel column chromatography (1:9 Ethyl acetate: *n*-Hexane) to obtain 4-azido-4,6-dideoxy-D-glucose derivative **5** (1.84 g, 83% over two steps) as a white solid.

¹H NMR (400 MHz, CDCl₃) *δ* 8.00 (d, *J* = 7.0 Hz, 2H, -ArH), 7.52 - 7.44 (m, 1H, -ArH), 7.39 - 7.32 (m, 2H, -ArH), 7.29 - 7.23 (m, 2H, -ArH), 7.11 - 7.03 (m, 5H, -ArH), 6.97 (d, *J* = 8.2 Hz, 2H, -ArH), 5.18 (t, *J* = 9.5 Hz, 1H, H-2), 4.66 - 4.58 (m, 2H, H-1, -*CH*Ph), 4.53 (d, *J =* 10.6 Hz, 1H, -*CH*Ph), 3.62 (t, *J* = 9.1 Hz, 1H, H-3), 3.30 - 3.22 (m, 1H, H-5), 3.17 (t, *J* = 9.6 Hz, 1H, H-4), 2.20 (s, 3H, -C*H*₃(STol)), 1.32 (d, *J* = 6.0 Hz, 3H, H-6).

¹³C NMR (101 MHz, CDCl₃) *δ* 165.00, 138.23, 136.88, 133.32, 133.30, 129.78, 129.63, 129.57, 128.48, 128.46, 128.29, 128.24, 127.86, 86.22 (C-1), 82.47, 77.48, 77.16, 76.84, 75.10, 74.93, 72.46, 67.35, 21.08, 18.63.

HRMS (ESI): calculated for C₂₇H₂₇N₃O₄SNa [M+Na]⁺: 512.1614, found: 512.1617.

### N-(Benzyl)benzyloxycarbonyl-5-amino-pentanyl 4-azido-3-O-benzyl-4,6-dideoxy-β-Dglucopyranoside (16)

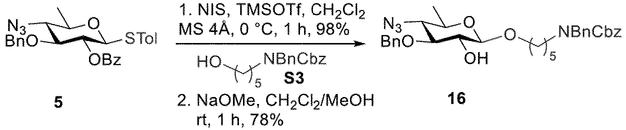

Anhydrous CH₂Cl₂ (7 mL) was added to the mixture of 4-azido-D-viosamine donor **5** (0.202 g, 0.413 mmol), known linker acceptor **S3** (0.09 g, 0.276 mmol) and 4Å molecular sieves (500 mg) and the solution was kept stirring at room temperature for 0.5 h. Then NIS (93 mg, 0.413 mmol) and TMSOTf (5 *µ*L, 0.027 mmol) were added to the stirring solution at 0°C and it was allowed to stir for 1 h. After completion of reaction (consumption of both donor and acceptor were monitored by TLC), Et₃N was added and 4Å molecular sieves was filtered out through celite bed and the reaction mixture was diluted with CH₂Cl₂ (20 mL) and washed with saturated Na₂S₂O₃ solution. The separated organic layer was dried over anhydrous Na₂SO₄, concentrated and purified by silica gel column chromatography (1:4 Ethyl acetate: *n*-Hexane) to give linker coupled D-viosamine compound (0.186 g, 98%) as colorless viscous liquid.

Sodium methoxide (54 mg, 0.99 mmol) was added to the stirring solution of linker coupled D-viosamine compound (0.15 g, 0.217 mmol) in Methanol/CH₂Cl₂ (1/1, *v*/*v*, 5 mL). After 1 h, Amberlite IR120H+ was added to the solution and the reaction mixture was filtered out through celite, concentrated and purified by silica gel column chromatography (3:7 Ethyl acetate: *n*-Hexane) to get debenzoylated compound **16** (0.09 g, 78%) as white foam.

¹H NMR (600 MHz, CDCl₃) *δ* 7.42 - 7.17 (m, 14H, -ArH), 7.17 - 7.06 (m, 1H, -ArH), 5.14 (d, *J =* 19.7 Hz, 2H, -C*H*₂Cbz), 4.97 - 4.88 (m, 1H, -*CH*Ph), 4.80 (d, *J =* 11.0 Hz, 1H, -*CH*Ph), 4.52 - 4.38 (m, 2H, -C*H*₂Ph), 4.15 - 4.05 (m, 1H, H-1*β*), 3.87 - 3.68 (m, 1H, -C*H*(linker)), 3.52 - 3.31 (m, 3H, H-2, H-3, -C*H*(linker)), 3.31 - 3.12 (m, 3H, H-5, - C*H*₂(linker)), 3.09 (t, *J* = 9.6 Hz, 1H, H-4), 1.62 - 1.43 (m, 4H, -C*H*₂(linker)), 1.30 (d, *J* = 6.1 Hz, 3H, H-6), 1.24 (d, *J* = 16.3 Hz, 2H, -C*H*₂(linker)).

¹³CNMR (151 MHz, CDCl₃) *δ* 156.88, 156.43, 138.18, 137.96, 136.97, 136.78, 128.66, 128.56, 128.38, 128.05, 127.97, 127.41, 127.31, 102.83, 102.58, 82.64, 77.37, 77.16, 76.95, 75.12, 75.08, 70.82, 70.17, 69.87, 67.43, 67.33, 50.56, 50.34, 47.10, 46.13, 29.81, 29.29, 29.11, 27.89, 27.35, 23.45, 23.25, 18.60.

HRMS (ESI): calculated for C₃₃H₄₁N₄O₆ [M+H]⁺: 589.3021, found: 589.4294.

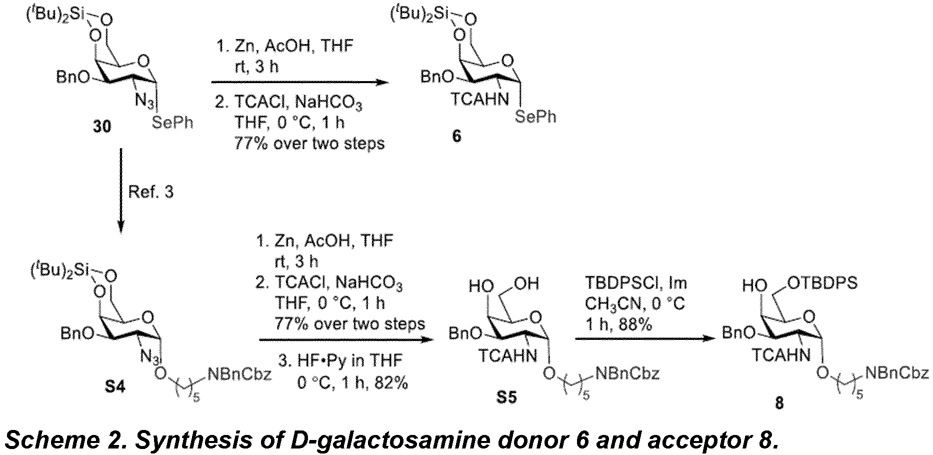

### Phenyl-2-trichloroacetamido-3-O-benzyl-4,6-O-silylidene-2-deoxy-1-seleno-α-D-galactopyranoside (6)

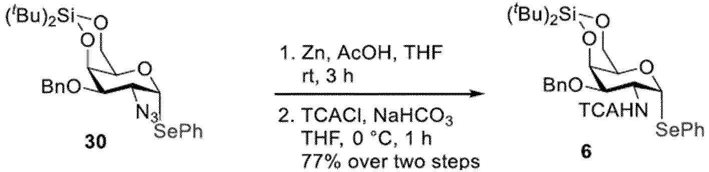

Activated zinc (1.15 g) and acetic acid (AcOH, 1 mL) were added sequentially to the stirring solution of known compound 30 (1.0 g, 2.17 mmol) in THF (10 mL). After 3 h and complete consumption of starting material (indicated by TLC), the reaction mixture was diluted with ethyl acetate (30 mL). Zinc was filtered out through celite bed. Crude amine was directly concentrated under reduced pressure, azeotroped with toluene twice and dried under high vacuum for next step without purification.

The crude amine was dissolved in anhydrous THF (16 mL) and sodium bicarbonate (0.39 g, 4.66 mmol), TCACI (0.8 mL, 6.99 mmol) were added sequentially at 0 °C. The reaction was kept stirring at same temperature for 1 h. After conversion of the starting material, the crude was diluted with ethyl acetate (50 mL) and washed with brine, aq. NaHCOs solution. The separated organic layer was dried over anhydrous Na₂SO₄, concentrated and purified by silica gel column chromatography (1:9 Ethyl acetate: n-Hexane) to give compound **6** (1.25 g, 77% over two steps) as a white solid.

¹H NMR (400 MHz, CDCl₃) δ 7.42 (dd, J = 6.1, 2.0 Hz, 2H), 7.27 (d, J = 4.5 Hz, 4H), 7.24 - 7.19 (m, 1H), 7.19 - 7.13 (m, 3H), 6.80 (d, J = 7.1 Hz, 1H, ), 6.04 (d, J = 4.8 Hz, 1H), 4.71 - 4.63 (m, 2H, -CHPh, H-2), 4.60 (s, 1H, H-4), 4.47 (d, J = 11.9 Hz, 1H, -CHPh), 4.23 (dd, J = 12.7, 2.3 Hz, 1H, H-6a), 4.07 (dd, J = 12.7, 1.7 Hz, 1H, H-6b), 3.95 (s, 1H, H-5), 3.41 (dd, J =11.0, 2.7 Hz, 1H, H-3), 0.97 (d, J = 4.3 Hz, 18H, -C(CH3)3).

¹³C NMR (101 MHz, CDCl₃) δ 161.77, 137.47, 134.46, 129.47, 128.83, 128.61, 128.26, 128.23, 127.92, 92.55, 89.22 (C-1), 77.48, 77.16, 76.84, 76.55, 70.94, 69.73, 69.07, 67.30, 51.03, 27.77, 27.42, 23.55, 20.93.

HRMS (ESI): calculated for C₂₉H₃₈Cl₃NO₅SeSiNa [M+Na]⁺: 716.0642, found: 716.0686.

### N-(Benzyl)benzyloxycarbonyl-5-amino-pentanyl 2-trichloroacetamido-4-O-benzyl-α-Dgalactopyranoside (S5)

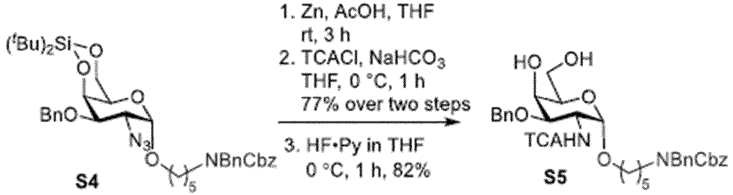

Activated zinc dust (900 mg) and AcOH (0.6 mL) were added sequentially to the stirring solution of known compound **S4** (285 mg, 0.382 mmol) in THF (6 mL). After 3 h and complete conversion of azide to amine, zinc dust was removed by filtering through a celite bed. The crude was then concentrated under reduced pressure, azeotroped with toluene twice and dried under high *vacuum* for next step without purification.

The crude amine was dissolved in anhydrous THF (3 mL) and sodium bicarbonate (NaHCOs, 78 mg, 1.14 mmol), trichloroacetyl chloride (TCACI, 0.13 mL, 1.14 mmol) were added sequentially at 0 °C. The reaction was kept stirring at same temperature for 1 h. After conversion of starting material, crude was diluted with ethyl acetate (10 mL) and washed with brine, aq. NaHCOs solution. The separated organic layer was dried over anhydrous Na₂SO₄, concentrated and purified by silica gel column chromatography (3:7 Ethyl acetate: *n*-Hexane) to give linker coupled D-galactosamine derivative (254 mg, 77% over two steps) as a sticky white solid.

¹H NMR (400 MHz, CDCl₃) *δ* 7.45 - 7.34 (m, 14H, -ArH), 7.26 - 7.25 (m, 1H, -ArH), 6.91 - 6.80 (m, 1H, -N*H*), 5.26 (d, *J* = 13.7 Hz, 2H, -C*H*₂Cbz), 5.08 (s, 1H, H-1*α*), 4.83 (dd, *J* = 12.2, 3.3 Hz, 1H, -CHPh), 4.69 (d, *J* = 8.6 Hz, 3H, H-2, H-5, -CHPh), 4.57 (s, 2H, -C*H*2Ph), 4.35 (d, *J* = 13.0 Hz, 1H, H-6a), 4.24 (d, *J* = 13.1 Hz, 1H, H-6b), 3.78 - 3.64 (m, 2H, H-3, H-4), 3.55 - 3.36 (m, 2H, -C*H*₂(linker)), 3.36 - 3.16 (m, 2H, -C*H*₂(linker)), 1.69-1.62 (m, 4H, -C*H*₂(linker)), 1.38-1.28 (m, 2H, -C*H*₂(linker)), 1.17 (s, 9H, -C(C*H*₃)₃), 1.16 (s, 9H, -C(C*H*₃)₃).

¹³C NMR (151 MHz, CDCl₃) *δ* 161.85, 156.35, 138.19, 137.98, 128.72, 128.61, 128.12, 127.98, 127.88, 127.77, 127.51, 127.35, 97.19 (C-1*a*), 92.93, 77.37, 77.16, 77.04, 76.95, 75.46, 70.84, 70.35, 70.03, 69.93, 69.78, 68.25, 67.87, 67.41, 67.35, 64.48, 50.44, 50.10, 47.21, 46.22, 45.13, 29.11, 27.80, 27.50, 26.66, 25.68, 23.59, 20.90.

HRMS (ESI): calculated for C₄₃H₅₇Cl₃N₂O₈SiNa [M+Na]⁺: 885.2842, found: 885.2913. To a solution of above formed linker coupled compound (126 mg, 0.146 mmol) in THF (3 mL), was added dropwise solution of HF•Py (70% in THF, 0.03 mL) at 0 °C. After 1 h, Et₃N was added and crude was directly concentrated under reduced pressure, purified by silica gel column chromatography (1:1 ethyl acetate: n-Hexane to 100% ethyl acetate) to get diol compound **S5** (86 mg, 82%) as white foam.

¹H NMR (400 MHz, CDCl₃) *δ* 7.37 (d, *J* = 4.5 Hz, 1H, -ArH), 7.31 (t, *J* = 4.5 Hz, 10H, -ArH), 7.28 - 7.22 (m, 3H, -ArH), 7.17 (d, *J* = 7.0 Hz, 1H, -ArH), 6.92 - 6.71 (m, 1H, -N*H*), 5.20-5.11 (m, 2H, -C*H*₂Cbz), 4.92-4.87 (m, 1H, H-1*a*GalN), 4.70 (d, *J =* 11.9 Hz, 1H, -C*H*Ph), 4.58 - 4.43 (m, 4H, H-2, -C*H*Ph, -C*H*₂Ph), 4.21 (d, *J* = 2.9 Hz, 1H, H-4), 3.91 (dd, *J* = 11.3, 5.9 Hz, 1H, H-6a), 3.86 - 3.80 (m, 2H, H-5, H-6b), 3.75 - 3.67 (m, 2H, H-3, -C*H*(linker)), 3.67 - 3.56 (m, 1H, -C*H*(linker)), 3.43 - 3.35 (m, 1H, -O*H*), 3.31 (tt, *J* = 9.4, 5.5 Hz, 1H, -C*H*(linker)), 3.19 (q, *J* = 6.5 Hz, 2H, -C*H*(linker), -O*H*), 1.53 (ddd, *J* = 32.4, 15.1, 7.9 Hz, 4H, -C*H*₂(linker)), 1.35 - 1.26 (m, 2H, -C*H*₂(linker)).

¹³C NMR (101 MHz, CDCl₃) *δ* 161.87, 156.78, 156.36, 137.77, 137.32, 136.83, 136.54, 128.64, 128.56, 128.50, 128.08, 128.03, 127.88, 127.84, 127.80, 127.72, 127.43, 127.24, 96.95 (C-1*α*), 92.70, 77.50, 77.39, 77.18, 76.87, 76.09, 71.17, 70.04, 69.88, 67.87, 67.32, 66.19, 62.43, 60.50, 50.56, 50.32, 47.16, 46.09, 28.97, 28.76, 27.87, 27.15, 23.50, 21.13.

HRMS (ESI): calculated for C₃₅H₄₁Cl₃N₂O₈Na [M+Na]⁺: 745.1820, found: 745.1926.

### N-(Benzyl)benzyloxycarbonyl-5-amino-pentanyl 2-trichloroacetamido-2-deoxy-3-O-benzyl-6-O-tert-butyldiphenylsilyl-a-D-galactopyranoside (8)

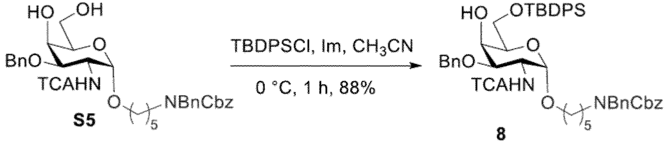

Imidazole (Im, 21 mg, 0.304 mmol) and *tert*-butyldiphenylsilylchloride (TBDPSCI, 0.07 mL, 0.276 mmol) were added sequentially to the stirring solution of diol **S5** (100 mg, 0.138 mmol) in CH₃CN (4 mL) at 0 °C. After 1 h and complete consumption of starting material (monitored by TLC), the crude reaction mixture was diluted with EtOAc (20 mL) and washed with brine solution.

The separated organic layer was dried over anhydrous Na₂SO₄, concentrated and purified by silica gel column chromatography (3:7 Ethyl acetate: *n*-Hexane) to afford 4-OH acceptor **8** (117 mg, 88%) as a white sticky solid. 1H NMR (400 MHz, CDCl₃) *δ* 7.62 - 7.60 (m, 4H, -ArH), 7.38 -7.29 (m, 7H, -ArH), 7.28-7.23 (m, 10H, -ArH)), 7.21-7.20 (m, 3H, -ArH)), 7.10 (d, *J* = 7.6 Hz, 1H, -ArH), 6.80-6.62 (m, 1H, -N*H*), 5.10 (d, *J* = 18.1 Hz, 2H, -C*H*2Cbz), 4.81-4.77 (m, 1H, H-1*a*), 4.62 (d, *J =* 12.0 Hz, 1H, -C*H*Ph), 4.53 (t, *J* = 12.7 Hz, 1H, -C*H*Ph), 4.47 - 4.32 (m, 3H, H-2, -C*H*₂Ph), 4.06 (s, 1H, H-4), 3.87 (dd, *J* = 10.5, 6.1 Hz, 1H, -C*H*(linker)), 3.79 (dd, *J* = 10.5, 5.8 Hz, 1H, -C*H*(linker)), 3.70 - 3.42 (m, 3H, H-3, H-5, H-6a), 3.29 - 3.03 (m, 3H, H-6b, -C*H*₂(linker)), 2.47 (s, 1H, -O*H*), 1.49 - 1.35 (m, 4H, -C*H*₂(linker)), 1.23-1.15 (m, 2H, -C*H*₂(linker)), 0.99 (s, 9H, -C(C*H*₃)₃).

¹³C NMR (101 MHz, CDCl₃) *δ* 161.72, 156.79, 156.31, 137.91, 137.52, 136.75, 135.76, 135.71, 133.37, 133.25, 129.93, 128.76, 128.72, 128.58, 128.21, 128.09, 127.94, 127.86, 127.81, 127.49, 127.31, 96.86 (C-1*a*), 92.84, 77.48, 77.36, 77.16, 76.96, 76.84, 76.49, 71.48, 70.40, 67.93, 67.33, 65.69, 63.30, 50.68, 50.37, 47.16, 46.18, 29.84, 28.93, 28.00, 27.47, 26.96, 23.75, 23.43, 19.34.

HRMS (ESI): calculated for C₅₁H₅₉Cl₃N₂O₈SiNa [M+Na]⁺: 985.3004, found: 985.3055.

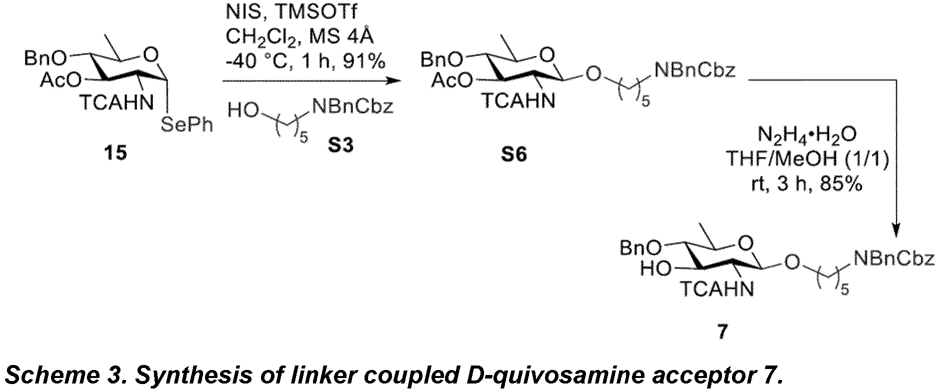

### N-(Benzyl)benzyloxycarbonyl-5-amino-pentanyl 2-trichloroacetamido-3-O-acetyl-4-O-benzyl-β-D-glucopyranoside (S6)

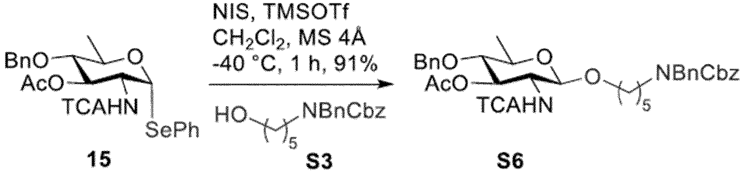

Anhydrous CH₂Cl₂ (37 mL) was added to the mixture of D-quinovosamine donor **15** (1.3 g, 2.24 mmol), linker acceptor **S3** (0.489 g, 1.49 mmol) and 4Å molecular sieves (2.0 g) and the solution was kept for stirring at room temperature for 0.5 h. Then NIS (0.505 g, 2.24 mmol) and TMSOTf (41 *µ*L, 0.224 mmol) were added to the stirring solution at -40 °C and it was stirred for 1 h. After completion of reaction (consumption of both donor and acceptor were monitored by TLC), Et₃N was added and 4Å molecular sieves were filtered through a celite bed and the reaction mixture was diluted with CH₂Cl₂ (30 mL) and washed with saturated Na₂S₂O₃ solution. The separated organic layer was dried over anhydrous Na₂SO₄, concentrated and purified by silica gel column chromatography (3:7 Ethyl acetate: *n*-Hexane) to give linker coupled D-quinovosamine compound **S6** (1.01 g, 91%) as a white solid.

¹H NMR (400 MHz, CDCl₃) *δ* 7.38-7.35 (m, 2H, -ArH), 7.34-7.32 (m, 1H, -ArH), 7.32-7.28 (m, 6H, -ArH), 7.28-7.27 (m, 1H, -ArH), 7.26-7.25 (m, 2H, -ArH), 7.24-7.23 (m, 1H, -ArH), 7.17 - 7.04 (m, 2H, -ArH), 5.40 - 5.22 (m, 1H, H-3), 5.18 (d, *J =* 11.0 Hz, 2H, -C*H*₂Cbz), 4.73 - 4.57 (m, 2H, -C*H*₂Ph), 4.52 - 4.42 (m, 2H, -C*H*₂Ph), 4.37-4.28 (m, 1H, H-1*β*), 3.98 (q, *J* = 9.2 Hz, 1H, H-2), 3.81 - 3.67 (m, 1H, -C*H*(linker)), 3.52 - 3.42 (m, 1H, H-5),3.29 (t, *J* = 9.2 Hz, 1H, H-4), 3.26 - 3.15 (m, 3H, -C*H*₂(linker), -C*H*(linker)), 1.97 (s, 3H, -COC*H*₃), 1.57 - 1.41 (m, 4H, -C*H*₂(linker)), 1.30 (d, *J* = 6.1 Hz, 3H, H-6), 1.26-1.20 (m, 2H, -C*H*₂(linker)).

¹³C NMR (101 MHz, CDCl₃) *δ* 171.45, 162.16, 156.81, 156.27, 137.97, 137.66, 137.28, 136.83, 128.77, 128.64, 128.53, 128.20, 128.09, 128.06, 127.98, 127.93, 127.38, 127.30, 100.67 (C-1*β*_{Quip}), 92.66, 81.75, 77.48, 77.36, 77.16, 76.84, 75.40, 74.46, 71.38, 69.71, 67.24, 56.12, 50.62, 50.36, 47.21, 46.26, 29.12, 27.97, 27.43, 23.26, 20.96, 17.89.

HRMS (ESI): calculated for C₃₇H₄₃Cl₃N₂O₈Na [M+Na]⁺: 771.1977, found: 771.2029.

### N-(Benzyl)benzyloxycarbonyl-5-amino-pentanyl 2-trichloroacetamido-4-O-benzyl-β-D-glucopyranoside (7)

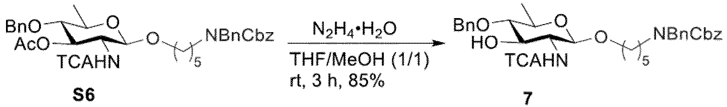

Hydrazine hydrate (6 mL) was added to the stirring solution of compound **S6** (0.93 g, 1.24 mmol) in THF/Methanol (1/1, *v*/*v,* 20 mL). After 3 h, the crude was diluted with ethyl acetate (50 mL) and washed with brine solution (2×10 mL). The separated organic layer was dried over anhydrous Na₂SO₄, concentrated and purified by silica gel column chromatography (3:7 Ethyl acetate: *n*-Hexane) to get deacetylated compound **7** (0.746 g, 85%) as white foam.

¹H NMR (400 MHz, CDCl₃) *δ* 7.49 - 7.32 (m, 13H, -ArH), 7.26 (d, *J* = 7.6 Hz, 2H, -ArH), 7.05 (bs, 1H, -N*H*), 5.26 (d, *J* = 11.7 Hz, 2H, -C*H*₂Cbz), 4.93 (d, *J =* 11.3 Hz, 1H, -C*H*Ph), 4.83 (dd, *J* = 11.2, 2.4 Hz, 1H, -C*H*Ph), 4.74-4.69 (m, 1H, H-1*β*), 4.57 (s, 2H, -C*H*₂Ph), 4.16 (t, *J* = 7.7 Hz, 1H, H-3), 3.91 (dd, *J* = 21.8, 9.1 Hz, 1H, -C*H*(linker)), 3.65 - 3.42 (m, 3H, H-2, H-5, -C*H*(linker)), 3.38 - 3.09 (m, 4H, H-4, -C*H*₂(linker), -OH), 1.61 (dt, *J* = 16.0, 7.9 Hz, 4H, -C*H*₂(linker)), 1.45 (d, *J* = 6.7 Hz, 3H, H6), 1.41 - 1.24 (m, 2H, -C*H*₂(linker)).

¹³C NMR (100 MHz, CDCl₃) *δ* 162.47, 156.76, 156.25, 138.07, 137.86, 136.72, 128.60, 128.58, 128.48, 128.10, 128.05, 127.98, 127.87, 127.35, 99.40 (C-1*β*), 92.60, 84.05, 77.41, 77.39, 77.30, 77.10, 77.07, 76.78, 76.76, 75.09, 73.21, 71.37, 69.58, 67.23, 59.26, 50.56, 50.29, 47.13, 46.17, 29.73, 29.07, 27.87, 27.33, 23.29, 18.23.

HRMS (ESI): calculated for C₃₅H₄₁Cl₃N₂O₇Na [M+Na]⁺: 729.1871, found: 729.1728.

### N-(Benzyl)benzyloxycarbonyl-5-amino-pentanyl 4-azido-3-O-benzyl-2-O-benzoyl-4,6-dideoxy-β-D-glucopyranosyl-(1 → 4)-2-trichloroacetamido-2-deoxy-3-O-benzyl-6-O-tertbutyldiphenylsilyl-α-D-galactopyranoside (9)

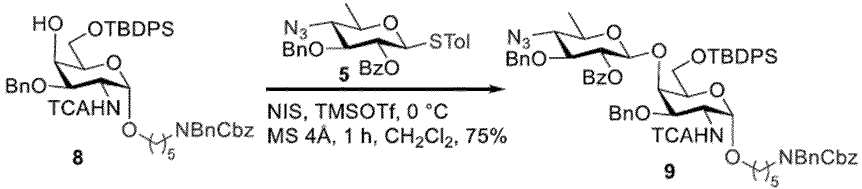

Anhydrous CH₂Cl₂ (2.63 mL) was added to the mixture of 4-azido-D-viosamine donor **5** (76 mg, 0.16 mmol), 4-OH-D-galactosamine acceptor **8** (100 mg, 0.104 mmol) and 4Å molecular sieves (100 mg) and the solution was stirred at room temperature for 0.5 h. Then NIS (35 mg, 0.159 mmol) and TMSOTf (2 *µ*L, 0.0104 mmol) were added to the stirring solution at 0 °C and it was allowed to stir for 1 h. After completion of reaction (consumption of both donor and acceptor were monitored by TLC), Et₃N was added and 4Å molecular sieves were removed by filtering through a celite bed and the reaction mixture was diluted with CH₂Cl₂ (10 mL) and washed with saturated Na₂S₂O₃ solution. The separated organic layer was dried over anhydrous Na₂SO₄, concentrated and purified by silica gel column chromatography (0.5:0.5:9 Ethyl acetate: CH₂Cl₂: Toluene) to give disaccharide compound **9** (103 mg, 75%) as colorless viscous liquid.

¹H NMR (600 MHz, CDCl₃) *δ* 8.10-8.08 (m, 2H, -ArH), 7.67-7.64 (m, 4H,-ArH), 7.51-7.48 (m, 1H, -ArH), 7.44-7.36 (m, 7H, -ArH), 7.35-7.28 (m, 7H, -ArH), 7.26-7.24 (m, 5H, -ArH), 7.24-7.16 (m, 8H, -ArH), 7.14-7.13 (m, 1H, -ArH) 6.58-6.46 (m, 1H, -N*H*) 5.18-5.13 (m, 3H, H-2Vio, -C*H*₂Cbz)), 5.01 (d, *J* = 12.0 Hz, 1H, H-1*β*^{Vio}), 4.67-4.61 (dd, *J* = 18.0, 6.0 Hz, 2H, -C*H*₂Ph), 4.57-4.44 (m, 5H, H-1*α*GalN, -C*H*₂Ph, -C*H*₂Ph), 4.28 (s, 1H, H-4^{GalN}), 4.23-4.20 (m, 1H, H-2^{GalN}), 3.89-3.86 (m, 1H, H-6a^{GalN}), 3.74-3.71 (m, 1H, H-6b^{GalN}), 3.64-3.41 (m, 4H, H-4^{Vio}, H-3^{Vi0}, H-3^{GalN}, -C*H*(linker)), 3.19-3.04 (m, 5H, H-5^{Vio}, H-5^{GalN}, -C*H*(linker), -C*H*₂(linker)), 1.48-1.33 (m, 4H, -C*H*₂(linker)), 1.20-1.10 (m, 5H, H-6^{Vio}, -C*H*₂(linker)), 1.04 (s, 9H, -C(C*H*₃)₃).

¹³C NMR (151 MHz, CDCl₃) *δ* 165.45, 161.07, 156.31, 137.95, 137.39, 135.71, 133.77, 132.91, 130.25, 130.09, 129.86, 129.82, 128.71, 128.61, 128.58, 128.45, 128.42, 128.14, 127.97, 127.94, 127.80, 127.58, 127.32, 99.55 (C-1*β*^{Vio}, 1*J*_{C-H} = 161.0 Hz), 96.45 (C-1*α*^{GalN}, 1*J*_{C-H} = 168.0 Hz), 92.94, 81.25, 77.64, 77.37, 77.29, 77.16, 76.95, 75.00, 74.56, 71.75, 71.59, 70.73, 69.65, 67.67, 67.32, 63.11, 51.00, 50.69, 50.37, 47.19, 46.20, 29.85, 28.97, 27.03, 23.64, 19.40, 18.47.

HRMS (ESI): calculated for C₇₁H₇₈Cl₃N₅O₁₂SiNa [M+Na]⁺: 1348.4374, found: 1348.4565.

### N-(Benzyl)benzyloxycarbonyl-5-amino-pentanyl 4-azido-3-O-benzyl-2-O-benzoyl-4,6- dideoxy-β-D-glucopyranosyl-(1→4)-2-trichloroacetamido-2-deoxy-3-O-benzyl-α-Dgalactopyranoside (10)

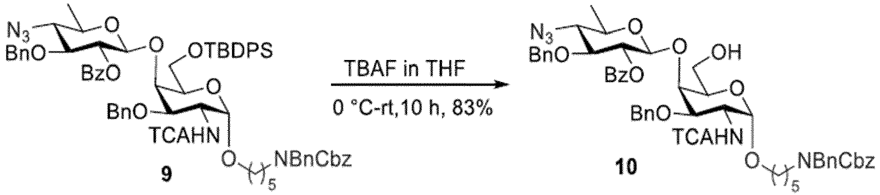

Tetrabutylammoniumfluoride (TBAF, 1M in THF solution, 2.73 mL) was added to the stirring solution of compound **9** (0.32 g, 0.241 mmol) in THF (7.5 mL) at 0 °C. The reaction mixture was then slowly warm up to room temperature for 10 h. After complete consumption of the starting material (monitored by TLC), the crude was directly concentrated under reduced pressure and purified by silica gel chromatography (100% Ethyl acetate) to get compound **10** (0.218 g, 83%) as a colorless sticky compound.

¹H NMR (400 MHz, CDCl₃) *δ* 8.07 (dd, *J* = 8.3, 1.3 Hz, 2H), 7.60 - 7.54 (m, 1H), 7.44 (d, *J* = 7.9 Hz, 2H), 7.43 - 7.39 (m, 5H), 7.38 - 7.33 (m, 5H), 7.31-7.23 (m, 10H), 6.45 (dd, *J* = 65.6, 8.3 Hz, 1H), 5.32 (t, *J* = 8.0 Hz, 1H), 5.22 (d, *J* = 22.1 Hz, 2H), 4.85 (d, *J* = 7.9 Hz, 2H), 4.77 - 4.61 (m, 3H), 4.59 - 4.49 (m, 3H), 4.30 (s, 1H), 4.17 - 4.06 (m, 1H), 3.90 - 3.77 (m, 2H), 3.72-3.62 (m, 3H), 3.65 - 3.49 (m, 1H), 3.39 - 3.23 (m, 4H), 3.22 - 2.99 (m, 2H), 1.57-1.45 (m, 7H), 1.34 - 1.18 (m, 2H).

¹³C NMR (101 MHz, CDCl₃) *δ* 165.47, 160.80, 156.71, 156.24, 137.85, 137.54, 137.43, 137.03, 136.67, 132.97, 130.11, 129.70, 129.10, 128.71, 128.62, 128.49, 128.41, 128.38, 128.34, 128.22, 127.99, 127.91, 127.84, 127.40, 127.24, 101.30 (C-1*β*^{Vio}), 96.48 (C-1*α*^{GalN}), 92.68, 80.90, 77.48, 77.36, 77.16, 76.84, 76.06, 75.02, 73.92, 72.12, 71.28, 71.00, 69.57, 68.58, 68.06, 67.24, 67.10, 60.37, 51.11, 50.58, 50.29, 47.13, 46.09, 29.77, 28.95, 28.83, 27.86, 27.27, 23.57, 23.25, 22.23, 18.36.

HRMS (ESI): calculated for C₅₅H₆₀Cl₃N₅O₁₂Na [M+Na]⁺: 1110.3196 found: 1110.3295.

### N-(Benzyl)benzyloxycarbonyl-5-amino-pentanyl 4-azido-3-O-benzyl-2-O-benzoyl-4,6-dideoxy-β-D-glucopyranosyl-(1 → 4)-2-trichloroacetamido-2-deoxy-3-O-benzyl-6-pentafluorophenyl-α-D-galactopyrano uronaside (11)

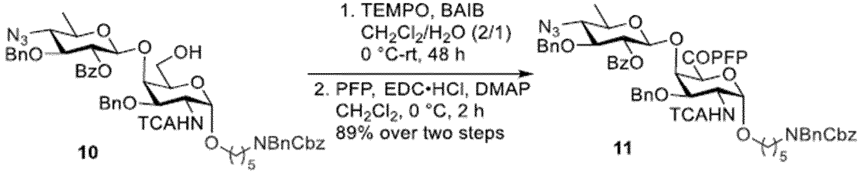

2,2,6,6-Tetramethylpiperidinyloxyl (TEMPO, 11 mg, 0.068 mmol), (diacetoxyiodo)benzene (BAIB, 0.131 g, 0.408 mmol) were added sequentially to a stirring solution of compound **10** (0.160 g, 0.147 mmol) in CH₂Cl₂ (7.3 mL) and H₂O (3.5 mL) at 0 °C. After 1 h, another batch of BAIB (0.131 g, 0.408 mmol) was added followed by addition of excess H₂O (2 mL) at 0 °C. The mixture was stirred at room temperature for 48 h. After complete conversion of alcohol to acid via aldehyde formation (monitored by TLC), ethyl acetate (50 mL) was added to it and the crude was washed with sat. NaHCOs solution. The separated organic layer was dried over anhydrous Na₂SO₄, concentrated and dried under high *vacuum* to get crude carboxylate compound as yellowish liquid.

To *vacuum* dried crude carboxylate compound in anhydrous CH₂Cl₂ (10 mL), pentafluorophenol (PFPOH, 40 mg, 0.218 mmol) and catalytic amount of 4-dimethylaminopyridine (DMAP, 8.3 mg, 0.068 mmol) were added sequentially at 0 °C. After that, (3-dimethylamino-propyl)-ethylcarbodiimide hydrochloride (EDC•HCl, 91 mg, 0.476 mmol) was added and the reaction mixture was stirred at same temperature for 2 h. After completion of reaction (monitored by TLC), the crude reaction mixture was diluted with CH₂Cl₂ (20 mL) and washed with saturated NaHCOs and brine solution. The separated organic layer was dried over anhydrous Na2SO4, concentrated and purified by silica gel column chromatography (1:4 Ethyl acetate: *n*-Hexane) to obtain 6-O disaccharide ester compound **11** (0.166 g, 89% over two steps) as a white solid. ¹H NMR (400 MHz, CDCl₃) *δ* 8.18 (dd, *J* = 8.4, 1.4 Hz, 2H, -ArH), 7.61-7.57 (m, 1H, -ArH), 7.50-7.46 (m, 2H, -ArH), 7.42 - 7.39 (m, 4H, -ArH), 7.38 - 7.36 (m, 4H, -ArH), 7.34 - 7.29 (m, 5H, -ArH), 7.28 - 7.20 (m, 7H, -ArH), 6.59 (dd, *J* = 62.4, 8.5 Hz, 1H, -N*H*), 5.29 - 5.14 (m, 4H, H-1*β*^{Vio}, H-2^{Vio}, C*H*₂Cbz), 4.93 - 4.88 (m, 2H, H-1*α*^{GalN}, H-5^{GalN}), 4.75 - 4.60 (m, 5H, H-4^{GalN}, -C*H*₂Ph), 4.54 (d, *J* = 7.1 Hz, 2H, -C*H*2Ph), 4.42 (d, *J* = 7.5 Hz, 1H, H-2^{GalN}), 3.91 - 3.79 (m, 1H, H-3^{GalN}), 3.73 - 3.60 (m, 2H, H-3^{Vi0}, -C*H*(linker)), 3.46-3.34 (m, 1H, -C*H*(linker)), 3.31-3.21 (m, 3H, H-4^{Vio}, -C*H*₂(linker)), 3.16-3.10 (m, 1H, H-5^{Vio}), 1.62 - 1.46 (m, 4H, -C*H*₂(linker)), 1.27-1.21 (m, 5H, H6^{Vio}, -C*H*₂(linker)).

¹³C NMR (101 MHz, CDCl₃) *δ* 165.19, 163.91, 161.32, 156.79, 156.34, 142.31, 137.84, 137.16, 133.03, 130.21, 129.94, 128.84, 128.70, 128.56, 128.53, 128.49, 128.43, 128.01, 127.88, 127.61, 99.31 (C-1*β*^{Vio}), 97.03 (C-1*α*^{GalN}), 92.64, 81.22, 77.48, 77.36, 77.16, 77.03, 76.84, 76.23, 75.10, 74.26, 71.89, 70.85, 70.79, 70.38, 69.15, 68.99, 67.33, 50.36, 47.08, 29.84, 28.83, 27.33, 23.53, 18.37.

HRMS (ESI): calculated for C₆₁H₅₇Cl₃F₅N5O₁₃ Na [M+Na]⁺: 1290.2831 found: 1290.2983.

### 5-Amino-pentanyl 4-N-formamido-4,6-dideoxy- β -D-glucopyranosyl-(1 → 4)-2-acetamido-2- deoxy-o-D-galactouronamide (12)

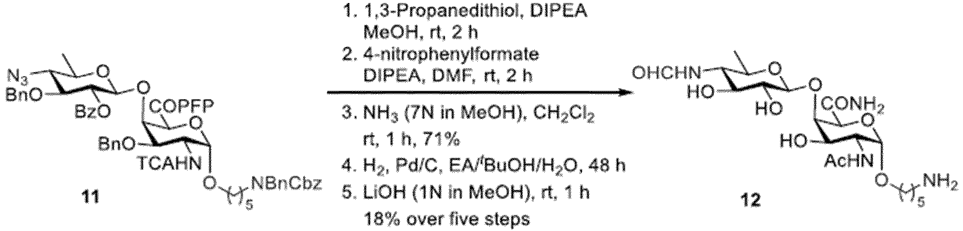

1,3-Propanedithiol (0.5 mL, 4.9 mmol) was added to the stirring solution of disaccharide **11** (0.13 g, 0.102 mmol) in diisopropylethylamine (DIPEA, 0.17 mL) and methanol (7.5 mL). After 2 h stirring at room temperature, the crude residue was directly concentrated under reduced pressure and co-evaporated with toluene twice. The concentrated residue was dried under high *vacuum* for next step.

The crude amine residue was dissolved in anhydrous DMF (9.76 mL). 4-Nitrophenyl-formate (85 mg, 0.51 mmol) was added followed by dropwise addition of DIPEA (0.2 mL, 1.02 mmol). After 2 h and complete consumption of amine (indicated by TLC), the reaction mixture was diluted with ethyl acetate (30 mL) and washed with brine solution three times. The separated organic layer was dried over anhydrous Na₂SO₄, concentrated to obtain *N*-formamido disaccharide compound as sticky solid. Above prepared compound was dissolved in CH₂Cl₂ (1 mL). After that ammonia solution (7 N in MeOH, 3.5 mL) was added to the solution in dropwise at room temperature. After 2 h and complete consumption of the starting material (indicated by TLC), the crude was directly concentrated under reduced pressure to get amide compound as viscous liquid.

The obtained amide compound was dissolved in EA/*t*BuOH/H₂O (2/1/1, *v*/*v*/*v,* 4 mL). To this solution a suspension of Pd/C (60 mg) was added and stirred under hydrogen atmosphere (1atm, balloon) for 48 h. The reaction mixture was then filtered out through Celite^{®} 353, concentrated to get desired mono benzoylated disaccharide compound.

To the crude mono benzoylated compound, 3 mL LiOH (1N in methanol) was added. The solution was stirred at room temperature for 1 h. After complete consumption of the starting material (monitored by mass-spectrometry), Amberlite IR120H+ was added. The reaction mixture was then filtered out, concentrated and purified by HPLC (Hypercarb Column, 150×10 mm at a flow rate of 3 mL/min, eluted by H₂O (with 0.1% formic acid, Solvent A) and acetonitrile (Solvent B) with a linear gradient from 0% to 30% of solvent B over 30 minutes and lyophilized to get disaccharide **12** (3.52 mg, 18% over five steps) as a white powder.

¹H NMR (600 MHz, D₂O) *δ* 8.15 (s, 1H, -NHC*H*O), 7.97 (s, -NHC*H*O), 4.98 (d, *J* = 3.8 Hz, 1H, H-1*α*GalN), 4.89 (d, *J* = 3.8 Hz, 1H, H-1*α*GalN), 4.58-4.56 (d, *J* = 6.0 Hz, 1H, H-1*β*^{Vio}), 4.48 - 4.44 (m, 3H, H-1*β*^{Vio}, H-5^{GalN}), 4.36 (s, 1H, H-4^{GalN}), 4.25-4.20 (m, 1H, H-2^{GalN}), 4.18 (s, 1H, H-4^{GalN}), 4.00-3.96 (m, 1H, H-3^{GalN}), 3.68 - 3.61 (m, 1H, -C*H*(linker)), 3.59 (t, *J* = 9.9 Hz, 1H, H-4^{Vio}), 3.53 - 3.41 (m, 3H, H-3^{Vio}, H-5^{Vio}, -C*H*(linker)), 3.36 (dt, *J* = 9.3, 7.3 Hz, 1H, H-2^{Vio}), 2.95-2.92 (m, 2H, -C*H*₂(linker)), 2.00 (d, *J* = 2.9 Hz, 3H, -NHCOC*H*₃), 1.66 - 1.54 (m, 4H, -C*H*₂(linker), -C*H*₂(linker)), 1.42 - 1.35 (m, 2H, -C*H*₂(linker)), 1.22 - 1.15 (m, 3H, H-6^{Vio}).

¹³C NMR (151 MHz, D₂O) *δ* 174.57, 164.62, 103.87 (C-1*β*^{Vio}), 103.15 (C-1*β*^{Vio}), 97.10 (C-1*α*^{GalN}), 96.95 (C-1*α*^{GalN}), 79.07, 78.72, 74.05, 73.20, 73.05, 70.75, 70.67, 69.86, 68.87, 68.23, 68.12, 67.86, 55.50, 55.35, 50.18, 39.25, 27.93, 26.38, 26.21, 22.20, 21.83, 21.79, 16.87, 16.75.

HRMS (ESI): calculated for C₂₀H₃₇N₄O₁₀ [M+H]⁺: 493.2503 found: 493.2550.

### 5-Amino-pentanyl 4-N-formamido-4,6-dideoxy- β -D-glucopyranosyl-(1 → 4)-2-acetamido-2-deoxy-α-D-galactopyranoside (13)

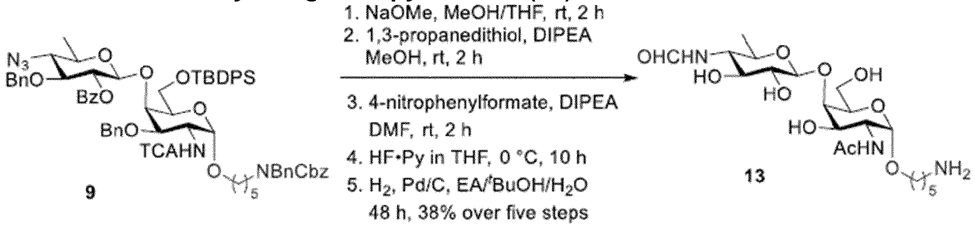

Sodium methoxide (0.108 g, 1.99 mmol) was added to the stirring solution of compound **9** (0.132 g, 0.099 mmol) in MeOH/THF (1/1, *v*/*v,* 4 mL) at room temperature. After 1 h, TLC showed complete conversion of the starting material. Then Amberlite IR-120 H+ was added to neutralize the mixture. Then it was filtered out, concentrated to afford debenzoylated disaccharide compound as colorless sticky solid.

1,3-Propanedithiol (0.4 mL) was added to the stirring solution of disaccharide in DIPEA (0.12 mL) and methanol (5.5 mL). After 2 h stirring at room temperature, the crude residue was directly concentrated under reduced pressure and co-evaporated with toluene twice. The concentrated compound was dried under high *vacuum* for next step.

The crude amine residue was dissolved in anhydrous DMF (7.3 mL). 4-Nitrophenyl-formate (62 mg, 0.372 mmol) was added to it followed by dropwise addition of DIPEA (0.13 mL, 0.74 mmol) at room temperature. After 2 h and complete consumption of the amine (indicated by TLC), the reaction mixture was diluted with ethyl acetate (30 mL) and washed with brine solution. The separated organic layer was dried over anhydrous Na₂SO₄, concentrated to obtain *N*-formamido disaccharide compound as a sticky colorless liquid.

To a stirring solution of above prepared *N*-formamido disaccharide in THF (3 mL) was added dropwise solution of 70% HF•Py (0.15 mL) at 0 °C. After 1 h, Et₃N was added and crude was directly concentrated under reduced pressure and passed through a small bed of silica gel column chromatography (1:1 Ethyl acetate: *n*-Hexane to 100% Ethyl acetate) to get disaccharide diol as colorless sticky liquid. The obtained diol *N*-formamido compound was dissolved in EA/*t*BuOH/H₂O (2/1/1, *v*/*v*/*v,* 5 mL). To this solution a suspension of Pd/C (90 mg) was added and stirred under hydrogen atmosphere for 48 h. The reaction mixture was then filtered out through Celite^{®} 353, concentrated and purified by HPLC (Hypercarb Column, 150×10 mm) at a flow rate of 3 mL/min, eluted by H₂O (with 0.1% formic acid, Solvent A) and acetonitrile (Solvent B) with a linear gradient from 0% to 30% of solvent B over 30 minutes and lyophilized to get desired compound **13** (18 mg, 38% over five steps) as a white powder.

¹H NMR (600 MHz, D₂O) *δ* 8.21 (s, 1H, -NHC*H*O), 4.93 (d, *J* = 3.8 Hz, 1H, H-1*α*^{GalN}), 4.65 (d, *J* = 8.0 Hz, 1H, H-1*β*^{Vio}), 4.27 - 4.20 (m, 2H, H-2^{GalN}, H-4^{GalN}), 4.03 - 3.96 (m, 2H, H-3^{GalN}, H-5^{GalN}), 3.84 - 3.76 (m, 2H, H-6a^{GalN}, H-6b^{GalN}), 3.75 - 3.68 (m, 1H, -C*H*(linker)), 3.68 - 3.63 (m, 1H, H-4^{Vio}), 3.63 - 3.59 (m, 1H, H-5^{Vio}), 3.59 - 3.53 (m, 1H, H-3^{Vio}), 3.53 - 3.48 (m, 1H, -C*H*(linker)), 3.45 (t, *J* = 8.0 Hz, 1H, H-2^{Vio}), 3.00 (dd, *J* = 8.4, 6.9 Hz, 2H, -C*H*₂(linker)), 2.05 (s, 3H, -NHCOC*H*₃), 1.72 - 1.61 (m, 4H, -C*H*₂(linker)), 1.49 - 1.40 (m, 2H, -C*H*₂(linker)), 1.30 - 1.22 (m, 3H, H-6^{Vio}).

¹³C NMR (151 MHz, D₂O) *δ* 174.63, 167.74, 164.72, 103.63 (C-1*β*^{Vio}), 96.97 (C-1*α*^{GalN}), 77.95, 77.89, 74.15, 73.99, 73.28, 70.92, 70.46, 70.44, 68.30, 68.27, 67.82, 61.41, 61.39, 60.20, 55.37, 50.54, 39.35, 27.93, 26.44, 22.25, 21.88, 16.77. HRMS (ESI): calculated for C₂₀H₃₈N₃O₁₀ [M+H]⁺: 480.2551 found: 480.2556.

### Phenyl-3-O-acetyl-4-O-benzyl-2-trichloroacetamido-2,6-dideoxy-1-seleno-α-D-glucopyranoside (15)

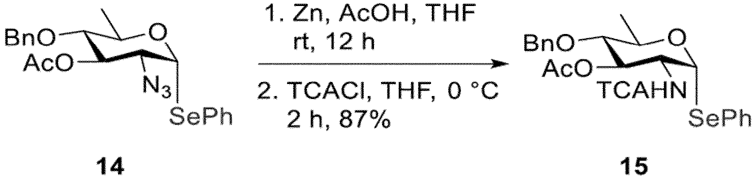

Activated zinc (1.15 g), and AcOH (1 mL) were added sequentially to the stirring solution of compound **14** (1.0 g, 2.17 mmol) in THF (10 mL). After 3 h and complete consumption of starting material (indicated by TLC), the reaction mixture was diluted with ethyl acetate (30 mL). Zinc was
removed by filtering through a celite bed. The crude was directly concentrated under reduced pressure, azeotroped with toluene twice and dried under high *vacuum* for next step without purification.

The crude amine was dissolved in THF (20 mL) and TCACI (0.48 mL, 4.34 mmol) was added at 0 °C. After 2 h, the reaction mixture was diluted with ethyl acetate (10 mL) and washed with H₂O and brine solution. The separated organic layer was dried over anhydrous Na₂SO₄, concentrated and purified by silica gel column chromatography (1:4 Ethyl acetate: *n*-Hexane) to give compound **15** (1.09 g, 87% over two steps) as white foam.

¹H NMR (600 MHz, CDCl₃) *δ* 7.56 - 7.53 (m, 2H, -ArH), 7.37 (td, *J* = 6.9, 1.1 Hz, 2H, -ArH), 7.34 - 7.32 (m, 1H, -ArH), 7.31 - 7.30 (m, 2H, -ArH), 7.30 - 7.29 (m, 1H, -ArH), 7.28 - 7.26 (m, 2H, -ArH), 7.26 (d, *J* = 1.2 Hz, 1H, -NH), 5.81 (d, *J =* 6.0 Hz, 1H, H-1), 5.13 (t, *J =* 12.0 Hz, 1H, H-3), 4.71 (s, 2H, -C*H*₂Ph), 4.33 - 4.26 (m, 1H, H-2), 4.21 - 4.14 (m, 1H, H-5), 3.41 - 3.35 (m, 1H, H-4), 2.01 (s, 3H, -OCOC*H*₃), 1.38 (d, *J* = 6.3 Hz, 3H, H-6).

¹³C NMR (151 MHz, CDCl₃) *δ* 171.75, 161.88, 137.61, 134.31, 129.49, 128.87, 128.70, 128.28, 128.22, 127.92, 92.10, 87.30 (C-1), 81.12, 77.37, 77.35, 77.16, 77.14, 76.95, 76.93, 75.48, 74.03, 71.12, 55.83, 20.95, 17.85.

HRMS (ESI): calculated for C₂₃H₂₄Cl₃NO₅SeNa [M+Na]⁺: 601.9772, found: 601.9790.

### N-(Benzyl)benzyloxycarbonyl-5-amino-pentanyl 3-O-acetyl-4-O-benzyl-2-trichloroacetamido-2,6-dideoxy-α-D-glucopyranosyl-(1→2)-4-azido-3-O-benzyl-4,6-dideoxy-β-D-glucopyranoside (17)

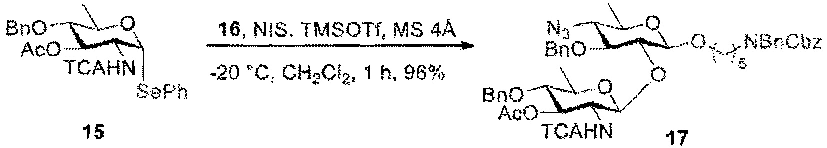

Anhydrous CH₂Cl₂ (11.95 mL) was added to the mixture of D-quinosamine donor **15** (0.42 g, 0.72 mmol), linker coupled 4-azido-D-viosamine acceptor **16** (0.28 g, 0.476 mmol) and activated 4Å molecular sieves (1.0 g) and the solution was stirred at room temperature 0.5 h. Then NIS (0.16 g, 0.72 mmol), and TMSOTf (17 *µ*L, 0.096 mmol) were added sequentially to the stirring solution at -20 °C and the mixture was stirred at same temperature for 1 h. After completion of reaction (indicated by TLC), Et₃N was added and 4Å molecular sieves were removed by filtering through a celite pad. The solution was then diluted with CH₂Cl₂ (30 mL) and washed with sat. Na₂S₂O₃ solution. The separated organic layer was dried over anhydrous Na₂SO₄, concentrated and purified by silica gel column chromatography (1:4 Ethyl acetate: n-Hexane) to give disaccharide compound **17** (0.46 g, 96%) as white foam.

¹H NMR (700 MHz, CDCl₃) *δ* 7.39 - 7.35 (m, 5H, -ArH), 7.34 (ddd, *J* = 7.5, 6.1, 1.4 Hz, 3H, -ArH), 7.31 - 7.28 (m, 5H, -ArH), 7.27 - 7.24 (m, 6H, -ArH), 7.17 (d, *J =* 7.2 Hz, 1H, -ArH), 6.71-6.6 (m, 1H, -N*H*), 5.20 - 5.09 (m, 3H, -C*H*₂Cbz, H-3^{QuiN}), 4.99-4.89 (m, 1H, H-1*β*^{QuiN}), 4.83 - 4.74 (m, 2H, -C*H*₂Ph), 4.65 - 4.58 (m, 2H, -C*H*₂Ph), 4.56 - 4.44 (m, 2H, -C*H*₂Ph), 4.31-4.28 (m, 1H, H-1*β*^{Vio}), 3.96-3.98 (m, 1H, H-2^{QuiN}), 3.83 - 3.76 (m, 1H, -C*H*(linker)), 3.63 (q, *J* = 8.9 Hz, 1H, H-2^{Vio}), 3.51 - 3.36 (m, 3H, H-5^{QuiN}, H-3^{Vi0}, -C*H*(linker)), 3.33-3.26 (m, 2H, H-4^{QuiN}, -C*H*(linker)), 3.24 - 3.15 (m, 2H, H-5^{Vi0}, -C*H*(linker)), 3.05 (t, *J* = 9.7 Hz, 1H, H-4^{Vio}), 1.94 (s, 3H, -OCOC*H*₃), 1.61 - 1.51 (m, 4H, -C*H*₂(linker)), 1.32-1.28 (m, 8H, H-6^{QuiN}, H-6^{Vio}, -C*H*₂(linker)).

¹³C NMR (151 MHz, CDCl₃) *δ* 170.90, 162.13, 156.40, 138.09, 137.78, 137.03, 128.69, 128.67, 128.59, 128.17, 128.03, 127.97, 127.42, 101.74 (C-1*β*^{QuiN}), 99.54 ((C-1*β*^{Vio}), 92.53, 82.96, 81.57, 79.95, 77.37, 77.25, 77.16, 77.08, 76.95, 75.15, 75.09, 74.25, 71.65, 70.58, 69.92, 69.71, 68.04, 67.38, 57.37, 50.61, 50.34, 47.30, 46.21, 29.84, 29.44, 27.94, 27.52, 23.50, 20.90, 18.57, 18.17.

HRMS (ESI): calculated for C₅₀H₅₈Cl₃N₅O₁₁Na [M+Na]⁺: 1032.3090, found: 1032.3191.

### 5-Amino-pentanyl 2-acetamido-2,6-dideoxy-α-D-glucopyranosyl-(1→2)-4-N-formamido-4,6-dideoxy-β-D-glucopyranoside (18)

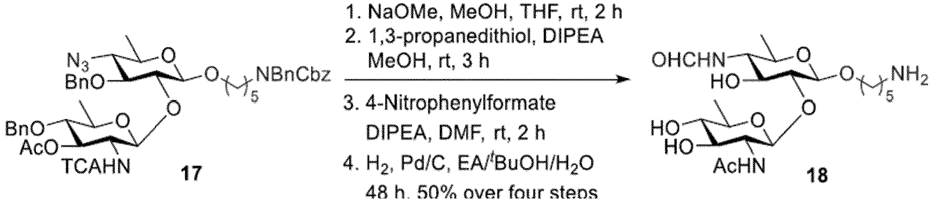

Sodium methoxide (4.2 mg, 0.07 mmol) was added to the stirring solution of compound 17 (18 mg, 0.018 mmol) in MeOH/THF (1/1, *v*/*v,* 1 mL) at room temperature. After 1 h, TLC showed complete conversion of the starting material. Then Amberlite IR-120 H+ was added to neutralize the mixture. Then it was filtered out, concentrated to afford deacetylated disaccharide compound as colorless foam.

1,3-Propanedithiol (87 *µ*L) was added to the stirring solution of above formed disaccharide in DIPEA (27 *µ*L) and methanol (1.5 mL). After 2 h stirring at room temperature, the crude residue was directly concentrated under reduced pressure and co-evaporated with toluene twice. The
concentrated compound was then dried under high *vacuum* for the next step.

The crude amine residue was dissolved in anhydrous DMF (1.7 mL). 4-Nitrophenyl-formate (15 mg, 0.09 mmol) was added to it followed by dropwise addition of DIPEA (0.03 mL, 0.18 mmol). After 2 h and complete consumption of the amine (indicated by TLC), the reaction mixture was diluted with ethyl acetate (20 mL) and washed with brine solution. The separated organic layer was dried over anhydrous Na₂SO₄, concentrated to obtain *N*-formamido disaccharide compound as a sticky white solid. The obtained amide compound was dissolved in EA/*t*BuOH/H₂O (2/1/1, *v*/*v*/*v,* 5 mL). To this solution a suspension of Pd/C (50 mg) was added and stirred under hydrogen atmosphere (1 atm, balloon) for 48 h. the reaction mixture was then filtered out through Celite^{®} 353, concentrated and purified by HPLC (Hypercarb Column, 150×10 mm) at a flow rate of 3 mL/min, eluted by H₂O (with 0.1% formic acid, Solvent A) and acetonitrile (Solvent B) with a linear gradient from 0% to 30% of solvent B over 30 minutes and lyophilized to get desired disaccharide **18** (4.05 mg, 50% over four steps) as a white solid.

¹H NMR (400 MHz, D₂O) *δ* 8.14-7.94 (m, -1H, -NHCHO), 4.66 (d, *J* = 8.4 Hz, 1H, H-1*β*^{QuiN}), 4.41 (d, *J* = 7.9 Hz, 1H, H-1*β*^{Vio}), 3.86-3.80 (m, 1H, -C*H*(linker)), 3.68 - 3.56 (m, 2H, H-2^{Vio}, -C*H*(linker)), 3.56 - 3.45 (m, 3H, H-3^{QuiN}, H-4^{QuiN}, H-5^{QuiN}), 3.45 - 3.29 (m, 3H, H-2^{QuiN}, H-3^{Vi0}, H-5^{Vio}), 3.14 (t, *J* = 9.2 Hz, 1H, H-4^{Vio}), 2.94 (t, *J =* 7.6 Hz, 2H, -C*H*₂(linker)), 1.96 (s, 3H, -NHCOC*H*₃), 1.67 - 1.55 (m, 4H, -C*H*₂(linker)), 1.44 - 1.34 (m, 2H, -C*H*₂(linker)), 1.26 (d, *J* = 6.2 Hz, 3H, H-6^{Vio}), 1.17 (dd, *J* = 6.1 Hz, 3H, H 6^{QuiN}).

¹³C NMR (151 MHz, D₂O) *δ* 174.80, 169.65, 164.70, 101.81 (C-1*β*^{QuiN}), 101.20 (C-1*β*^{Vio}), 82.01, 75.05, 73.30, 73.02, 71.80, 70.40, 70.00, 60.42, 56.20, 55.52, 39.27, 28.36, 26.46, 22.19, 22.09, 16.88, 16.62.

HRMS (ESI): calculated for C₂₀H₃₈N₃O₉ [M+H]+: 464.2603, found: 464.2615.

### N-(Benzyl)benzyloxycarbonyl-5-amino-pentanyl 3-O-benzyl-2-trichloro-acetamido-α-Dgalactopyranosyl-(1 → 3)-4-O-benzyl-2-trichloroacetamido-2,6-dideoxy-β-D-glucopyranoside (19)

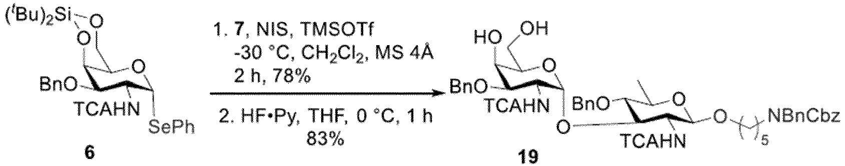

Anhydrous CH₂Cl₂ (13 mL) was added to the mixture of D-galactosamine donor **6** (0.54 g, 0.78 mmol), D-quinosamine acceptor **7** (0.365 g, 0.51 mmol) and molecular sieves (1.5 g) and this solution was stirred at room temperature for 0.5 h. Then NIS (0.174 g, 0.77 mmol) and TMSOTf (9 *µ*L, 0.051 mmol) were added to the stirring solution at -30 °C and it was allowed to stir for 1 h. After completion of the reaction (consumption of both donor and acceptor were monitored by TLC), Et₃N was added and 4Å molecular sieves were removed by filtering through a celite bed and the reaction mixture was diluted with CH₂Cl₂ (20 mL) and washed with saturated Na₂S₂O₃ solution. The separated organic layer was dried over anhydrous Na₂SO₄, concentrated and purified by silica gel column chromatography (1:4 Ethyl acetate: n-Hexane) to give disaccharide compound (0.494 g, 77%) as colorless viscous liquid. ¹H NMR (700 MHz, CDCl₃) *δ* 7.36 (s, 1H, -ArH), 7.33 (d, *J* = 8.2 Hz, 4H, -ArH), 7.30 (d, *J* = 7.2 Hz, 6H, -ArH), 7.25 (dd, *J* = 11.9, 5.9 Hz, 7H, -ArH), 7.14 (dd, *J* = 23.3, 8.3 Hz, 2H, -ArH), 6.94 - 6.76 (m, 1H, -N*H*), 5.60 (s, 1H, H-1*α*^{GalN}), 5.17 (d, *J =* 22.8 Hz, 2H, -C*H*₂Cbz), 4.69 - 4.67 (m, 2H, H-2^{GalN}, H-6a^{GalN}, 4.58 - 4.52 (m, 4H, H-1*β*^{QuiN}, H-6b^{GalN}, -C*H*₂Ph), 4.51-4.43 (m, 3H, H-4^{GalN}, -C*H*₂Ph), 4.15 - 4.06 (m, 3H, H-3^{QuiN}, -C*H*₂Ph), 3.81 - 3.63 (m, 4H, -C*H*(linker), H-2^{QuiN}, H-5^{GalN}, H-3^{GalN}), 3.53-3.51 (m, 1H, H-5^{QuiN}), 3.42 - 3.27 (m, 1H, -C*H*(linker), 3.27 - 3.13 (m, 3H, -C*H*₂(linker), H-4^{QuiN}), 1.56 - 1.45 (m, 4H, -C*H*₂(linker)), 1.35 - 1.27 (m, 5H, H-6^{QuiN}, -C*H*₂(linker)), 1.04 (s, 18H, -C(C*H*₃)₃).

¹³C NMR (176 MHz, CDCl₃) *δ* 161.94, 161.77, 156.83, 156.36, 137.93, 137.90, 137.01, 136.68, 128.85, 128.71, 128.66, 128.60, 128.28, 128.08, 127.99, 127.92, 127.86, 127.48, 127.34, 99.59 (C-1*β*^{QuiN}), 96.96 (C-1*α*^{GalN}), 92.76, 92.59, 84.23, 84.04, 77.34, 77.16, 76.98, 75.22, 75.04, 74.50, 74.45, 71.33, 70.29, 69.84, 69.40, 68.33, 67.34, 67.29, 56.96, 56.88, 50.62, 50.40, 49.86, 47.20, 46.19, 29.84, 29.34, 29.07, 27.73, 27.43, 23.61, 23.31, 20.86, 18.46.

HRMS (ESI): calculated for C₅₈H₇₃Cl₆N₃O₁₂SiNa [M+Na]⁺: 1264.2987, found: 1264.2646.

To a stirring solution of protected disaccharide (0.68 g, 0.55 mmol) in THF (5.6 mL) was added dropwise solution of HF•Py (70% in THF, 0.1 mL) at 0 °C. After 1 h, Et₃N was added and the crude was directly concentrated under reduced pressure and purified by silica gel column chromatography (1:1 Ethyl acetate: *n*-Hexane to 100% Ethyl acetate) to get disaccharide diol **19** (0.501 g, 83%) as white foam.

¹H NMR (700 MHz, CDCl₃) *δ* 7.97-7.90 (m, 1H, -N*H*), 7.39 - 7.35 (m, 2H, -ArH), 7.33 - 7.29 (m, 10H, -ArH), 7.28 - 7.24 (m, 4H, -ArH), 7.23 (d, *J* = 6.8 Hz, 2H, -ArH), 7.17 (dd, *J* = 14.8, 7.3 Hz, 2H, -ArH), 7.11 - 6.99 (m, 1H, -NH), 5.54 (s, 1H, H-1*α*^{GalN}), 5.16 (d, *J* = 34.3 Hz, 2H, -C*H*₂Cbz), 4.60 (d, *J* = 14.0 Hz, 1H, -C*H*Ph), 4.57 - 4.41 (m, 7H, -C*H*Ph, -C*H*₂Ph, H-2^{GalN}, H-1*β*^{QuiN}), 4.09-4.03 (m, 1H, H-3^{QuiN}), 3.99 - 3.92 (m, 4H, H-2^{QuiN}, H-4^{GalN}, H-6a^{GalN}, H-5^{GalN}), 3.82 - 3.74 (m, 1H, -C*H*(linker)), 3.71-3.67 (m, 2H, H-3^{GalN}, H-6b^{GalN}), 3.48 - 3.46 (m, 1H, H-5^{QuiN}), 3.40-3.37 (m, 1H, -C*H*(linker)), 3.33 - 3.23 (m, 2H, -C*H*(linker), H-4^{QuiN}), 3.18-3.14 (m, 1H, -C*H*(linker)), 1.57-1.45 (m, 4H, -C*H*₂(linker)), 1.31 - 1.26 (m, 5H, H-6^{QuiN}, -C*H*₂(linker)).

¹³C NMR (176 MHz, CDCl₃) *δ* 162.89, 162.45, 162.10, 156.88, 156.41, 137.79, 137.09, 136.91, 136.72, 136.60, 134.91, 129.13, 128.79, 128.73, 128.66, 128.60, 128.55, 128.36, 128.32, 128.19, 128.16, 128.10, 128.06, 127.93, 127.86, 127.78, 127.44, 127.31, 125.39, 100.09 (C-1*β*^{QuiN}, 1 *J*_{C-H} = 161.0 Hz), 96.47 (C-1*α*^{GalN}, 1 *J*_{C-H} = 175.0 Hz), 92.73, 92.59, 84.84, 77.34, 77.16, 76.98, 75.56, 75.09, 74.64, 71.71, 71.09, 70.56, 69.32, 67.35, 67.32, 66.54, 62.99, 56.80, 50.51, 50.47, 50.29, 47.18, 46.10, 29.34, 29.17, 27.90, 27.32, 23.19, 18.06.

HRMS (ESI): calculated for C₅₀H₅₇Cl₆N₃O₁₂Na [M+Na]+: 1124.1965, found: 1124.1958.

### N-(Benzyl)benzyloxycarbonyl-5-amino-pentanyl 3-O-benzyl-2-trichloro-acetamido-6-pentafluorophenyl-α-D-galactopyranosyl uronate-(1 → 3)-4-O-benzyl-2-trichloroacetamido-2,6-dideoxy-β-D-glucopyranoside (20)

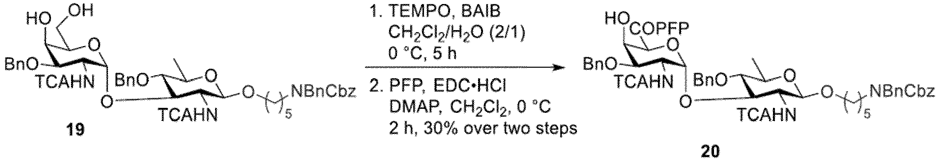

TEMPO (2.2 mg, 0.014 mmol), BAIB (101 mg, 0.314 mmol) were added sequentially to a stirring solution of diol **19** (77 mg, 0.069 mmol) in CH₂Cl₂ (4.6 mL) and H₂O (2.3 mL) at 0 °C. The mixture was stirred at same temperature for 5 h. After complete conversion of alcohol to acid (monitored by TLC), ethyl acetate (20 mL) was added to it and crude was washed with sat. NaHCOs solution. Separated organic layer was dried over anhydrous Na₂SO₄, concentrated and dried under high *vacuum* to get crude carboxylate compound as yellowish liquid.

To *vacuum* dried crude carboxylate compound in anhydrous CH₂Cl₂ (2 mL), pentafluorophenol (20 mg, 0.11 mmol) and catalytic amounts of DMAP (4.2 mg, 0.035 mmol) were added sequentially at 0 °C. After 5 minutes, EDC•HCl (47 mg, 0.24 mmol) was added to the reaction mixture. The mixture was stirred at same temperature for 3 h. After completion of the reaction (monitored by TLC), the crude reaction mixture was diluted with CH₂Cl₂ (20 mL) and washed with saturated NaHCOs and brine solution. The separated organic layer was dried over anhydrous Na₂SO₄, concentrated and purified by silica gel column chromatography (1:4 Ethyl acetate: *n*-Hexane) to obtain 6-O ester compound **20** (27 mg, 30% over two steps) as a white solid.

¹H NMR (400 MHz, CDCl₃) *δ* 7.36 (d, *J* = 2.3 Hz, 3H), 7.32 (td, *J* = 6.4, 2.4 Hz, 13H), 7.27 (d, *J* = 2.1 Hz, 2H), 7.18 - 7.12 (m, 1H), 7.09 - 7.01 (m, 1H), 5.90 (d, *J* = 5.4 Hz, 1H), 5.18 (d, *J* = 4.2 Hz, 2H), 4.90 (d, *J* = 12.1 Hz, 1H), 4.72 (dd, *J* = 10.8, 6.8 Hz, 2H), 4.64 (d, *J* = 10.5 Hz, 1H), 4.55 (d, *J* = 10.5 Hz, 2H), 4.48 (q, *J* = 4.8 Hz, 3H), 4.38 - 4.16 (m, 2H), 4.01 - 3.76 (m, 3H), 3.45 - 3.35 (m, 2H), 3.34 - 3.14 (m, 4H), 1.58 - 1.40 (m, 7H), 1.37 (d, *J* = 6.1 Hz, 3H).

¹³C NMR (101 MHz, CDCl₃) *δ* 170.95, 162.84, 162.15, 156.90, 156.50, 139.27, 137.83, 136.93, 136.68, 136.34, 128.97, 128.84, 128.70, 128.60, 128.20, 128.00, 127.90, 127.50, 100.37-100.12 (C-1*β*^{QuiN}), 97.78 (C-1*α*^{GalN}), 92.35, 91.97, 84.90, 78.68, 77.48, 77.36, 77.26, 77.16, 77.03, 76.84, 75.52, 74.28, 72.73, 71.00, 69.74, 69.44, 67.44, 56.26, 55.17, 50.48, 47.25, 31.56, 30.30, 29.84, 29.80, 29.50, 28.80, 27.14, 23.20, 22.84, 17.97, 14.28.

HRMS (ESI): calculated for C₅₆H₅₄Cl₆F₅N₃O₁₃Na [M+Na]⁺: 1304.1600, found: 1304.1756.

### 5-Amino-pentanyl 2-acetamido-α-D-galactopyranosyl uronamide-(1→3)-2-acetamido-2,6-dideoxy-β-D-glucopyranoside (21)

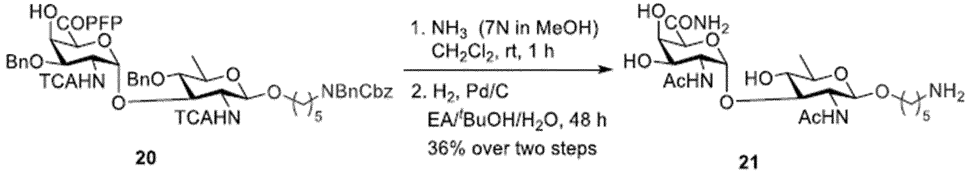

Ammonia solution (7N in methanol, 2.4 mL) was added dropwise to the stirred solution of compound **20** (20 mg, 0.016 mmol) in CH₂Cl₂ (0.5 mL) at room temperature. After 1 h and complete consumption of the starting material, the crude was directly concentrated under reduced pressure to get amide compound as viscous liquid.

The obtained amide compound was dissolved in EA/*t*BuOH/H₂O (2/2/1, *v*/*v*/*v,* 5 mL). To this solution, a suspension of Pd/C (30 mg) was added and stirred under hydrogen atmosphere (1 atm, balloon) for 48 h. The reaction mixture was then filtered through Celite^{®} 353, concentrated, purified by HPLC (Hypercarb Column, 150×10 mm at a flow rate of 3 mL/min, eluted by H₂O (with 0.1% formic acid, Solvent A) and acetonitrile (Solvent B) with a linear gradient from 0% to 30% of solvent B over 30 minutes and lyophilized to get disaccharide compound **21** (2.8 mg, 36% over two steps) as a white solid.

¹H NMR (600 MHz, D₂O) *δ* 5.35 (d, *J* = 3.9 Hz, 1H, H-1*α*^{GalN}), 4.48 (d, *J* = 8.4 Hz, 1H, H-1*β*^{QuiN}), 4.27 (s, 2H, H-4^{GalN}, H-5^{GalN}), 4.23 (dd, *J* = 6.0 Hz, 1H, H-2^{GalN}), 3.93 (dd, *J* = 11.2, 3.5 Hz, 1H, H-3^{GalN}), 3.87 (dt, *J* = 10.1, 6.1 Hz, 1H, -C*H*(linker)), 3.73 (t, *J =* 9.0 Hz, 1H, H-2^{QuiN}), 3.63 (d, *J* = 9.7 Hz, 1H, H-3^{QuiN}), 3.58 (dt, *J* = 10.0, 6.3 Hz, 1H, -C*H*(linker)), 3.49 - 3.43 (m, 1H, H-5^{QuiN}), 3.43 - 3.37 (m, 1H, H-4^{QuiN}), 3.02 - 2.95 (m, 2H, -C*H*₂(linker)), 2.06 (s, 3H, -NHCOC*H*₃), 1.98 (s, 3H, -NHCOC*H*₃), 1.67 (p, *J =* 7.6 Hz, 2H, -C*H*₂(linker)), 1.61 - 1.54 (m, 2H, -C*H*₂(linker)), 1.38 (dtd, *J* = 14.6, 7.0, 4.2 Hz, 2H, -C*H*₂(linker)), 1.31 (d, *J* = 6.1 Hz, 3H, H-6^{QuiN}).

¹³C NMR (151 MHz, D₂O) *δ* 174.47, 174.31, 173.70, 101.00 (C-1*β*^{QuiN}), 98.63 (C-1*α*^{GalN}), 80.24, 75.97, 71.68, 71.20, 70.12, 68.67, 67.00, 54.59, 49.23, 39.20, 28.08, 26.34, 22.07, 21.98, 21.88, 16.40.

HRMS (ESI): calculated for C²¹H³⁹N⁴O¹⁰ [M+H]⁺: 507.2660, found: 507.2665.

### 5-Amino-pentanyl 2-acetamido-α-D-galactopyranosyl-(1→3)-2-acetamido-2,6-dideoxy-β-Dglucopyranoside (22)

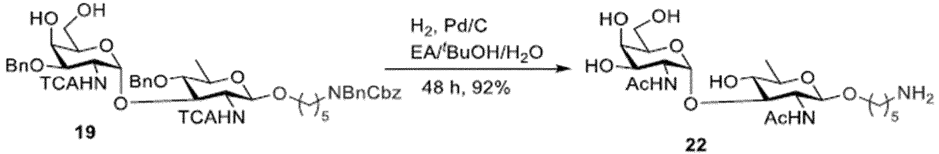

To a solution of compound **19** (7.7 mg, 0.007 mmol) in EA/*t*BuOH/H₂O (2/1/1, *v*/*v*/*v,* 4 mL) was added Pd/C (20 mg) at room temperature. After stirring under hydrogen atmosphere (1 atm, balloon) for 48 h, the reaction mixture was filtered through Celite^{®} 353, concentrated and purified by HPLC (Hypercarb Column, 150×10 mm at a flow rate of 3 mL/min, eluted by H2O (with 0.1% formic acid, Solvent A) and acetonitrile (Solvent B) with a linear gradient from 0% to 30% of solvent B over 30 minutes and lyophilized to obtain disaccharide compound **22** (3.2 mg, 92%) as a white solid.

¹H NMR (600 MHz, D₂O) *δ* 5.40 (d, *J* = 3.9 Hz, 1H, H-1*α*^{GalN}), 4.49 (d, *J* = 8.5 Hz, 1H, H-1*β*^{QuiN}), 4.15 (dd, *J* = 11.1, 3.9 Hz, 1H, H-2^{GalN}), 3.96 (d, *J* = 3.7 Hz, 1H, H-4^{GalN}), 3.86-3.82 (m, 2H, -C*H*(linker), H-6a^{GalN}), 3.79 - 3.68 (m, 4H, H-3^{GalN}, H-5^{GalN}, H-2^{QuiN}, -C*H*(linker)), 3.64 - 3.54 (m, 2H, H-3^{QuiN}, H-6b^{GAlN}), 3.47 - 3.41 (m, 1H, H-5^{QuiN}) 3.37 (t, *J* = 12.0 Hz, 1H, H-4^{QuiN}), 2.95 (t, *J* = 7.7 Hz, 2H, -C*H*₂(linker)), 2.02 (s, 6H, -NHCOC*H*₃), 1.64 (p, *J* = 7.6 Hz, 2H, -CH(linker)), 1.60 - 1.53 (m, 2H, -CH(linker)), 1.40 - 1.32 (m, 2H, -C*H*₂(linker), 1.27 (d, *J* = 6.1 Hz, 3H, H-6^{QuiN}).

¹³C NMR (151 MHz, D₂O) *δ* 174.45, 174.28, 100.79 (C-1*α*^{GalN}), 97.19 (C-1*β*^{QuiN}), 77.78, 76.34, 71.76, 70.86, 70.13, 68.16, 67.49, 60.56, 54.40, 49.59, 39.30, 28.11, 26.40, 22.24, 22.12, 21.94, 16.41.

HRMS (ESI): calculated for C₂₁H₄₀N₃O₁₀ [M+H]⁺: 494.2707, found: 494.2721.

### N-(Benzyl)benzyloxycarbonyl-5-amino-pentanyl 3-O-benzyl-2-trichloro-acetamido-6-O-tertbutyldiphenylsilyl-α-D-galactopyranosyl-(1→3)-4-O-benzyl-2-trichloroacetamido-2,6-dideoxy-β-D-glucopyranoside (23)

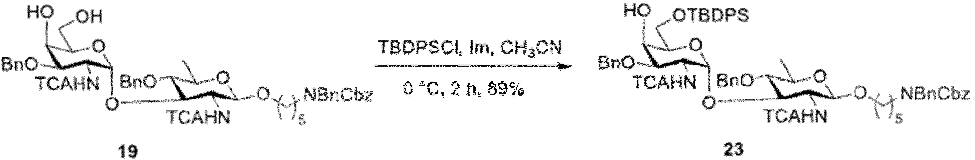

Imidazole (68 mg, 0.996 mmol) and TBDPSCI (0.23 mL, 0.905 mmol) were added sequentially to the stirring solution of disaccharide diol **19** (500 mg, 0.453 mmol) in CH₃CN (9 mL) at 0 °C. After 2 h and complete consumption of the starting material, the crude reaction mixture was diluted with EtOAc (20 mL) and washed with brine solution. The separated organic layer was dried over anhydrous Na₂SO₄, concentrated and purified by silica gel column chromatography (3:7 Ethyl acetate: *n*-Hexane) to afford 4-OH disaccharide acceptor **23** (541 mg, 89%) as a white sticky solid.

¹H NMR (400 MHz, CDCl₃) *δ* 7.78 - 7.66 (m, 5H), 7.48 - 7.30 (m, 24H), 7.20 (dd, *J* = 5.1, 3.1 Hz, 1H), 7.09 - 6.98 (m, 1H), 6.95-6.75 (m, 1H), 5.63 (d, *J* = 3.9 Hz, 1H, H-1*α*^{GalN}), 5.25 - 5.12 (m, 2H), 4.66 - 4.58 (m, 4H), 4.53 (dd, *J* = 9.7, 4.0 Hz, 3H, H-1*β*^{QuiN}), 4.35 (q, *J* = 8.0 Hz, 1H), 4.10 - 4.01 (m, 3H), 4.01 - 3.91 (m, 2H), 3.88 (dd, *J =* 10.3, 4.5 Hz, 1H), 3.84 - 3.73 (m, 1H), 3.72 - 3.64 (m, 1H), 3.48 - 3.34 (m, 2H), 3.29 (t, *J* = 8.6 Hz, 2H), 3.25 - 3.15 (m, 1H), 2.53 (s, 1H), 1.55 - 1. 50 (m, 4H), 1.37 (d, *J* = 6.2 Hz, 3H), 1.33-1.30 (m, 2H), 1.11 (s, 9H).

¹³C NMR (101 MHz, CDCl₃) *δ* 162.04, 161.92, 156.80, 156.29, 137.92, 137.30, 136.97, 136.76, 135.76, 135.33, 134.91, 133.05, 132.89, 130.02, 129.95, 129.73, 128.79, 128.66, 128.56, 128.33, 128.28, 128.05, 127.97, 127.92, 127.90, 127.81, 127.44, 127.32, 100.22 (C-1*β*^{QuiN}), 95.95 (C-1*α*^{GalN}), 92.70, 92.59, 85.01, 77.48, 77.36, 77.16, 76.84, 74.45, 74.22, 71.86, 71.33, 70.87, 69.54, 67.27, 66.03, 63.86, 56.73, 50.60, 50.38, 47.16, 46.19, 29.81, 29.25, 27.97, 27.42, 27.24, 26.66, 23.29, 19.38, 18.02.

HRMS (ESI): calculated for C₆₆H₇₅Cl₆N₃O₁₂SiNa [M+Na]⁺: 1362.3143, found: 1362.2823.

### N-(Benzyl)benzyloxycarbonyl-5-amino-pentanyl 3-O-benzyl-2-trichloro-acetamido-4,6-Osilylidene-α-D-galactopyranosyl-(1 → 4)-3-O-benzyl-2-trichloro-acetamido-6-O-tertbutyldiphenylsilyl-α-D-galactopyranosyl-(1→3)-4-O-benzyl-2-trichloroacetamido-2,6-dideoxy-β-D-glucopyranoside (24)

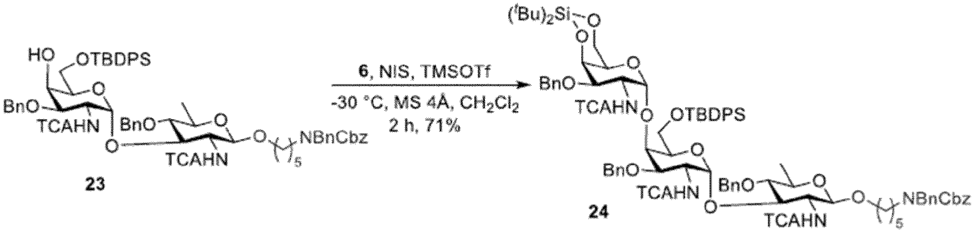

Anhydrous CH₂Cl₂ (13 mL) was added to the mixture of D-galactosamine donor **6** (0.593 g, 0.855 mmol), 4-OH-disaccharide acceptor **23** (0.574 g, 0.428 mmol) and molecular sieves (1.5 g) and this solution was stirred at room temperature for 0.5 h. Then NIS (0.192 g, 0.855 mmol) and TMSOTf (15 *µ*L, 0.085 mmol) were added to the stirring solution at -30 °C and it was stirred for 2 h. After completion of the reaction (monitored by TLC), Et₃N was added and 4Å molecular sieves were removed by filtering through a celite bed and the reaction mixture was diluted with CH₂Cl₂ (20 mL) and washed with saturated Na₂S₂O₃ solution. The separated organic layer was dried over anhydrous Na₂SO₄, concentrated and purified by silica gel column chromatography (1:4 Ethyl acetate: n-Hexane) to give protected trisaccharide **24** (0.57 g, 71%) as colorless viscous liquid.

¹H NMR (400 MHz, CDCl₃) *δ* 7.54 (dd, *J* = 23.0, 7.6 Hz, 4H), 7.31 (t, *J* = 7.3 Hz, 7H), 7.27 - 7.21 (m, 10H), 7.21 - 7.14 (m, 11H), 7.10 (d, *J* = 7.7 Hz, 4H), 6.99 (t, *J =* 11.9 Hz, 1H), 6.60 (d, *J* = 9.7 Hz, 2H), 5.23 (s, 1H, H-1*α*^{GalN}), 5.16 (d, *J* = 3.3 Hz, 1H, H-1'*α*^{GalN}), 5.08 (d, *J* = 16.1 Hz, 2H), 4.63 - 4.52 (m, 3H), 4.46 (q, *J* = 5.4 Hz, 4H), 4.43 - 4.35 (m, 4H), 4.32-4.27 (m, 2H, H-1*β*^{QuiN}), 4.03 (s, 1H), 3.84 (t, *J* = 8.8 Hz, 1H), 3.71 (t, *J* = 11.6 Hz, 4H), 3.59 (dd, *J* = 18.9, 12.0 Hz, 3H), 3.32 (d, *J* = 7.1 Hz, 2H), 3.09 (d, *J* = 8.9 Hz, 4H), 1.40 (dd, *J* = 14.9, 7.5 Hz, 4H), 1.17 (d, *J* = 5.6 Hz, 5H), 0.98 (d, *J* = 13.7 Hz, 18H), 0.87 (s, 9H).

¹³C NMR (101 MHz, CDCl₃) *δ* 162.25, 161.95, 161.49, 156.78, 156.29, 138.10, 137.97, 137.89, 136.97, 136.77, 135.64, 135.60, 133.46, 132.30, 130.45, 130.17, 129.15, 128.85, 128.67, 128.56, 128.50, 128.47, 128.34, 128.23, 128.07, 128.03, 127.92, 127.87, 127.64, 127.44, 127.30, 126.94, 125.41, 99.39 (C-1*β*^{Quip}), 97.28 (C-1'*α*^{GalN}), 96.54 (C-1*α*^{GalN}), 92.58, 83.86, 77.48, 77.36, 77.16, 77.01, 76.84, 76.02, 75.62, 74.37, 72.05, 71.93, 71.08, 70.70, 69.83, 69.51, 68.91, 67.96, 67.28, 66.96, 61.62, 57.12, 51.53, 50.59, 50.32, 49.72, 47.13, 46.18, 29.81, 29.27, 27.66, 27.44, 27.13, 23.39, 23.29, 21.59, 20.75, 19.36, 18.15.

HRMS (ESI): calculated for C₈₉H₁₀₇Cl₉N₄O₁₇Si₂Na [M+Na]⁺: 1897.4259, found: 1897.3906.

### N-(Benzyl)benzyloxycarbonyl-5-amino-pentanyl 3-O-benzyl-2-trichloro-acetamido-o-Dgalactopyranosyl-(1 → 4)-3-O-benzyl-2-trichloroacetamido-α-D-galactopyranosyl-(1 → 3)-4-O-benzyl-2-trichloroacetamido-2,6-dideoxy- β -D-glucopyranoside (25)

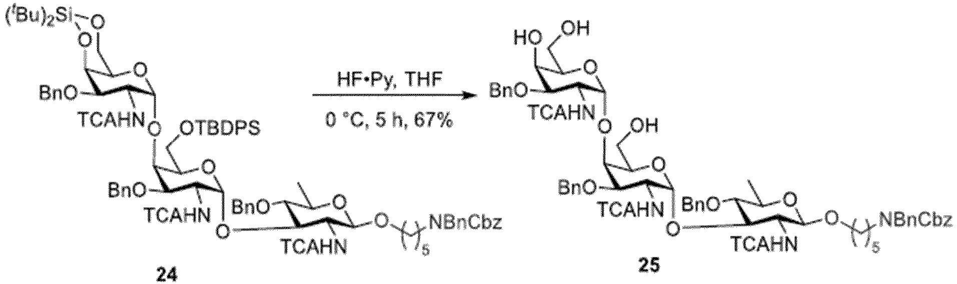

To a stirring solution of protected trisaccharide **24** (0.68 g, 0.363 mmol) in THF (6.7 mL) was added dropwise solution of HF•Py (70% in THF, 0.07 mL) at 0 °C. After 5 h, Et₃N was added and the crude was directly concentrated under reduced pressure and purified by silica gel column chromatography (100% ethyl acetate to 5:95 Methanol: Dichloromethane) to get trisaccharide triol **25** (0.364 g, 67%) as colorless liquid.

¹H NMR (600 MHz, CDCl₃) *δ* 7.39 - 7.28 (m, 15H), 7.26 (td, *J* = 8.1, 4.4 Hz, 8H), 7.22 - 7.14 (m, 2H), 7.07 - 6.92 (m, 2H), 5.43 (d, *J* = 4.0 Hz, 1H, H-1*α*^{GalN}), 5.16 (d, *J* = 25.2 Hz, 2H), 5.08 (d, *J* = 3.7 Hz, 1H, H-1'*α*^{GalN}), 4.71 - 4.64 (m, 2H), 4.64 - 4.56 (m, 3H), 4.56 - 4.50 (m, 2H, H-1*β*^{QuiN}), 4.50 - 4.43 (m, 4H), 4.26 (s, 1H), 4.17 - 4.08 (m, 3H), 4.08 - 3.99 (m, 1H), 3.89 (t, *J* = 8.9 Hz, 1H), 3.83 - 3.73 (m, 3H), 3.71 (dd, *J* = 10.9, 2.5 Hz, 1H), 3.62 (dd, *J* = 10.8, 5.3 Hz, 1H), 3.58 - 3.47 (m, 2H), 3.44 - 3.31 (m, 2H), 3.28 - 3.15 (m, 3H), 1.52-1.45 (m, 4H), 1.32-1.25 (m, 5H).

¹³C NMR (151 MHz, CDCl₃) *δ* 162.22, 162.13, 156.44, 137.78, 137.14, 137.07, 137.00, 136.59, 134.52, 128.77, 128.75, 128.72, 128.67, 128.57, 128.13, 128.10, 128.07, 127.85, 127.54, 127.40, 99.88 (C-1*β*^{QuiN}), 97.58 (C-1'*α*^{GalN}), 96.72 (C-1*α*^{GalN}), 92.70, 92.51, 92.47, 84.72, 84.37, 77.37, 77.26, 77.16, 76.95, 75.62, 75.49, 74.31, 74.10, 71.92, 71.78, 71.53, 71.15, 70.94, 69.68, 69.39, 67.37, 67.25, 62.79, 61.15, 60.52, 56.69, 51.22, 50.67, 50.30, 47.18, 46.02, 29.78, 29.30, 29.14, 27.82, 27.36, 23.25, 21.16, 18.16, 14.29.

HRMS (ESI): calculated for C₆₅H₇₃Cl₉N₄O₁₇Na [M+Na]⁺: 1519.2059, found: 1519.2161.

### 5-Amino-pentanyl 2-acetamido-o-D-galactopyranosyl-(1 → 4)-2-acetamido-α-D-galactopyranosyl-(1→3)-2-acetamido-2,6-dideoxy-β-D-glucopyranoside (26)

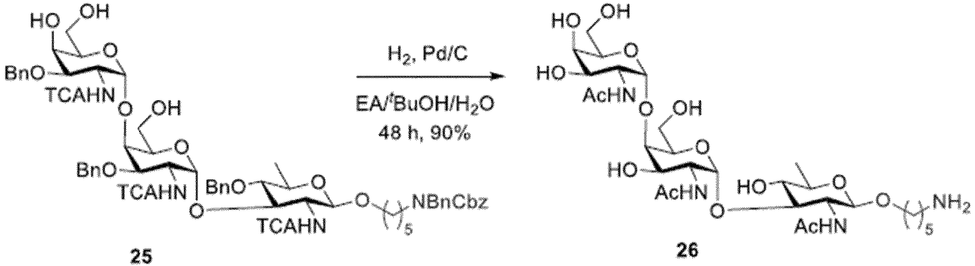

To a solution of trisaccharide triol **25** (10.8 mg, 0.0072 mmol) in EA/*t*BuOH/H₂O (2/1/1, *vlvlv,* 4 mL), Pd/C (20 mg) was added at room temperature and the reaction was stirred under hydrogen atmosphere (1 atm, balloon) for 48 h. Then crude reaction mixture was filtered through Celite^{®} 353, concentrated, purified by HPLC (Hypercarb Column, 150×10 mm at a flow rate of 3 mL/min, eluted by H₂O (with 0.1% formic acid, Solvent A) and acetonitrile (Solvent B) with a linear gradient from 0% to 30% of solvent B over 30 minutes and lyophilized to afford trisaccharide **26** (4.5 mg, 90%) as a white solid.

¹H NMR (400 MHz, D₂O) *δ* 5.43 (d, *J* = 3.8 Hz, 1H, H-1*α*^{GalN}), 4.93 (d, *J =* 3.9 Hz, 1H, H-1'*α*^{GalN}), 4.48 (d, *J* = 8.6 Hz, 1H, H-1*β*^{QuiN}), 4.33 (t, *J* = 6.7 Hz, 1H), 4.23 (dd, *J =* 11.4, 3.9 Hz, 1H), 4.20 - 4.14 (m, 1H), 4.00 (d, *J* = 9.3 Hz, 3H), 3.89 - 3.78 (m, 3H), 3.75 - 3.61 (m, 6H), 3.61 - 3.51 (m, 1H), 3.46 - 3.33 (m, 2H), 2.99 - 2.91 (m, 2H), 2.05 (s, 3H), 2.01 (d, *J* = 3.4 Hz, 6H), 1.63 (p, *J* = 7.6 Hz, 2H), 1.59 - 1.51 (m, 2H), 1.41 - 1.29 (m, 2H), 1.26 (d, *J* = 6.0 Hz, 3H).

¹³C NMR (151 MHz, D₂O) *δ* 174.48, 174.40, 174.24, 100.72 (C-1*β*^{QuiN}), 98.24 (C-1*α*^{GalN}), 97.14 (C-1'*α*^{GalN}), 77.84, 76.41, 76.25, 71.68, 71.54, 70.56, 70.09, 68.05, 66.99, 66.80, 60.34, 59.68, 54.33, 50.06, 49.68, 39.21, 28.06, 26.33, 22.15, 22.07, 21.84, 21.79, 16.33.

HRMS (ESI): calculated for C₂₉H₅₃N₄O₁₅ [M+H]+: 697.3501, found: 697.3515.

### N-(Benzyl)benzyloxycarbonyl-5-amino-pentanyl 3-O-benzyl-2-trichloro-acetamido-6-O-tertbutyldiphenylsilyl-α-D-galactopyranosyl-(1→4)-3-O-benzyl-2-trichloroacetamido-6-O-tert-butyldiphenylsilyl-α-D-galactopyranosyl-(1→3)-4-O-benzyl-2-trichloroacetamido-2,6-dideoxy-β-D-glucopyranoside (4)

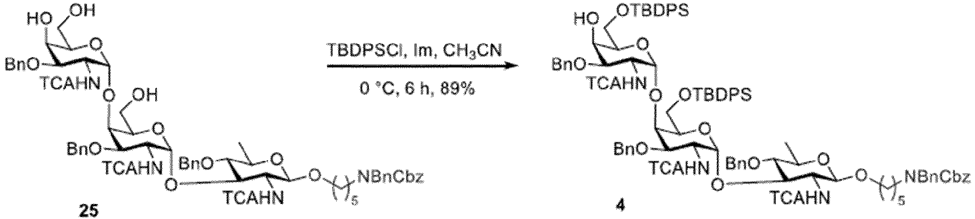

To the stirring solution of trisaccharide triol **25** (200 mg, 0.13 mmol) in CH₃CN (4.8 mL), imidazole (39 mg, 0.58 mmol) and TBDPSCI (0.13 mL, 0.53 mmol) were added sequentially at 0°C. After 6 h and complete consumption of the starting material (indicated by TLC), the crude reaction mixture was diluted with EtOAc (20 mL) and washed with brine solution. The separated organic layer was dried over anhydrous Na₂SO₄, concentrated and purified by silica gel column chromatography (3:7 Ethyl acetate: n-Hexane) to afford 4-OH trisaccharide acceptor **4** (234 mg, 89%) as white sticky liquid.

¹H NMR (600 MHz, CDCl₃) *δ* 7.58 (dt, *J* = 8.2, 1.5 Hz, 1H), 7.56 - 7.53 (m, 4H), 7.51 (dd, *J* = 8.0, 1.4 Hz, 2H), 7.46 (d, *J* = 7.1 Hz, 2H), 7.35 - 7.31 (m, 3H), 7.30 (s, 2H), 7.28 - 7.23 (m, 13H), 7.23 - 7.20 (m, 7H), 7.18 (t, *J* = 1.5 Hz, 2H), 7.16 - 7.12 (m, 4H), 7.10 - 7.06 (m, 2H), 7.02 - 6.99 (m, 3H), 6.90 - 6.85 (m, 3H), 6.66 - 6.45 (m, 2H), 5.31 (d, *J* = 3.9 Hz, 1H, H-1*α*^{GalN}), 5.08 (d, *J* = 22.8 Hz, 2H), 4.92 (s, 1H, H-1'*α*^{GalN}), 4.61 (d, *J =* 11.7 Hz, 1H), 4.51 - 4.44 (m, 2H), 4.44 - 4.37 (m, 6H), 4.36 - 4.28 (m, 1H), 4.28 - 4.18 (m, 3H, H-1*β*^{QuiN}), 4.15 - 4.07 (m, 2H), 3.86 - 3.76 (m, 2H), 3.74 - 3.65 (m, 4H), 3.60 - 3.50 (m, 3H), 3.41 - 3.34 (m, 1H), 3.31 - 3.21 (m, 2H), 3.16 (dd, *J* = 17.9, 8.9 Hz, 2H), 3.08 (s, 2H), 1.45 - 1.34 (m, 4H), 1.18-1.10 (m, 5H), 1.00 (s, 9H), 0.93 (s, 9H).

¹³C NMR (151 MHz, CDCl₃) *δ* 162.17, 161.93, 161.40, 156.32, 137.95, 137.73, 136.87, 135.97, 135.78, 135.68, 135.62, 134.93, 133.27, 132.90, 132.81,129.17, 128.84, 128.81, 128.69, 128.63, 128.59, 128.55, 128.33, 128.09, 127.98, 127.96, 127.94, 127.92, 127.82, 127.77, 127.35, 125.43, 99.64 (C-1*β*^{QuiN}), 97.24 (C-1'*α*^{GalN}), 96.10 (C-1*α*^{GalN}), 92.69, 92.61, 84.13, 77.37, 77.16, 76.95, 75.26, 74.99, 74.39, 72.31, 71.91, 71.58, 69.52, 69.04, 67.31, 64.34, 62.16, 56.99, 51.47, 50.65, 50.32, 47.20, 46.24, 29.83, 29.31, 28.02, 27.30, 26.92, 26.69, 23.33, 21.60, 19.39, 19.26, 18.14.

HRMS (ESI): calculated for C₉₇H₁₀₉Cl₉N₄O₁₇Si₂Na [M+Na]⁺: 1995.4415, found: 1995.4016.

### N-(Benzyl)benzyloxycarbonyl-5-amino-pentanyl 4-azido-3-O-benzyl-2-O-benzoyl-4,6-dideoxy- β -D-glucopyranosyl-(1 → 2)-3-O-benzyl-2-trichloro-acetamido-6-O-tertbutyldiphenylsilyl-α-D-galactopyranosyl-(1→4)-3-O-benzyl-2-trichloroacetamido-6-O-tertbutyldiphenylsilyl-α-D-galactopyranosyl-(1 → 3)-4-O-benzyl-2-trichloroacetamido-2,6-dideoxy-β-D-glucopyranoside (3)

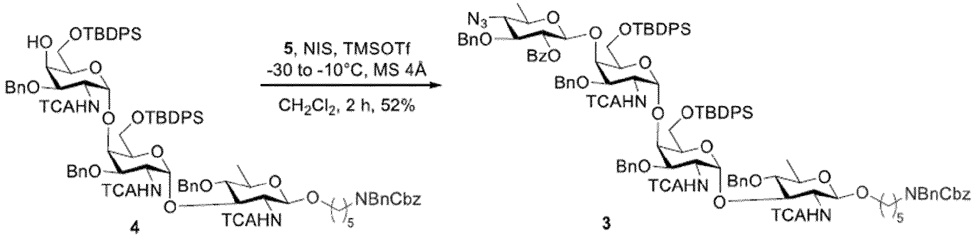

Anhydrous CH₂Cl₂ (1.1 mL) was added to the mixture of 4-azido-D-viosamine donor **5** (33 mg, 0.067 mmol), 4-OH-trisaccharide acceptor **4** (90 mg, 0.046 mmol) and molecular sieves (100 mg) and this solution was stirred at room temperature for 0.5 h. Then NIS (15 mg, 0.068 mmol) and TMSOTf (1.7 *µ*L, 0.009 mmol) were added to the stirring solution at -30 °C and it was allowed to warm up to -10 °C for 2 h. After completion of reaction (monitored by TLC), Et₃N was added and 4Å molecular sieves were removed by filtering through a celite bed and the reaction mixture was diluted with CH₂Cl₂ (10 mL) and washed with saturated Na2S2O3 solution. The separated organic layer was dried over anhydrous Na2SO4, concentrated and purified by silica gel column chromatography (0.5:0.5:9 Ethyl acetate: Dichloromethane: Toluene) to give protected tetrasaccharide compound **3** (55 mg, 52%) as colorless viscous liquid. 1H NMR (700 MHz, CDCl₃) *δ* 8.16 (d, *J* = 8.3 Hz, 2H), 7.63 (d, *J* = 7.0 Hz, 2H), 7.53 (dd, *J* = 15.5, 7.3 Hz, 4H), 7.46 (d, *J* = 7.5 Hz, 2H), 7.39 - 7.30 (m, 17H), 7.26 - 7.20 (m, 13H), 7.18 (d, *J* = 4.7 Hz, 8H), 7.15 (d, *J* = 7.3 Hz, 3H), 7.04 (d, *J* = 7.3 Hz, 2H), 6.68 - 6.50 (m, 2H), 5.37 (d, *J* = 3.9 Hz, 1H, H-1*α*^{GalN}), 5.30 (d, *J* = 8.0 Hz, 1H, H-1*β*Vio), 5.20 - 5.14 (m, 2H), 5.14 (s, 1H), 4.69 - 4.62 (m, 2H), 4.62 - 4.57 (m, 3H, H-1'*α*^{GalN}), 4.50 - 4.42 (m, 2H), 4.42 - 4.36 (m, 3H), 4.36 - 4.25 (m, 3H), 4.16 (s, 2H), 4.02 (d, *J* = 12.6 Hz, 1H, H-1*β*^{QuiN}), 3.92 - 3.84 (m, 3H), 3.80 (t, *J* = 6.7 Hz, 1H), 3.68 (dd, *J* = 10.4, 7.0 Hz, 2H), 3.61 - 3.50 (m, 4H), 3.50 - 3.44 (m, 1H), 3.34 - 3.23 (m, 1H), 3.20 (q, *J* = 7.8 Hz, 1H), 3.12 (t, *J* = 9.7 Hz, 2H), 3.06 (t, *J* = 8.4 Hz, 1H), 2.85 (dd, *J* = 9.9, 6.1 Hz, 1H), 1.49 - 1.36 (m, 4H), 1.21 (s, 2H), 1.15 - 1.10 (m, 6H), 1.06 (s, 9H), 0.90 (s, 9H).

¹³C NMR (176 MHz, CDCl₃) *δ* 165.51, 162.03, 161.62, 161.42, 156.79, 156.28, 137.96, 137.66, 137.38, 136.95, 135.84, 135.74, 135.49, 135.47, 133.39, 133.30, 133.26, 132.99, 132.41, 130.23, 128.82, 128.79, 128.68, 128.65, 128.54, 128.50, 128.46, 128.40, 128.21, 128.17, 128.04, 127.96, 127.94, 127.71, 127.68, 127.36, 99.63 (C-1*β*^{QuiN}, 1*J*_{C-H} = 154.0 Hz), 98.13 (C-1*β*^{Vio}, 1*J*_{C-H} = 168.0 Hz), 96.61 (C-1'*α*^{GalN}, 1 *J*_{C-H} = 168.0 Hz), 96.14 (C-1*α*^{GalN}, 1 *J*_{C-H} = 175.0 Hz), 92.60, 92.51, 83.71, 81.43, 77.34, 77.26, 77.16, 77.06, 76.98, 75.24, 74.69, 73.02, 72.19, 72.03, 71.68, 71.01, 70.67, 69.50, 68.28, 67.83, 67.28, 66.67, 62.75, 61.94, 56.64, 51.39, 50.62, 50.31, 47.16, 46.23, 29.85, 29.29, 27.98, 27.49, 27.37, 27.18, 27.15, 23.30, 19.44, 19.28, 18.44, 18.10.

HRMS (ESI): calculated for C₁₁₇H₁₂₈Cl₉N₇O₂₁Si₂Na [M+Na]⁺: 2360.5791, found: 2360.6353.

### 5-Amino-pentanyl 4-acetamido-4,6-dideoxy- β -D-glucopyranosyl-(1 → 2)-2-acetamido-α-Dgalactopyranosyl-(1 → 4)-2-acetamido-α-D-galactopyranosyl-(1 → 3)-2-acetamido-2,6-dideoxy-β-D-glucopyranoside (27)

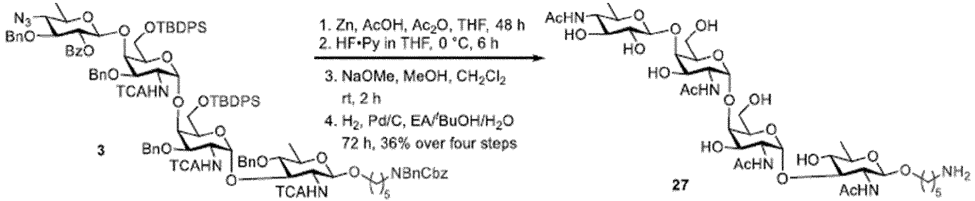

Activated zinc dust (0.1 g), acetic acid (0.2 mL) and acetic anhydride (1.0 mL) were added sequentially to the stirring solution of protected tetrasaccharide **3** (70 mg, 0.029 mmol) in THF (3 mL). The mixture was stirred for 48 h at room temperature. After completion of reaction (monitored by mass spectrometry analysis), the reaction mixture was diluted with ethyl acetate and zinc was filtered through celite pad, then the liquor was directly concentrated under reduced pressure. Toluene azeotrope was performed to remove the remaining acetic acid and kept under high *vacuum* for the next step without purification. R*f*_{product} = 0.5 (100% Ethyl acetate) To a stirring solution of crude tetrasaccharide in THF (3 mL), was added dropwise solution of HF•Py (70% in THF, 0.1 mL) at 0 °C. After 6 h and complete conversion of starting material (monitored by TLC, R*f*_{product} = 0.1 (100% Ethyl acetate)), Et₃N was added and the crude was directly concentrated under reduced pressure to get tetrasaccharide diol. Sodium methoxide (22 mg, 0.41 mmol) was added to the stirring solution of the above prepared tetrasaccharide diol in CH₂Cl₂/MeOH (1/1, *v*/*v,* 2 mL) at room temperature. After 2 h, TLC showed complete conversion of the starting material. Then Amberlite IR-120 H+ was added to neutralize the mixture. Then it was filtered out, concentrated and passed through a small bed of silica gel column chromatography (5:95 to 1:9 Methanol: Dichloromethane) to afford the debenzoylated tetrasaccharide triol compound as a colorless sticky solid (R*f*_{product} = 0.5, 5:95 Methanol:Dichloromethane)). To the crude tetrasaccharide triol compound in EA/*t*BuOH/H₂O (2/1/1, *v*/*v*/*v,* 5 mL), was added Pd/C (30 mg) at room temperature and the reaction was stirred under hydrogen atmosphere (1 atm, balloon) for 72 h.

The reaction mixture was then filtered through Celite^{®} 353, concentrated, purified by HPLC (Hypercarb Column, 150×10 mm at a flow rate of 3 mL/min, eluted by H₂O (with 0.1% formic acid, Solvent A) and acetonitrile (Solvent B) with a linear gradient from 0% to 30% of solvent B over 30 minutes and lyophilized to obtain tetrasaccharide **27** (9.5 mg, 36% over four steps) as a white solid.

¹H NMR (700 MHz, D₂O) *δ* 5.42 (d, *J* = 4.0 Hz, 1H, H-1*α*GalN), 4.92 (d, *J* = 3.9 Hz, 1H, H-1'*α*GalN), 4.60 (d, *J* = 7.9 Hz, 1H, H-1*β*Vio), 4.47 (d, *J* = 8.6 Hz, 1H, H-*1β*^{QuiN}), 4.36 (t, *J* = 6.4 Hz, 1H, H-3^{QuiN}), 4.26 (dd, *J* = 11.2, 3.8 Hz, 1H, H-2'^{GalN}), 4.23 - 4.18 (m, 2H, H-4^{GalN}, H-2^{GalN}), 4.09 (dd, *J* = 11.2, 3.1 Hz, 1H, H-3'^{GalN}), 4.00 (d, *J* = 3.2 Hz, 1H, H-4'^{GalN}), 3.89 - 3.81 (m, 4H, H-3^{GalN}, H-5'^{GalN}, -C*H*(linker)), 3.73 - 3.68 (m, 3H, H- 2^{QuiN}, H-6'a^{GalN}, H-6'b^{GalN}), 3.65 (tt, *J* = 11.4, 5.4 Hz, 3H, H-5^{GalN}, H-6a^{GalN}, H-6b^{GalN}), 3.62 - 3.58 (m, 1H, H-4^{QuiN}), 3.57 - 3.50 (m, 3H, H-4^{Vio}, H-5^{Vio}, -C*H*(linker)), 3.46 (t, *J* = 9.4 Hz, 1H, H-3^{Vio}), 3.43 - 3.38 (m, 2H, H-2^{Vio}, H-5^{QuiN}), 3.36 (t, *J* = 9.1 Hz, 1H), 2.94 (t, *J* = 7.7 Hz, 2H), 2.04 (s, 3H, -NHCOC*H*₃), 2.01 (s, 3H, -NHCOC*H*₃), 1.99 (s, 3H, -NHCOC*H*₃), 1.99 (s, 3H, -NHCOC*H*₃), 1.62 (p, *J* = 7.7 Hz, 2H, -C*H*₂(linker)), 1.56 - 1.51 (m, 2H, -C*H*₂(linker)), 1.34 (tt, *J* = 7.9, 3.8 Hz, 2H, -C*H*₂(linker)), 1.25 (d, *J* = 6.1 Hz, 3H, H-6^{Quip}), 1.16 (d, *J* = 5.9 Hz, 3H, H-6^{Vio}).

¹³C NMR (176 MHz, D₂O) *δ* 174.53, 174.47, 174.23, 170.90, 103.66 (C-1*β*^{Vio}), 100.72 (C-1*β*^{QuiN}), 98.27 (C- 1'*α*^{GalN}), 97.11 (C-1*α*^{GalN}) 77.83, 77.60, 76.26, 74.12, 73.41, 71.68, 71.45, 71.13, 70.09, 70.01, 67.50, 66.67, 60.33, 59.57, 56.46, 54.33, 50.56, 49.59, 39.21, 28.06, 26.33, 22.14, 22.07, 22.02, 21.83, 21.77, 16.63, 16.32.

HRMS (ESI): calculated for C₃₇H₆₆N₅O₁₉ [M+H]⁺: 884.4346, found: 884.4346.

### N-(Benzyl)benzyloxycarbonyl-5-amino-pentanyl 4-azido-3-O-benzyl-2-O-benzoyl-4,6-dideoxy-β-D-glucopyranosyl-(1→2)-3-O-benzyl-2-trichloro-acetamido-α-D-galactopyranosyl-(1→4)-3-O-benzyl-2-trichloroacetamido-α-D-galactopyranosyl-(1→3)-4-O-benzyl-2-trichloroacetamido-2,6-dideoxy-β-D-glucopyranoside (28)

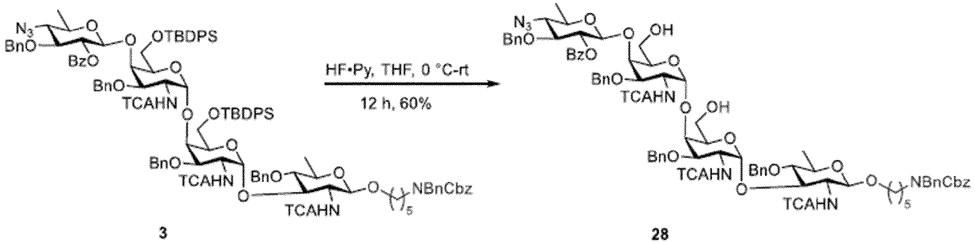

To a stirring solution of protected tetrasaccharide **3** (35 mg, 0.15 mmol) in THF (2 mL) was added dropwise solution of HF•Py (70% in THF, 0.1 mL) at 0 °C. After 12 h, Et₃N was added and the crude was directly concentrated under reduced pressure and purified by silica gel column chromatography (1:1 Ethyl acetate: *n-*Hexane to 100% Ethyl acetate) to get tetrasaccharide diol **28** (17 mg, 60%) as colorless sticky liquid.

₁H NMR (600 MHz, CDCl₃) *δ* 8.02 - 7.95 (m, 2H), 7.52 - 7.48 (m, 1H), 7.38 - 7.34 (m, 4H), 7.34 - 7.31 (m, 4H), 7.31 - 7.29 (m, 4H), 7.29 - 7.26 (m, 8H), 7.24 (td, *J =* 8.1, 1.4 Hz, 4H), 7.16 (dd, *J* = 5.0, 1.9 Hz, 3H), 7.14 - 7.12 (m, 2H), 6.81 (d, *J =* 9.0 Hz, 1H), 6.48 (s, 1H), 5.37 (s, 1H, H-1*α*^{GalN}, 5.22 - 5.10 (m, 3H), 4.87 (d, *J* = 3.7 Hz, 1H, H-1'*α*^{GalN}), 4.78 (d, *J* = 8.3 Hz, 1H, H-1*β*^{Vio}), 4.72 - 4.61 (m, 2H), 4.55 (ddd, *J* = 21.5, 11.2, 6.0 Hz, 4H), 4.49-4.42 (m, 3H, H-1*β*^{QuiN}), 4.43 - 4.35 (m, 2H), 4.34 (dd, *J* = 8.1, 2.9 Hz, 1H), 4.27 - 4.18 (m, 2H), 4.16 - 4.04 (m, 3H), 3.98 (s, 1H), 3.86 - 3.77 (m, 2H), 3.72 (s, 1H), 3.66 - 3.59 (m, 2H), 3.57 (t, *J* = 9.1 Hz, 2H), 3.45 - 3.35 (m, 2H), 3.32 (d, *J* = 7.5 Hz, 1H), 3.25 - 3.12 (m, 5H), 2.72 (t, *J* = 7.0 Hz, 1H), 1.54 - 1.40 (m, 4H), 1.35 (d, *J* = 5.7 Hz, 3H), 1.28 - 1.23 (m, 5H).

¹³C NMR (151 MHz, CDCl₃) *δ* 165.66, 162.10, 161.12, 156.49, 137.91, 137.42, 137.14, 137.12, 136.93, 133.09, 130.24, 129.77, 128.91, 128.85, 128.79, 128.72, 128.62, 128.52, 128.47, 128.42, 128.37, 128.33, 128.31, 128.29, 128.10, 128.06, 128.03, 127.92, 127.63, 127.50, 127.38, 101.17 (C-1*β*^{Vio}, 1*J*_{C-H} = 168.0 Hz), 99.99 (C-1*β*^{QuiN}, 1*J*_{C-H} = 161.0 Hz), 97.19 (C-1'*α*^{GalN}, 1*J*_{C-H} = 175.0 Hz), 96.85 (C-1*α*^{GalN}, 1*J*_{C-H} = 175.0 Hz), 92.78, 92.55, 83.97, 81.25, 77.37, 77.29, 77.16, 77.08, 76.95, 75.67, 75.26, 74.75, 73.99, 72.41, 71.93, 71.61, 71.14, 70.75, 70.21, 69.40, 67.46, 67.42, 61.20, 60.61, 56.48, 51.25, 50.38, 47.24, 46.07, 29.85, 29.22, 27.90, 27.44, 27.20, 23.32, 18.40, 18.29.

HRMS (ESI): calculated for C₈₅H₉₂Cl₉N₇O₂₁Na [M+Na]+: 1884.3435, found: 1884.3804.

### N-(Benzyl)benzyloxycarbonyl-5-amino-pentanyl 2-azido-3-O-benzyl-4,6-O-silylidene-α-Dgalactopyranosyl-(1→3)-4-O-benzyl-2-trichloroacetamido-2,6-dideoxy-β-D-glucopyranoside (31)

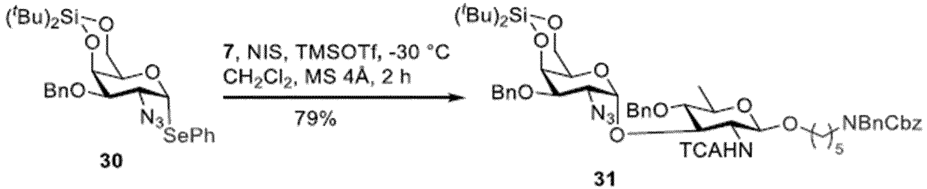

Anhydrous CH₂Cl₂ (21 mL) was added to the mixture of D-galactosamine donor **30** (0.73 g, 1.27 mmol), D-quinosamine acceptor **7** (0.600 g, 0.847 mmol) and molecular sieves (2.0 g) and this solution was kept stirring at room temperature for 0.5 h. Then NIS (0.286 g, 1.27 mmol) and TMSOTf (16 *µ*L, 0.085 mmol) were added to the stirring solution at -30 °C and it was stirred for another 2 h. After completion of reaction (consumption of both donor and acceptor were monitored by TLC), Et₃N was added and 4Å molecular sieves were filtered through a celite bed and the reaction mixture was diluted with CH₂Cl₂ (30 mL) and washed with saturated Na₂S₂O₃ solution. The separated organic layer was dried over anhydrous Na₂SO₄, concentrated and purified by silica gel column chromatography (1:4 Ethyl acetate: n-Hexane) to give protected disaccharide **31** (0.751 g, 79%) as white foam.

¹H NMR (400 MHz, CDCl₃) *δ* 7.43 - 7.39 (m, 2H, -ArH), 7.38 - 7.35 (m, 3H, -ArH), 7.33 (d, *J* = 2.9 Hz, 5H, -ArH), 7.32 - 7.29 (m, 5H, -ArH), 7.28 (t, *J* = 1.7 Hz, 2H, -ArH), 7.26 (d, *J* = 1.7 Hz, 1H, -ArH), 7.15 (dd, *J* = 14.7, 7.1 Hz, 2H, -ArH), 5.55 (s, 1H, H-1*α*^{GalN}), 5.18 (d, *J* = 11.0 Hz, 2H, -C*H*₂Cbz), 4.94 (d, *J* = 10.6 Hz, 1H, -C*H*Ph), 4.74 - 4.62 (m, 4H, - C*H*₂Ph, -CHPh, H-1*β*^{QuiN}), 4.52 - 4.41 (m, 3H, H-5^{GalN3}, -C*H*₂Ph), 4.42-4.15 (m, 1H, H-3^{QuiN}), 4.12 (d, *J* = 2.8 Hz, 2H, -C*H*₂(linker)), 3.89 - 3.77 (m, 3H, H-2^{GalN3}, H-4^{GalN3}, H-6a^{GalN3}), 3.73 - 3.62 (m, 2H, H-6b^{GalN3}, H-2^{QuiN}), 3.56 - 3.47 (m, 1H, H-5^{QuiN}), 3.38 (q, *J* = 9.5 Hz, 2H, H-4^{QuiN}, H-3^{GalN3}), 3.20 (dt, *J* = 30.0, 7.6 Hz, 2H, -C*H*₂(linker)), 1.58 - 1.47 (m, 4H, -C*H*₂(linker)), 1.41 (d, *J =* 6.2 Hz, 3H, H-6^{QuiN}), 1.25-1.22 (m, 2H, -C*H*₂(linker)), 1.04 (s, 9H, -C(C*H*₃)₃), 0.98 (s, 9H, -C(C*H*₃)₃).

¹³C NMR (101 MHz, CDCl₃) *δ* 161.60, 156.75, 156.24, 137.85, 137.68, 137.58, 136.89, 136.66, 128.60, 128.57, 128.50, 128.06, 127.99, 127.96, 127.86, 127.63, 127.36, 127.25, 99.40 (C-1*β*^{QuiN}), 97.76 (C-1*α*^{GalN}), 92.55, 84.87, 84.68, 77.41, 77.30, 77.09, 76.78, 75.91, 75.46, 74.29, 71.37, 70.65, 69.78, 69.52, 67.70, 67.37, 67.23, 58.08, 57.52, 50.28, 47.12, 46.12, 31.98, 29.75, 27.63, 27.29, 23.48, 23.33, 23.20, 22.75, 20.73, 18.30.

HRMS (ESI): calculated for C₅₆H₇₂Cl₃N₅O₁₁SiNa [M+Na]⁺: 1146.3955, found: 1146.4083.

### N-(Benzyl)benzyloxycarbonyl-5-amino-pentanyl 2-azido-3-O-benzyl-α-D-galactopyranosyl- (1→3)-4-O-benzyl-2-trichloroacetamido-2,6-dideoxy-β-D-glucopyranoside (32)

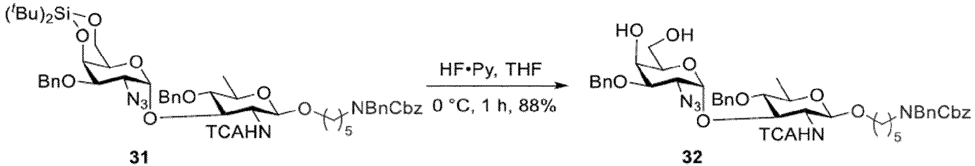

To a stirring solution of protected disaccharide **31** (0.552 g, 0.496 mmol) in THF (4.9 mL) was added dropwise solution of HF•Py (70% in THF, 0.11 mL) at 0 °C. After 1 h, Et₃N was added and crude was directly concentrated under reduced pressure and purified by silica gel column chromatography (1:1 Ethyl acetate: *n*-Hexane to 100% Ethyl acetate) to get disaccharide diol **32** (0.425 g, 88%) as white foam.

¹H NMR (400 MHz, CDCl₃) *δ* 7.89 - 7.46 (m, 1H, -N*H*), 7.41 7.25 (m, 19H, -ArH), 7.22 - 7.14 (m, 1H, -ArH), 5.45 (d, *J* = 3.7 Hz, 1H, H-1*α*^{GalN3}), 5.18 (d, *J* = 16.7 Hz, 2H, -C*H*₂Cbz), 4.95 (d, *J* = 10.7 Hz, 1H, -C*H*Ph), 4.72 - 4.60 (m, 4H, H-1*β*^{QuiN}, -C*H*₂Ph, -C*H*Ph), 4.53-4.43 (m, 2H, -C*H*₂Ph), 4.22 - 4.14 (m, 1H, H-3^{QuiN}), 3.98 (s, 2H, H-4^{GalN3}, H-5^{GalN3}), 3.88 (dd, *J* = 10.5, 3.0 Hz, 2H, H-3^{GalN3}, H-6a^{GalN3}), 3.84 - 3.70 (m, 4H, H-2^{GalN3}, H-6b^{GalN3}, H- 2^{QuiN}, -C*H*(linker)), 3.54 (t, *J* = 7.6 Hz, 1H, H-5^{QuiN}), 3.36 (q, *J* = 9.5 Hz, 2H, -C*H*(linker), H-4^{QuiN}), 3.30 - 3.13 (m, 2H, -C*H*₂(linker)), 2.88 (bs, 1H, -OH), 1.55 (s, 4H, -C*H*₂(linker)), 1.39 (d, *J* = 6.2 Hz, 3H, H-6^{QuiN}), 1.32 - 1.25 (m, 2H, -C*H*₂(linker)).

¹³C NMR (101 MHz, CDCl₃) *δ* 162.01, 156.93, 156.38, 137.74, 137.69, 137.03, 136.78, 136.55, 128.72, 128.60, 128.53, 128.34, 128.15, 128.02, 127.84, 127.76, 127.38, 99.66 (C-1*β*^{QuiN}), 97.62 (C-1*α*^{GalN3}), 92.73, 84.24, 77.49, 77.37, 77.17, 76.85, 76.07, 74.56, 72.00, 71.08, 70.17, 69.42, 68.00, 67.31, 67.26, 62.93, 60.49, 59.24, 57.64, 50.40, 50.19, 47.15, 45.95, 29.73, 29.13, 27.75, 27.35, 25.62, 23.28, 23.08, 21.11, 18.17.

HRMS (ESI): calculated for C₄₈H₅₆Cl₃N₅O₁₁Na [M+Na]⁺: 1006.2934, found: 1006.2846.

### N-(Benzyl)benzyloxycarbonyl-5-amino-pentanyl 2-azido-3-O-benzyl-6-O-tertbutyldiphenylsilyl-α-D-galactopyranosyl-(1→3)-4-O-benzyl-2-trichloroacetamido-2,6-dideoxy-β-D-glucopyranoside (33)

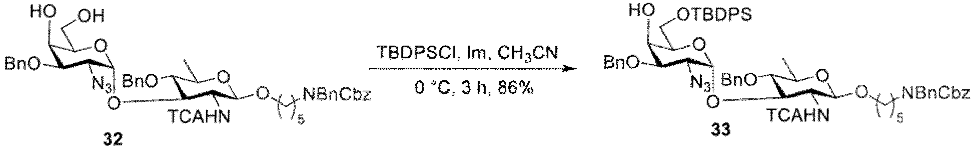

To the stirring solution of disaccharide diol **32** (0.301 g, 0.306 mmol) in CH₃CN (3.0 mL), imidazole (41.5 mg, 0.61 mmol) and TBDPSCI (0.17 mL, 0.672 mmol) were added sequentially at 0 °C. After 3 h and complete consumption of the starting material (indicated by TLC), the crude reaction mixture was diluted with EtOAc (20 mL) and washed with brine solution. The separated organic layer was dried over anhydrous Na₂SO₄, concentrated and purified by silica gel column chromatography (3:7 Ethyl acetate: *n*-Hexane) to afford 4-OH disaccharide acceptor **33** (0.321 g, 86%) as white foam.

¹H NMR (400 MHz, CDCI₃) *δ* 7.69 (dd, *J* = 6.6, 1.5 Hz, 4H, -ArH), 7.48 - 7.43 (m, 2H, -ArH), 7.43 - 7.29 (m, 23H, -ArH), 7.19 (d, *J* = 7.6 Hz, 1H, -ArH), 6.91 (dd, J = 57.1, 7.4 Hz, 1H, -NH), 5.53 (d, *J* = 3.3 Hz, 1H, H-1aGaIN3), 5.20 (d, *J* = 13.9 Hz, 2H, - CH2Cbz), 4.95 (d, *J* = 10.7 Hz, 1H, -CHPh), 4.78 - 4.65 (m, 3H, -CH2Ph, -CHPh), 4.60-4.55 (m, 1H, H- 1*β*QuiN), 4.51 (d, *J* = 6.8 Hz, 2H, -CH2Ph), 4.12 (d, *J* = 8.7 Hz, 1H, H-3QuiN), 4.06 (s, 1H, H-4GalN3), 3.96 (s, 1H, H-5GaIN3), 3.88 (tdd, *J* = 7.4, 4.1, 2.5 Hz, 3H, H-2QuiN, H-3GalN3, H-6aGaIN3), 3.79 (ddd, *J* = 10.5, 3.8, 1.4 Hz, 3H, H-2GalN3, H-6bGaIN3, -CH(linker)), 3.52 - 3.42 (m, 1H, H-5QuiN), 3.38 (t, *J =* 8.7 Hz, 2H, H-4QuiN, -C*H*(linker)), 3.22 (d, *J* = 29.7 Hz, 2H, -C*H*2(linker)), 2.66 (d, *J* = 11.6 Hz, 1H, -OH), 1.62 - 1.47 (m, 4H, -C*H*2(linker)), 1.43 (d, *J* = 6.2 Hz, 3H, H-6QuiN), 1.31 (d, *J =* 7.6 Hz, 2H, -C*H*2(linker)), 1.10 (s, 9H, -C(CH3)3).

¹³C NMR (101 MHz, CDCI₃) *δ* 161.74, 156.80, 137.98, 137.68, 135.85, 135.79, 132.94, 132.85, 130.02, 130.00, 128.77, 128.66, 128.58, 128.32, 128.16, 128.05, 127.94, 127.90, 127.74, 127.41, 99.77 (C-1*β*QuiN), 97.38 (C-1*a*GalN3), 92.56, 84.65, 77.48, 77.36, 77.16, 76.84, 76.03, 74.40, 72.01, 71.40, 70.34, 69.63, 67.25, 66.86, 64.10, 59.11, 57.52, 50.61, 47.19, 46.24, 29.26, 27.17, 23.35, 19.29, 18.21.

HRMS (ESI): calculated for C₆₄H₇₅Cl₃N₅O₁₁Si[M+H]⁺: 1222.4293, found: 1222.4196.

### N-(Benzyl)benzyloxycarbonyl-5-amino-pentanyl2-azido-3-O-benzyl-4,6-O-silylidene-α-Dgalactopyranosyl-( 1→4)-2-azido-3-O-benzyl-6-O-tert-butyldiphenylsilyl-α-Dgalactopyranosyl-(1→3)-4-O-benzyl-2-trichloroacetamido-2,6-dideoxy-β-D-glucopyranoside (34)

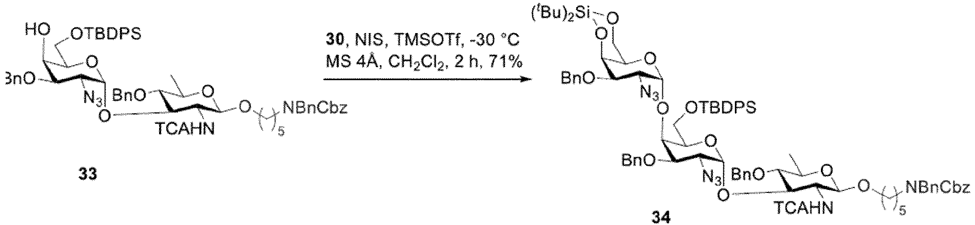

Anhydrous CH₂Cl₂ (14 mL) was added to the mixture of D-galactosamine donor 30 (0.479 g, 0.833 mmol), disaccharide acceptor 33 (0.68 g, 0.556 mmol) and molecular sieves (1 g) and this solution was kept stirring at room temperature for 0.5 h. Then NIS (0.187 g, 0.833 mmol) and TMSOTf (20 µL, 0.112 mmol) were added to the stirring solution at -30 °C and it was stirred for another 2 h. After completion of reaction (consumption of both donor and acceptor were monitored by TLC), Et₃N was added and 4Å molecular sieves were filtered through a celite bed and the reaction mixture was diluted with CH₂Cl₂ (20 mL) and washed with saturated Na₂S2O₃ solution. The separated organic layer was dried over anhydrous Na₂SO₄, concentrated and purified by silica gel column chromatography (1:4 Ethyl acetate: n-Hexane) to give protected trisaccharide **34** (647 mg, 71%) as a colorless sticky solid. ¹H NMR (400 MHz, CDCI₃) *δ* 7.55 (tt, *J* = 5.0, 2.5 Hz, 4H), 7.36 - 7.29 (m, 6H), 7.28 - 7.23 (m, 9H), 7.22 - 7.16 (m, 11H), 7.15 - 7.12 (m, 3H), 7.10 - 7.03 (m, 2H), 6.74-6.56 (m, 1H), 5.27 (d, *J* = 3.4 Hz, 1H, H-1*α*GalN3), 5.06 (d, *J =* 12.1 Hz, 2H), 4.91 (d, *J* = 2.9 Hz, 1H, H-1'*α*GaIN3), 4.75 (d, *J* = 10.8 Hz, 1H), 4.62 (d, *J* = 11.2 Hz, 1H), 4.50 (d, *J* = 10.9 Hz, 3H), 4.45 (d, *J* = 10.8 Hz, 2H, H-1*β*QuiN), 4.41 - 4.32 (m, 3H), 4.16 (s, 1H), 4.03 (t, *J* = 8.9 Hz, 1H), 3.95 - 3.86 (m, 2H), 3.85 - 3.75 (m, 2H), 3.75 - 3.66 (m, 4H), 3.63 (d, *J* = 3.4 Hz, 1H), 3.61 - 3.53 (m, 2H), 3.50 (dd, *J* = 10.6, 3.5 Hz, 1H), 3.45 (dd, *J* = 7.2, 3.8 Hz, 1H), 3.41 - 3.33 (m, 1H), 3.28 - 3.18 (m, 1H), 3.18 - 3.08 (m, 2H), 3.04 (t, *J* = 7.3 Hz, 1H), 1.41 (t, *J* = 7.8 Hz, 4H), 1.24 (d, *J* = 6.2 Hz, 3H), 1.16 (d, *J* = 6.7 Hz, 2H), 0.97 (s, 9H), 0.89 (d, *J* = 2.0 Hz, 18H).

¹³C NMR (101 MHz, CDCI₃) *δ* 161.61, 156.81, 156.26, 138.01, 137.98, 137.78, 137.72, 137.04, 135.70, 135.65, 132.89, 132.84, 130.30, 130.20, 129.17, 128.73, 128.67, 128.60, 128.56, 128.36, 128.13, 128.09, 128.05, 128.02, 127.99, 127.96, 127.47, 127.33, 125.43, 99.53 (C-1*β*QuiN), 98.54 (C- 1'aGaIN3), 97.49 (C-1*a*GalN3), 92.56, 83.71, 77.48, 77.36, 77.16, 76.84, 75.50, 74.73, 71.80, 71.15, 70.28, 69.50, 67.59, 67.27, 67.12, 62.20, 60.31, 58.05, 57.71, 50.61, 50.30, 47.18, 46.25, 29.84, 29.35, 27.71, 27.41, 27.19, 23.44, 21.61, 20.76, 19.33, 18.21.

HRMS (ESI): calculated for C₈₅H₁₀₅Cl₃N₈O₁₅Si₂Na [M+Na]⁺: 1661.6196, found: 1661.6432.

### N-(Benzyl)benzyloxycarbonyl-5-amino-pentanyl 2-azido-3-O-benzyl-α-D-galactopyranosyl- (1→4)-2-azido-3-O-benzyl-α-D-galactopyranosyl-(1→3)-4-O-benzyl-2-trichloroacetamido- 2,6-dideoxy-β-D-glucopyranoside (35)

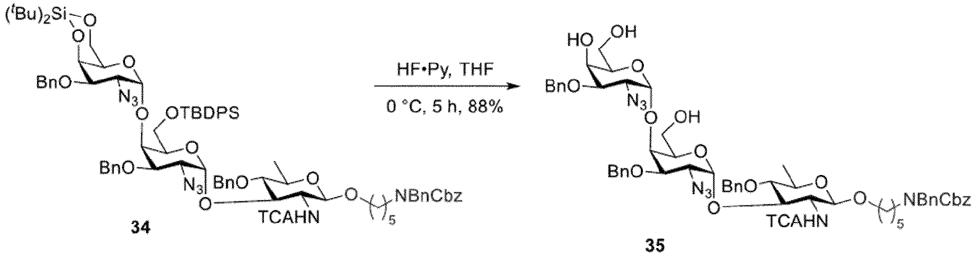

To a stirring solution of protected trisaccharide **34** (0.475 g, 0.289 mmol) in THF (10 mL) was added dropwise solution of HF•Py (70% in THF, 0.5 mL) at 0 °C. After 5 h, Et₃N was added and the crude was directly concentrated under reduced pressure and purified by silica gel column chromatography (100% ethyl acetate to 5:95 Methanol: CH₂Cl₂) to get trisaccharide triol **35** (0.321 g, 88%) as white foam.

¹H NMR (700 MHz, CDCI₃) *δ* 7.38 - 7.35 (m, 8H), 7.35 - 7.29 (m, 11H), 7.29 - 7.23 (m, 5H), 7.16 (d, *J* = 6.5 Hz, 1H), 5.41 (s, 1H, H-1*a*^{GalN3}) , 5.17 (d, *J* = 24.0 Hz, 2H), 4.95-4.92 (m, 2H, H-1'*a*^{GalN3}), 4.74 (d, *J* = 11.7 Hz, 2H, H-1*β*^{QuiN}), 4.69 (s, 2H), 4.63 (dd, *J* = 16.9, 11.1 Hz, 2H), 4.53 - 4.42 (m, 2H), 4.22 - 4.11 (m, 3H), 4.04 (q, *J =* 6.2 Hz, 2H), 3.92 (td, *J =* 12.0, 3.2 Hz, 2H), 3.87 (dd, *J =* 10.7, 2.9 Hz, 1H), 3.83 (q, *J* = 3.8 Hz, 1H), 3.72 (td, *J =* 10.3, 3.7 Hz,

4H), 3.59 - 3.51 (m, 3H), 3.42 - 3.32 (m, 2H), 3.28 - 3.17 (m, 2H), 1.56 - 1.45 (m, 4H), 1.37 (d, *J* = 6.2 Hz, 3H), 1.33 - 1.29 (m, 2H).

¹³C NMR (176 MHz, CDCI₃) *δ* 162.02, 157.11, 156.50, 137.86, 137.77, 137.10, 136.99, 136.62, 134.91, 134.53, 134.51, 128.80, 128.75, 128.68, 128.62, 128.59, 128.46, 128.27, 128.24, 128.21, 128.10, 128.00, 127.95, 127.92, 127.89, 127.86, 127.84, 127.81, 127.70, 127.45, 127.31, 125.40, 99.43 (C-1*β*QuiN), 99.02 (C-1'*a*GalN3), 97.88 (C-1*a*^{GalN3}), 92.80, 84.02, 83.80, 77.76, 77.34, 77.16, 76.98, 75.84, 75.52, 74.50, 74.42, 73.49, 72.17, 71.80, 71.78, 71.71, 71.00, 69.68, 69.54, 67.51, 67.41, 62.87, 61.34, 60.09, 59.35, 57.91, 57.80, 50.39, 50.24, 47.23, 45.85, 32.03, 31.78, 29.80, 29.14, 27.70, 27.40, 26.08, 23.38, 23.03, 18.24.

HRMS (ESI): calculated for C₆₁H₇₁Cl₃N₈O₁₅Na [M+Na]⁺: 1283.3996, found: 1283.3840.

### N-(Benzyl)benzyloxycarbonyl-5-amino-pentanyl 2-azido-3-O-benzyl-6-Opentafluorophenyl-o-D-galactopyranosyl uronate-(1→4)-2-azido-3-O-benzyl-6-Opentafluorophenyl-o-D-galactopyranosyl uronate-(1→3)-4-O-benzyl-2-trichloroacetamido-2,6-dideoxy-β-D-glucopyranoside (36)

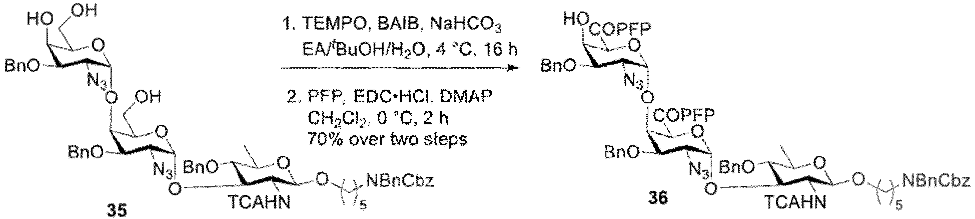

TEMPO (64 mg, 0.408 mmol) and NaHCOs (214 mg, 2.55 mmol) were added sequentially to a stirring solution of trisaccharide triol **35** (321 mg, 0.255 mmol) in EA/*t*BuOH/H₂O (1/1/1, *v*/*v*/*v,* 5.25 mL) at 0 °C. After 10 minutes, BAIB (658 mg, 2.04 mmol) were added at same temperature. After addition of all reagents, the reaction mixture was warmed up to 4 °C and allowed to stir at the same temperature for 16 h. After complete conversion of alcohol to acids (monitored by TLC), the reaction mixture was diluted with ethyl acetate (20 mL) and then quenched by addition of saturated aqueous Na₂S₂O₃ (2 mL) and aqueous NaH₂PO₄ (0.5 mL). The organic layer was washed with brine solution and aqueous layer was extracted with ethyl acetate (10 mL) three times. The separated organic layer was dried over anhydrous Na₂SO₄, concentrated and dried under high *vacuum* to get crude carboxylate compound as colorless liquid. To the *vacuum* dried crude carboxylate compound in anhydrous CH₂Cl₂ (6 mL), pentafluoro phenol (469 mg, 2.55 mmol) and catalytic amounts of DMAP (6.2 mg, 0.051 mmol) were added sequentially at 0 °C. After that, EDC•HCl (459 mg, 2.39 mmol) was added to the reaction mixture at same temperature. After 2 h and completion of reaction (monitored by TLC), the crude reaction mixture was diluted with CH₂Cl₂ (20 mL) and washed with brine solution two times. The separated organic layer was dried over anhydrous Na₂SO₄, concentrated and purified by silica gel column chromatography (1:4 Ethyl acetate: n-Hexane) to obtain trisaccharide diester **36** (214 mg, 70% over two steps) as a white solid.

¹H NMR (600 MHz, CDCI₃) *δ* 7.46 - 7.40 (m, 5H), 7.39 (d, *J* = 7.0 Hz, 3H), 7.36 - 7.31 (m, 9H), 7.29 (d, *J* = 1.3 Hz, 7H), 7.22 - 7.15 (m, 1H), 5.69 (d, *J* = 3.6 Hz, 1H, H-1aGaIN3), 5.36 - 5.28 (m, 2H, H-1'aGalN3), 5.23 - 5.12 (m, 2H), 4.90 - 4.77 (m, 3H), 4.77 - 4.65 (m, 4H), 4.65 - 4.55 (m, 2H, H-1*β*QuiN), 4.55 - 4.43 (m, 3H), 4.08 (t, *J* = 8.4 Hz, 1H), 4.02 (dd, *J* = 10.4, 3.2 Hz, 1H), 3.98 - 3.91 (m, 1H), 3.91 - 3.80 (m, 2H), 3.77 (d, *J* = 7.9 Hz, 1H), 3.69 - 3.58 (m, 2H), 3.45 - 3.16 (m, 4H), 1.63 (d, *J* = 4.7 Hz, 3H), 1.57 - 1.47 (m, 4H), 1.36 (d, *J* = 6.4 Hz, 3H).

¹³C NMR (151 MHz, CDCl₃) *δ* 164.69, 163.80, 161.90, 156.36, 141.99, 138.90, 137.84, 137.34, 136.76, 135.89, 129.08, 128.92, 128.88, 128.69, 128.67, 128.61, 128.53, 128.16, 128.11, 128.00, 127.94, 127.86, 127.80, 127.75, 127.45, 127.29, 124.37, 100.05 (C-1'aGalN3), 99.41(C-1*β*QuiN), 98.03 (C-1*a*GalN3), 92.33, 83.23, 77.37, 77.30, 77.16, 77.08, 76.95, 75.89, 73.95, 72.60, 72.50, 72.11, 71.01, 70.89, 70.51, 69.19, 67.32, 67.08, 58.84, 58.70, 50.62, 50.37, 47.13, 46.21, 32.04, 29.81, 29.48, 29.19, 29.04, 27.91, 27.28, 23.15, 22.81, 18.37, 14.25.

HRMS (ESI): calculated for C₇₃H₆₅Cl₃F₁₀N₈O₁₇Na [M+Na]⁺: 1643.3266, found: 1643.3289.

### 5-Amino-pentanyl 2-acetamido-o-D-galactopyranosyl uronamide-(1→4)-2-acetamido-α-Dgalactopyranosyl uronamide-(1→3)-2-acetamido-2,6-dideoxy-β-D-glucopyranoside (37)

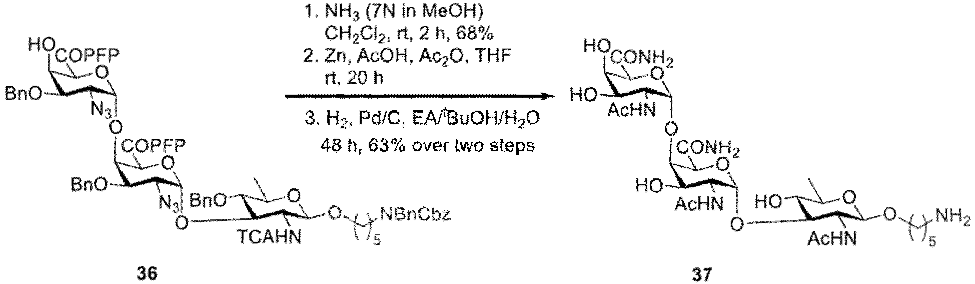

Ammonia solution (7N in methanol, 3.1 mL) was added dropwise to a stirred solution of compound **36** (32 mg, 0.0197 mmol) in CH₂Cl₂ (0.6 mL) at room temperature. After 2 h and complete consumption of the starting material (monitored by mass spectrometry analysis), the crude was directly concentrated under reduced pressure and the compound was purified by silica gel column chromatography (5:95 Methanol: Dichloromethane) to get trisaccharide diamide compound (17 mg, 68%) as colorless foam.

¹H NMR (600 MHz, CDCI₃) *δ* 7.39 - 7.34 (m, 8H), 7.34 - 7.28 (m, 15H,), 7.18 (s, 2H), 6.74 - 6.67 (m, 1H), 6.45 (s, 1H), 5.52 (d, *J* = 3.7 Hz, 1H, H-1*a*GalN3), 5.37 - 5.29 (m, 2H, H-1'aGalN3), 5.16 (s, 2H), 4.80 - 4.74 (m, 2H), 4.74 - 4.69 (m, 3H), 4.66 (dd, *J* = 13.6, 11.0 Hz, 3H, H-1*β*QuiN), 4.60 - 4.53 (m, 2H), 4.49 (s, 2H), 4.40 (s, 1H), 4.17 (s, 1H), 3.86 (ddd, *J* = 29.3, 10.7, 2.9 Hz, 2H), 3.79 - 3.66 (m, 3H), 3.63 (dd, *J =* 10.5, 3.7 Hz, 1H), 3.55 (dd, *J =* 10.8, 3.7 Hz, 1H), 3.41 (t, *J =* 6.9 Hz, 1H), 3.36 - 3.16 (m, 3H), 2.32 (s, 1H), 1.33 (d, *J* = 6.5 Hz, 3H), 1.24 - 1.21 (m, 2H).

¹³C NMR (151 MHz, CDCI₃) *δ* 171.04, 170.14, 162.23, 156.88, 138.01, 137.34, 136.97, 136.33, 134.95, 129.99, 129.80, 128.86, 128.83, 128.79, 128.70, 128.63, 128.47, 128.30, 128.26, 128.11, 128.00, 127.97, 127.91, 127.87, 127.45, 127.36, 122.22, 98.97 (C-1'*a*GalN3, 1*J*C-H = 168.0 Hz), 98.87 (C-1*a*GalN3, 1*J*C-H = 175.0 Hz, C-1*β*QuiN, 1*J*C-H = 168.0 Hz), 92.49, 83.05, 74.83, 73.93, 72.42, 71.83, 71.59, 71.48, 69.66, 67.33, 66.52, 58.93, 58.52, 56.28, 50.41, 47.28, 46.21, 32.08, 29.85, 29.51, 29.06, 27.46, 26.70, 23.59, 23.33, 22.84, 18.71, 14.27.

MALDI-QToF-MS: calculated for C₆₁H₆₉Cl₃N₁₀O₁₅K [M+K]+: 1327.7263, found: 1327.612.

Activated zinc dust (30 mg), acetic acid (0.2 mL) and acetic anhydride (0.5 mL) were added sequentially to a stirring solution of the diamide compound (12 mg, 0.009 mmol) in THF (2 mL). The mixture was stirred for 20 h at room temperature. After completion of reaction (monitored my MALDI-ToF MS analysis), the reaction mixture was diluted with ethyl acetate and zinc was removed by filtering through a celite pad, then the liquor was directly concentrated under reduced pressure. Toluene azeotrope was performed to remove the remaining acetic acid and the solute was passed through silica gel column chromatography (100% Ethyl acetate to 10% Methanol:CH₂Cl₂) to get triacetamido trisaccharide diamide compound as viscous liquid. The obtained crude triacetamido diamide trisaccharide compound was dissolved in EA /*t*BuOH/H₂O (2/1/1, *v*/*v*/*v,* 2 mL). To this solution, a suspension of Pd/C (30 mg) was added and stirred under hydrogen atmosphere (1 atm, balloon) for 48 h. The reaction mixture was then filtered through Celite^{®} 353, concentrated and purified by HPLC (Hypercarb Column, 150×10 mm at a flow rate of 3 mL/min, eluted by H₂O (with 0.1% formic acid, Solvent A) and acetonitrile (Solvent B) with a linear gradient from 0% to 30% of solvent B over 30 minutes and lyophilized to afford trisaccharide diamide **37** (4.2 mg, 63% over two steps) as a white powder.

¹H NMR (600 MHz, D₂O) *δ* 5.42 (d, *J* = 4.0 Hz, 1H, H-1*a*GalN), 5.04 (d, *J* = 4.0 Hz, 1H, H-1'*a*GalN), 4.89 (d, *J* = 1.5 Hz, 1H, H-5'GaIN), 4.50 (d, *J* = 8.4 Hz, 1H, H-5GaIN), 4.46 - 4.41 (m, 1H, H-1*β*Quip), 4.31 - 4.24 (m, 3H, H-2GaIN, H-4GaIN, H-4'GaIN), 4.19 (dd, *J* = 11.1, 3.8 Hz, 1H, H-2'GaIN), 4.08 - 4.03 (m, 2H, H-3'GaIN, H-3GaIN), 3.87 (dt, *J* = 10.2, 6.0 Hz, 1H, -CH(linker)), 3.72 (d, *J* = 9.4 Hz, 1H, H-2QuiN), 3.64 (t, *J* = 9.3 Hz, 1H, H-3QuiN), 3.59 - 3.54 (m, 1H, -CH(linker)), 3.47 (dd, *J* = 9.7, 6.2 Hz, 1H, H-5QuiN), 3.41 (d, *J* = 8.5 Hz, 1H, H-4QuiN), 3.00 - 2.95 (m, 2H, -C*H*2(linker)), 2.06 (d, *J* = 5.1 Hz, 6H, -NHCOCH3), 1.95 (s, 3H, -NHCOCH3), 1.67 (p, *J* = 7.7 Hz, 2H, -C*H*2(linker)), 1.62 - 1.54 (m, 2H, -C*H*2(linker)), 1.42 - 1.35 (m, 2H, -C*H*2(linker)), 1.31 (d, *J* = 6.2 Hz, 3H, H-6QuiN).

¹³C NMR (176 MHz, D₂O) *δ* 174.60, 174.34, 174.16, 174.13, 172.98, 100.91 (C-1*β*QuiN), 98.24 (C- 1aGaIN), 97.72 (C-1'aGaIN), 79.78, 76.15, 75.16, 71.73, 71.22, 70.28, 70.15, 68.66, 66.85, 66.20, 54.59, 49.23, 49.06, 39.22, 28.10, 26.36, 22.10, 22.04, 21.93, 21.87, 16.39.

HRMS (ESI): calculated for C₂₉H₅₁N₆O₁₅ [M+H]+: 723.3406 found: 723.3408.

### 4-Methyl phenyl 3-O-benzyl-2-O-benzoyl-4-N-(9-fluorenylmethyloxycarbonyl)-4,6-dideoxy-1-thio-β-D-glucopyranoside (38)

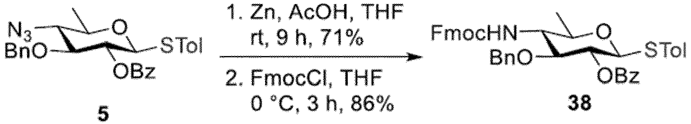

Acetic acid (0.6 mL) and activated zinc dust (600 mg) were added sequentially to the stirring solution of compound **5** (0.56 g, 1.14 mmol) in THF (6 mL) at room temperature. The reaction was kept stirring at same temperature for 9 h. After completion of reaction (indicated by TLC), zinc dust was removed by filtering through a celite bed and the reaction mixture was washed with EtOAc. Solvents were removed under reduced pressure and purified by silica gel column chromatography (1:1 Ethyl acetate: n-Hexane) to afford 4-amino compound (0.376 mg, 71%) as white foam.

¹H NMR (400 MHz, CDCI₃) *δ* 8.30 (d, *J* = 6.9 Hz, 2H, -ArH), 7.83 - 7.76 (m, 1H, - ArH), 7.71 - 7.63 (m, 2H, -ArH), 7.54 (d, *J* = 8.2 Hz, 2H, -ArH), 7.46 - 7.34 (m, 5H, - ArH), 7.27 (d, *J* = 8.2 Hz, 2H, -ArH), 5.49 (t, *J* = 9.5 Hz, 1H, H-2), 4.94 (d, *J* = 9.9 Hz, 1H, H-1), 4.87 (d, *J =* 11.1 Hz, 1H, -CHPh), 4.72 (d, *J =* 11.1 Hz, 1H, -CHPh), 3.74 (t, *J* = 9.3 Hz, 1H, H-3), 3.55 (dq, *J* = 9.3, 6.1 Hz, 1H, H-5), 2.93 (t, *J* = 9.5 Hz, 1H, H-4), 2.51 (s, 3H, -CH3(STol)), 1.55 (d, *J* = 6.1 Hz, 3H, H-6).

¹³C NMR (101 MHz, CDCI₃) *δ* 165.44, 138.12, 137.74, 133.38, 133.14, 130.06, 129.97, 129.68, 128.62, 128.60, 128.25, 128.09, 86.89 (C-1*β*), 84.49, 77.48, 77.16, 76.84, 74.63, 73.33, 57.96, 21.27, 18.42.

Then amine compound (0.526 mg, 1.14 mmol) was dissolved in anhydrous THF (5 mL) and 9-fluorenylmethoxycarbonylchloride (FmocCl, 0.89 g, 3.43 mmol) was added to the solution at 0 °C. The reaction was kept stirring at same temperature for 3 h. After completion of reaction, the crude was diluted in EtOAc (20 mL) and washed with aq. NaHCOs solution. The separated organic layer was dried over anhydrous Na₂SO₄, concentrated and purified by silica gel column chromatography (3:7 Ethyl acetate: n-Hexane) to afford compound **38** (0.67 g, 86%) as white foam.

¹H NMR (400 MHz, CDCI₃) *δ* 8.09 (t, *J* = 8.6 Hz, 2H, -ArH), 7.76 (dd, *J* = 7.5, 3.4 Hz, 2H, -ArH), 7.58 (q, *J* = 6.6 Hz, 4H, -ArH), 7.47 (t, *J* = 7.8 Hz, 1H, -ArH), 7.42 - 7.35 (m, 3H, - ArH), 7.33 - 7.24 (m, 3H, -ArH), 7.23 - 7.20 (m, 1H, -ArH), 7.13 - 7.06 (m, 5H, -ArH), 7.02 - 6.94 (m, 1H, -ArH), 5.26 (t, *J* = 9.4 Hz, 1H, H-2), 4.94 (d, *J* = 9.8 Hz, 1H, H-1), 4.76 (d, *J* = 10.0 Hz, 1H, -CHPh), 4.53 - 4.41 (m, 3H, -CHPh, - CH2Fmoc), 4.18 (q, *J* = 6.0 Hz, 1H, -CH Fmoc), 4.13 - 3.98 (m, 1H, H-3), 3.86 - 3.72 (m, 1H, H-4), 3.28 (q, *J* = 9.8 Hz, 1H, H-5), 2.38 (d, *J* = 37.9 Hz, 3H, -CH3(STol)), 1.29 (d, *J* = 7.1, 3H, H-6).

¹³C NMR (101 MHz, CDCI₃) *δ* 165.25, 155.81, 143.76, 141.43, 138.19, 137.46, 133.89, 133.36, 133.14, 129.96, 129.66, 128.55, 128.36, 128.25, 127.85, 127.18, 124.95, 120.12, 86.43 (C-1*β*), 79.41, 77.48, 77.36, 77.16, 76.84, 74.78, 73.62, 73.02, 66.48, 58.47, 47.35, 21.26, 18.25.

HRMS (ESI): calculated for C₄₂H₃₉NO₆SNa [M+Na]⁺: 708.2390, found: 708.2197.

### N-(Benzyl)benzyloxycarbonyl-5-amino-pentanyl 3-O-benzyl-2-O-benzoyl-4-N-(9-fluorenylmethyloxycarbonyl)-4,6-dideoxy-β-D-glucopyranosyl-(1→2)-2-azido-3-O-benzyl-6-O-pentafluorophenyl-α-D-galactopyranosyl uronate-(1→4)-2-azido-3-O-benzyl-6-O-pentafluorophenyl-α-D-galactopyranosyl uronate-(1→3)-4-O-benzyl-2-trichloroacetamido-2,6-dideoxy-β-D-glucopyranoside (39)

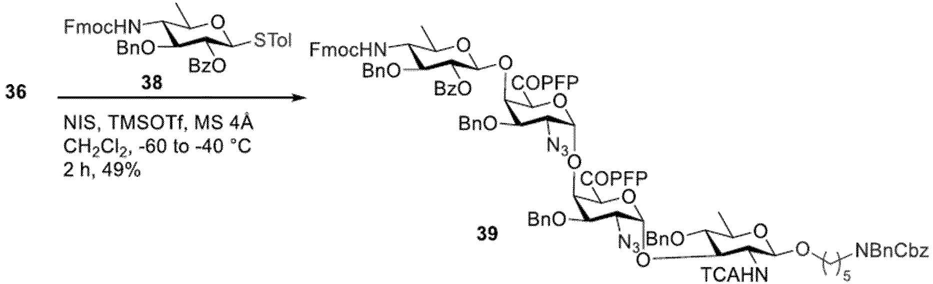

Anhydrous CH₂Cl₂ (2 mL) was added to the mixture of D-viosamine donor **38** (65 mg, 0.095 mmol), trisaccharide acceptor **36** (50 mg, 0.031 mmol) and molecular sieves (100 mg) and this solution was kept stirring at room temperature for 0.5 h. Then NIS (17 mg, 0.077 mmol) and TMSOTf (2.8 µL, 0.015 mmol) were added to the stirring solution at -60 °C and it was allowed to warm up to -40 °C for 2 h. After completion of reaction (monitored by TLC), pyridine was added and 4Å molecular sieves was filtered through a celite bed and the reaction mixture was diluted with CH₂Cl₂ (10 mL) and washed with saturated Na₂S₂O₃ solution. The separated organic layer was dried over anhydrous Na₂SO₄, concentrated and purified by silica gel column chromatography (2:3 Ethyl acetate: n-Hexane) to give protected tetrasaccharide diester **39** (33 mg, 49%) as a colorless sticky solid.

¹H NMR (700 MHz, CDCI₃) *δ* 7.90 (d, *J* = 7.6 Hz, 2H), 7.76 (dd, *J* = 7.7, 4.1 Hz, 2H), 7.57 (d, *J* = 7.4 Hz, 2H), 7.54 - 7.47 (m, 1H), 7.41 - 7.38 (m, 4H), 7.38 - 7.33 (m, 10H), 7.31 - 7.27 (m, 11H), 7.25 - 7.20 (m, 4H), 7.18 - 7.07 (m, 5H), 7.07 - 6.95 (m, 3H), 5.59 (s, 1H, H-1aGaIN3), 5.22 - 5.12 (m, 4H, H-1'oGaIN3), 4.88 (d, *J* = 8.2 Hz, 1H, H-1*β*Vio), 4.82 - 4.72 (m, 2H), 4.72 - 4.55 (m, 8H, H-1*β*QuiN), 4.51 - 4.36 (m, 8H), 4.19 (t, *J* = 6.6 Hz, 1H), 4.01 (s, 1H), 3.88 - 3.69 (m, 5H), 3.64 - 3.52 (m, 4H), 3.50 - 3.31 (m, 2H), 3.31 - 3.22 (m, 1H), 3.17 (dt, *J* = 19.7, 9.8 Hz, 3H), 1.70 - 1.62 (m, 2H), 1.52 - 1.42 (m, 4H), 1.31 (d, *J* = 6.5 Hz, 3H), 0.99 (d, *J* = 6.2 Hz, 3H).

¹³C NMR (176 MHz, CDCI₃) *δ* 171.40, 165.10, 163.75, 161.85, 156.87, 156.34, 155.74, 143.83, 141.47, 140.24, 138.70, 137.91, 137.38, 136.02, 133.19, 129.82, 128.93, 128.87, 128.80, 128.77, 128.68, 128.63, 128.57, 128.55, 128.41, 128.36, 128.24, 128.17, 127.96, 127.89, 127.82, 127.21, 125.03, 125.00, 121.82, 120.17, 100.32 (C-1*β*Vio), 99.72 (C-1*β*QuiN), 99.34 (C- 1*a*GalN3), 98.04 (C-1'aGalN3), 92.36, 83.05, 77.74, 77.34, 77.16, 76.98, 74.28, 74.07, 73.96, 73.29, 72.74, 71.86, 71.79, 71.45, 70.99, 70.90, 70.25, 69.20, 67.37, 67.33, 66.55, 60.58, 59.20, 58.96, 58.34, 50.64, 50.38, 47.40, 47.12, 46.22, 32.07, 31.73, 29.84, 29.05, 27.95, 27.32, 23.23, 23.15, 22.80, 21.21, 18.44, 17.77.

HRMS (ESI): calculated for C₁₀₈H₉₆Cl₃F₁₀N₉O₂₃Na [M+Na]⁺: 2204.5417, found: 2204.5584.

### 5-Amino-pentanyl 4-N-formamido-4,6-dideoxy-β-D-glucopyranosyl-(1→4)-2-acetamido-2-deoxy-α-D-galactopyranosyl uronamide-(1→4)-2-acetamido-α-D-galactopyranosyluronamide-(1→3)-2-acetamido-2,6-dideoxy-β-D-glucopyranoside (2)

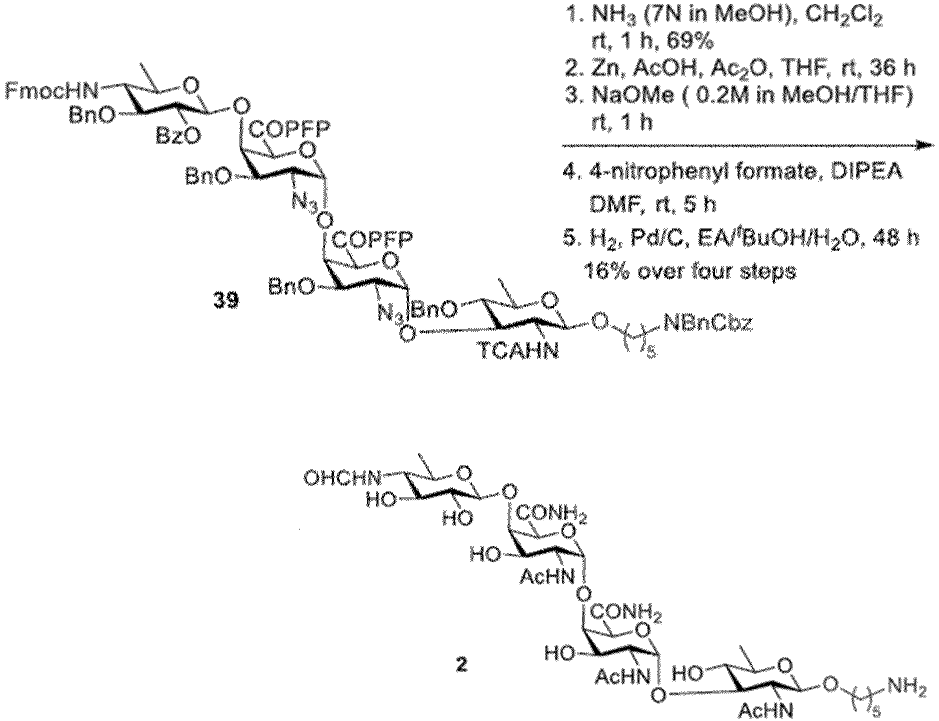

Protected tetrasaccharide diester **39** (40 mg, 0.0183 mmol) was dissolved in CH₂Cl₂ (0.6 mL). After that, ammonia solution (7*N* in methanol, 2.8 mL) was added to the solution at room temperature. After 1 h and complete consumption of the starting material (monitored by TLC), the crude was directly concentrated under reduced pressure and the compound was purified by silica gel column chromatography (3:2 Ethyl acetate: n-Hexane) to get tetrasaccharide diamide compound (24 mg, 69%) as a colorless sticky solid.

¹H NMR (700 MHz, CDCI₃) *δ* 7.78 (d, *J* = 7.7 Hz, 2H), 7.64 (d, *J* = 8.4 Hz, 2H), 7.48 - 7.38 (m, 3H), 7.27 (s, 2H), 7.23 (dt, *J* = 7.4, 4.1 Hz, 6H), 7.18 (q, *J* = 5.8 Hz, 16H), 7.14 (t, *J* = 2.9 Hz, 8H), 7.04 (d, *J* = 8.1 Hz, 1H), 6.97 (dt, *J* = 15.0, 7.3 Hz, 2H), 6.92 (d, *J* = 7.7 Hz, 2H), 6.48 (s, 1H, -NH), 6.25 (s, 1H, -NH), 5.38 - 5.30 (m, 2H, -NH, H-1*α*GaIN3), 5.08 - 5.00 (m, 3H), 4.94 (s, 1H,, H-1'*a*GalN3), 4.64 (dd, *J* = 18.7, 9.4 Hz, 2H, H-1*β*Vio), 4.58 (d, *J =* 11.8 Hz, 2H), 4.53 - 4.43 (m, 6H, H-1*β*QuiN), 4.39 - 4.31 (m, 8H), 4.28 - 4.21 (m, 1H), 4.16 (d, *J* = 28.9 Hz, 1H), 4.08 - 4.00 (m, 2H), 3.65 - 3.58 (m, 3H), 3.54 - 3.47 (m, 2H), 3.42 (d, *J* = 11.1 Hz, 1H), 3.34 - 3.25 (m, 2H), 3.22 (p, *J* = 8.6 Hz, 2H), 3.13 (dq, *J =* 17.1, 9.1 Hz, 2H), 3.04 (t, *J* = 8.1 Hz, 1H), 1.40 - 1.30 (m, 4H), 1.18 (d, *J* = 6.8 Hz, 5H), 1.11 (d, *J* = 6.6 Hz, 3H).

¹³C NMR (176 MHz, CDCI₃) *δ* 170.63, 169.88, 165.43, 162.05, 156.75, 156.23, 155.73, 143.80, 143.74, 141.37, 137.87, 137.56, 137.17, 136.92, 129.74, 128.70, 128.67, 128.63, 128.61, 128.57, 128.56, 128.53, 128.49, 128.47, 128.37, 128.28, 128.21, 128.13, 128.04, 127.99, 127.97, 127.88, 127.83, 127.76, 127.31, 127.22, 127.11, 124.92, 120.04, 100.27 (C-1*β*Vio, 1*J*C-H = 168.0 Hz), 98.74 (C-1*a*GalN3, 1*J*C-H = 175.0 Hz), 98.46 (C-1'*a*GalN3, 1*J*C-H = 175.0 Hz, C-1*β*QuiN, 1*J*C-H = 161.0 Hz), 92.34, 82.82, 77.94, 77.23, 77.05, 76.86, 75.70, 74.45, 73.75, 73.05, 72.03, 71.73, 71.34, 71.09, 70.52, 69.55, 69.38, 67.19, 66.41, 58.92, 58.11, 56.26, 50.49, 50.24, 47.32, 47.12, 46.09, 44.04, 31.96, 29.73, 29.69, 29.39, 29.12, 28.90, 27.86, 27.31, 23.41, 23.17, 22.73, 18.55, 17.77.

HRMS (ESI): calculated for C₉₆H₁₀₁Cl₃N₁₁O₂₁ [M+H]⁺: 1848.6234, found: 1848.6322.

Activated zinc dust (30 mg), acetic acid (0.1 mL) and acetic anhydride (0.25 mL) were added sequentially to the stirring solution of tetrasaccharide diamide (11 mg, 0.006 mmol) in THF (1 mL). The mixture was stirred for 36 h at room temperature. After completion of reaction (monitored by MALDI-ToF analysis), the reaction mixture was diluted with ethyl acetate and zinc was filtered out through celite pad, then the liquor was directly concentrated under reduced pressure. Toluene azeotrope was performed to remove the remaining acetic acid and kept under high *vacuum* for the next step.

MALDI-QToF-MS: calculated for C₁₀₀H₁₁₁N₇O₂₃Na [M+Na]⁺: 1802.0038, found: 1801.3690.

Sodium methoxide (11 mg, 0.20 mmol) was added to the stirring solution of so formed crude tetrasaccharide diamide in methanol/CH₂Cl₂ (2/1, *v*/*v*, 1 mL). After 1 h, Amberlite IR120H+ was added to the solution and the reaction mixture was filtered out, concentrated to obtain 4-amino- 2-OH tetrasaccharide diamide compound as brown sticky liquid. The crude compound was dissolved in anhydrous DMF (1 mL). 4-Nitrophenylformate (5 mg, 0.034 mmol) was added to it followed by dropwise addition of DIPEA (12 µL, 0.07 mmol). After 2 h and consumption of the amine (indicated by TLC), the reaction mixture was directly concentrated to obtain *N*-formamido tetrasaccharide compound as colorless sticky liquid. The obtained *N*-formamido tetrasaccharide compound was dissolved in EA/*t*BuOH/H₂O (2/1/1, *v*/*v*/*v,* 2 mL). To this solution, a suspension of Pd/C (20 mg) was added and stirred under hydrogen atmosphere (1 atm, balloon) for 48 h. The reaction mixture was then filtered through Celite^{®} 353, concentrated, purified by HPLC (Hypercarb Column, 150×10 mm at a flow rate of 3 mL/min, eluted by H₂O (with 0.1% formic acid, Solvent A) and acetonitrile (Solvent B) with a linear gradient from 0% to 30% of solvent B over 30 minutes and lyophilized to obtain tetrasaccharide **2** (0.9 mg, 16% over four steps) as a white solid. Notably a minor amount (3%) of 4-amino tetrasaccharide compound was obtained during final purification which indicated possible incompletion of 4-N-formido formation in previous step.

¹H NMR (700 MHz, D₂O) *δ* 8.18 (s, 1H, -NHCHO), 8.00 (s, -NHCHO), 5.38 (d, *J* = 3.8 Hz, 1H, H-1*a*GalN), 5.00 (d, *J* = 3.8 Hz, 1H, H- 1'*a*GalN), 4.86 (d, *J* = 1.7 Hz, 1H), 4.47 (d, *J* = 7.5 Hz, 1H), 4.42 - 4.40 (m, 2H, H-1*β*Vio, H-1*β* QuiN), 4.27 (d, *J* = 3.0 Hz, 1H), 4.24 (dd, *J =* 11.4, 3.9 Hz, 1H), 4.21 (s, 1H), 4.16-4.14 (m, 1H), 4.05 - 4.00 (m, 1H), 3.88 - 3.84 (m, 2H), 3.69-3.63 (m, 1H), 3.62- 3.58 (m, 3H), 3.54-3.50 (m, 2H), 3.45 - 3.42 (m, 1H), 3.37 (t, *J* = 8.7 Hz, 1H), 3.32 - 3.27 (m, 2H), 3.13-3.08 (m, 2H), 3.01 (t, *J* = 7.0 Hz, 1H), 2.95 (t, *J* = 7.0 Hz, 1H), 2.85 (solvent peak), 2.67 (solvent peak), 2.03 (s, 3H, - NHCOCH3), 2.02 (s, 3H, -NHCOCH3), 1.92 (s, 3H, - NHCOCH3), 1.74 - 1.70 (m, 1H), 1.68 (d, *J* = 7.6 Hz, 1H), 1.65 - 1.62 (m, 3H), 1.57-1.53 (m, 1H), 1.45-1.40 (m, 2H), 1.37-1.36 (m, 1H), 1.27- 1.25 (m, 3H), 1.20 (d, *J* = 5.8 Hz, 3H).

¹³C NMR (176 MHz, D₂O) *δ* 174.60, 174.35, 174.14, 172.97, 170.99, 167.70, 164.69, 101.96 (C-1*β*Vio, 1*J*C-H = 154 Hz), 101.94 (C-1*β*QuiN, 1*J*C-H = 154 Hz), 98.24 (C-1*a*GalN, 1*J*C-H = 175 Hz), 97.73 (C-1'aGalN, 1*J*C-H = 182 Hz), 76.15, 75.16, 73.55, 73.37, 73.31, 71.72, 71.22, 70.74, 70.28, 70.15, 69.99, 69.94, 68.66, 66.85, 66.20, 60.27, 57.61, 55.44, 54.57, 49.23, 48.89, 42.50, 39.22, 32.54, 28.15, 28.13, 28.10, 26.35, 25.04, 23.60, 22.10, 22.08, 22.04, 21.93, 21.87, 16.70, 16.62, 16.40.

HRMS (ESI): calculated for C₃₆H₆₂N₇O₁₉ [M+H]+: 896.4095, found: 896.4089.

### 5-Amino-pentanyl 2-acetamido-β-D-glucopyranoside (40)

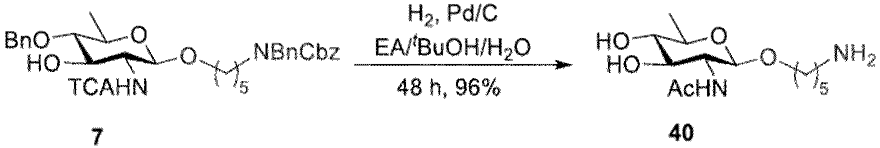

To a solution of compound **7** (12 mg, 0.0169 mmol) in EtOAc/*t*BuOH/H₂O (2/1/1, *v*/*v*/*v,* 4 mL), was added Pd/C (30 mg) at room temperature. The reaction mixture then was stirred under hydrogen atmosphere (1 atm, balloon) for 48 h. After completion of reaction, the crude mixture was then filtered through Celite^{®} 353, concentrated, purified by HPLC (Hypercarb Column, 150×10 mm at a flow rate of 3 mL/min, eluted by H₂O (with 0.1% formic acid, Solvent A) and acetonitrile (Solvent B) with a linear gradient from 0% to 30% of solvent B over 30 minutes and lyophilized to get linker D-quinovosamine **40** (2.51 mg, 96%) as a white powder.

¹H NMR (700 MHz, D₂O) *δ* 4.43 (d, *J* = 8.5 Hz, 1H, H-1), 3.86 - 3.78 (m, 1H, -CH(linker)), 3.65 - 3.59 (m, 1H, H-2), 3.57 - 3.49 (m, 1H, -CH(linker)), 3.47 - 3.38 (m, 2H, H-3, H-5), 3.19 - 3.08 (m, 1H, H-4), 2.93 (t, *J* = 6.5 Hz, 2H,-CH2(linker)), 1.98 (s, 3H, -COCH3), 1.65 - 1.50 (m, 4H, -C*H*2(linker)), 1.34 (dt, *J =* 8.1, 4.2 Hz, 2H, -C*H*2(linker)), 1.26 (d, *J* = 3.5 Hz, 3H, H-6).

¹³C NMR (176 MHz, D₂O) *δ* 174.37, 100.97 (C-1), 75.05, 73.43, 71.81, 70.00, 55.75, 39.22, 28.03, 26.30, 22.04, 16.56.

HRMS (ESI): calculated for C₁₃H₂₇N₂O₅ [M+H]+: 291.1915, found: 291.1941.

### 5-Amino-pentanyl 2-acetamido-α-D-galactopyranoside (41)

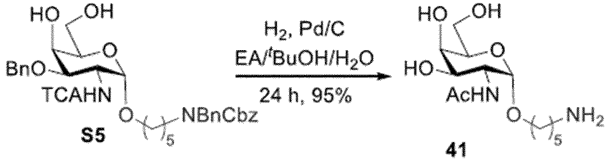

To a stirred solution of diol compound **S5** (80 mg, 0.111 mmol) in EA/*t*BuOH/H₂O/ (2/1/1, *v*/*v*/*v,* 6 mL), was added Pd/C (130 mg) at room temperature. The reaction mixture was then allowed to stir under hydrogen atmosphere (1 atm, balloon) for 24 h. After completion of the reaction, the crude mixture was then filtered through Celite^{®} 353, concentrated, purified by HPLC (Hypercarb Column, 150×10 mm at a flow rate of 3 mL/min, eluted by H₂O (with 0.1% formic acid, Solvent A) and acetonitrile (Solvent B) with a linear gradient from 0% to 30% of solvent B over 30 minutes and lyophilized to afford D-galactosamine derivative **41** (32 mg, 95%) as colorless sticky liquid.

¹H NMR (600 MHz, D₂O) *δ* 4.79 (d, *J =* 3.8 Hz, 1H, H-1), 4.03 (dd, *J =* 11.1, 3.8 Hz, 1H, H-2), 3.87 - 3.78 (m, 3H, H-3, H-4, H-5), 3.68 - 3.57 (m, 3H, H-6a, H-6b, - CH(linker)), 3.38 (dt, *J =* 10.1, 6.3 Hz, 1H, -CH(linker)), 2.88 (dd, *J* = 8.4, 7.0 Hz, 2H, -CH2(linker)), 1.93 (s, 3H, - NHCOC*H*₃), 1.61 - 1.48 (m, 4H, -C*H*₂(linker)), 1.37 - 1.28 (m, 2H, -C*H*₂(linker)).

¹³C NMR (151 MHz, D₂O) *δ* 174.46, 96.79 (C-1), 70.84, 68.46, 67.62, 67.59, 61.24, 49.96, 39.28, 27.89, 26.39, 22.23, 21.84.

HRMS (ESI): calculated for C₁₃H₂₇N₂O₆ [M+H]+: 307.1864, found: 307.1884.

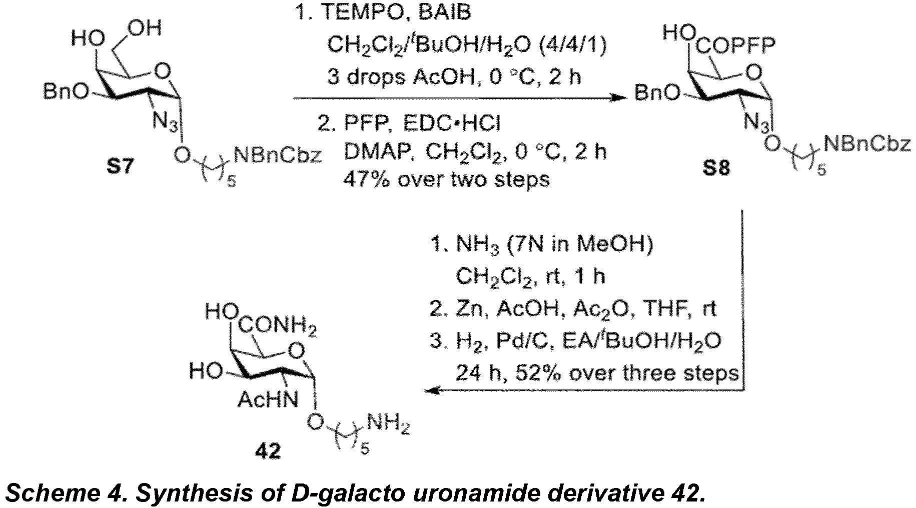

### N-(Benzyl)benzyloxycarbonyl-5-amino-pentanyl 2-trichloroacetamido-4-O-benzyl-6-Opentafluorophenyl-α-D-galactopyranoside uronate (S8)

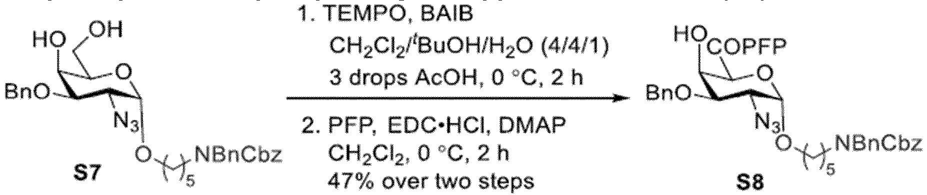

TEMPO (11 mg, 0.068 mmol), BAIB (275 mg, 0.85 mmol) and three drops of acetic acid were added sequentially to a stirring solution of known D-galactosamine diol **S7**3 (207 mg, 0.34 mmol) in CH₂Cl₂/*t*BuOH/H₂O (4/4/1, *v*/*v*/*v,* 2.25 mL) at 0 °C. The reaction mixture was allowed to stir at the same temperature for 2 h. After complete conversion of the alcohol to acid (monitored by TLC), the reaction mixture was quenched with addition of saturated aqueous Na₂S₂O₃ (1 mL) and aqueous NaH₂PO₄ (0.5 mL). The organic layer was diluted with ethyl acetate (20 mL) and washed with brine solution. The aqueous layer was again extracted with 10 mL ethyl acetate three times. The separated organic layer was dried over anhydrous Na₂SO₄, concentrated and dried under high *vacuum* to get crude carboxylate compound as brownish liquid. To *vacuum* dried crude carboxylate compound in anhydrous CH₂Cl₂ (25 mL), pentafluoro phenol (100 mg, 0.547 mmol) and catalytic amounts of DMAP (21 mg, 0.17 mmol) were added. After that, EDC•HCl (229 mg, 1.19 mmol) was added to the reaction mixture. The mixture was stirred at same temperature for 2 h. After completion of the reaction (monitored by TLC), the crude reaction mixture was diluted with CH₂Cl₂ (30 mL) and washed with brine solution two times. The separated organic layer was dried over anhydrous Na₂SO₄, concentrated and purified by silica gel column chromatography (1:4 Ethyl acetate: n-Hexane) to obtain 6-O ester compound **S8** (126 mg, 47% over two steps) as white foam.

¹H NMR (400 MHz, CDCI₃) *δ* 7.33 - 7.27 (m, 6H, -ArH), 7.25 - 7.17 (m, 8H, -ArH), 7.09 (d, *J* = 7.3 Hz, 1H, -ArH), 5.09 (d, *J =* 16.0 Hz, 2H, -CH2Cbz), 4.95 (dd, *J* = 12.7, 3.6 Hz, 1H, H-1o), 4.71 - 4.60 (m, 3H, H-5, -CH2Ph), 4.49 - 4.38 (m, 3H, H-4, -C*H*₂Ph), 3.99 - 3.89 (m, 1H, H-3), 3.64 (td, *J* = 11.6, 4.7 Hz, 2H, H-2, -CH(linker)), 3.41 (dt, *J* = 28.7, 8.0 Hz, 1H, -CH(linker)), 3.15 (dt, *J* = 24.1, 7.3 Hz, 2H, - C*H*₂(linker)), 2.54 (s, 1H, -OH), 1.62 - 1.37 (m, 6H, -C*H*₂(linker)).

¹³C NMR (101 MHz, CDCI₃) *δ* 164.50, 156.28, 137.86, 136.75, 128.83, 128.59, 128.54, 128.10, 127.99, 127.86, 127.35, 127.21, 98.39 (C-1*α*), 77.40, 77.28, 77.08, 76.76, 75.10, 72.47, 69.94, 69.31, 67.33, 67.23, 58.36, 50.53, 50.26, 47.04, 46.06, 29.74, 28.99, 27.85, 27.42, 23.27.

HRMS (ESI): calculated for C₂₄H₂₅F₅N₄O₆Na [M+Na]⁺: 807.2424, found: 807.2418.

### 5-Amino-pentanyl 2-acetamido-o-D-galactopyranoside uronamide (42)

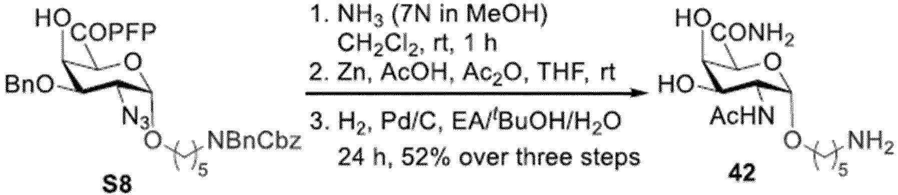

Ammonia solution (7N in methanol, 20 mL) was added dropwise to the stirred solution of compound **S8** (100 mg, 0.127 mmol) in CH₂Cl₂ (4 mL). After 1 h and complete consumption of the starting material (indicated by TLC), the crude was directly concentrated under reduced pressure to get 6-amido compound as viscous liquid. Activated zinc dust (100 mg), acetic anhydride (0.5 mL) and acetic acid (0.1 mL) were added sequentially to the stirring solution of 6-amido compound (78.4 mg, 0.127 mmol) in THF (3 mL). The mixture was stirred overnight at room temperature. After completion of reaction (indicated by TLC), the reaction mixture was diluted with ethyl acetate and zinc was filtered out through celite pad, then the liquor was directly concentrated under reduced pressure. Toluene azeotrope was performed to remove the remaining acetic acid and kept under high *vacuum* for the next step. The obtained amide compound (80 mg, 0.127 mmol) was dissolved in EA/*t*BuOH/H₂O (2/1/1, *v*/*v*/*v,* 7.5 mL1.5 mL). To this solution, Pd/C (150 mg) was added and stirred under hydrogen atmosphere (1 atm, balloon) for 24 h. The reaction mixture was then filtered through Celite^{®} 353, concentrated, purified by HPLC (Hypercarb Column, 150×10 mm at a flow rate of 3 mL/min, eluted by H₂O (with 0.1% formic acid, Solvent A) and acetonitrile (Solvent B) with a linear gradient from 0% to 30% of solvent B over 30 minutes and lyophilized to obtain D-galactose uronamide **42** (21 mg, 52% over three steps) as sticky colorless liquid.

¹H NMR (600 MHz, D₂O) *δ* 5.01 (d, *J* = 3.8 Hz, 1H, H-1*α*), 4.40 (d, *J =* 1.7 Hz, 1H, H-5), 4.29 (d, *J =* 1.6 Hz, 1H, H-4), 4.17 (dd, *J =* 11.1, 3.8 Hz, 1H, H-2), 3.98 (dd, *J =* 11.1, 3.2 Hz, 1H, H-3), 3.71 (dt, *J* = 10.2, 6.6 Hz, 1H, -CH(linker)), 3.53 (dt, *J* = 10.2, 6.4 Hz, 1H, -CH(linker)), 3.01 - 2.94 (m, 2H, -C*H*2(linker)), 2.04 (s, 3H, -NHCOC*H*₃), 1.72 - 1.59 (m, 4H, -C*H*₂(linker)), 1.48 - 1.35 (m, 2H, -C*H*₂(linker)).

¹³C NMR (151 MHz, D₂O) *δ* 174.05, 97.05 (C-1), 70.67, 68.77, 68.22, 67.34, 49.61, 39.32, 27.97, 26.45, 22.25, 21.88.

HRMS (ESI): calculated for C₁₃H₂₆N₃O₆ [M+H]⁺: 320.1816, found: 320.1835.

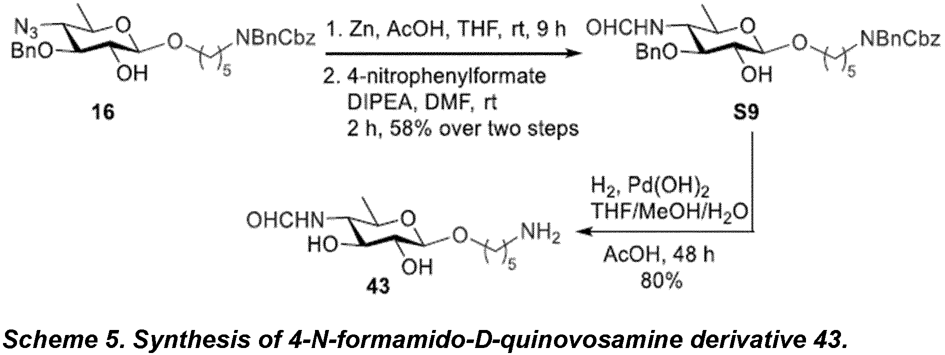

### N-(Benzyl)benzyloxycarbonyl-5-amino-pentanyl 4-N-formamido-3-O-benzyl-4,6-dideoxy-β-D-glucopyranoside (S9)

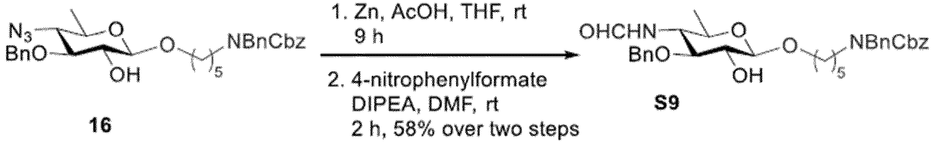

Activated zinc dust (106 mg) and acetic acid (0.1 mL) were added sequentially to the stirring solution of compound **16** (80 mg, 0.136 mmol) in THF (1 mL) at room temperature. The reaction was stirred at same temperature for 9 h. After completion of reaction (indicated by TLC), Zn dust was removed by filtering through a celite bed and the reaction mixture was washed with EtOAc. Solvents were removed under reduced pressure and dried under high *vacuum* for 0.5 h. The crude amine residue was dissolved in anhydrous DMF (2.3 mL). 4-Nitrophenylformate (114 mg, 0.68 mmol) was added to it followed by dropwise addition of DIPEA (0.24 mL, 1.36 mmol). After 2 h and complete consumption of the amine (indicated by TLC), the reaction mixture was diluted with ethyl acetate (30 mL) and washed with brine solution. The separated organic layer was dried over anhydrous Na₂SO₄, concentrated and purified by silica gel column chromatography (5:95 Methanol: Dichloromethane) to obtain *N*-formamido compound **S9** (46 mg, 58% over two steps) as a sticky white solid.

¹H NMR (600 MHz, CDCI₃) *δ* 8.10 - 7.97 (m, 1H), 7.38 - 7.20 (m, 14H), 7.16 (d, *J* = 7.7 Hz, 1H), 5.17 (d, *J* =24.0 Hz, 2H) 4.89 (q, *J* = 10.0 Hz, 1H), 4.66 (dd, *J =* 11.3, 3.5 Hz, 1H), 4.53 - 4.40 (m, 2H), 4.20-4.14 (m, 1H, H-1), 3.89 - 3.77 (m, 1H), 3.57 (dq, *J* = 16.8, 7.8 Hz, 2H), 3.51 - 3.37 (m, 2H), 3.32 - 3.16 (m, 3H), 3.10 - 2.94 (m, 1H), 1.64 - 1.46 (m, 4H), 1.37 - 1.28 (m, 2H), 1.23 (d, *J* = 5.9 Hz, 3H).

¹³C NMR (151 MHz, CDCI₃) *δ* 165.61, 163.27, 161.57, 156.93, 156.46, 138.49, 128.66, 128.60, 128.55, 128.45, 128.36, 128.06, 127.89, 127.29, 126.20, 115.76, 102.83-102.74 (C-1), 81.35, 79.95, 77.37, 77.16, 76.95, 75.25, 75.15, 74.84, 73.36, 70.71, 70.47, 69.96, 69.74, 67.36, 59.59, 55.37, 50.56, 50.33, 47.12, 46.20, 32.01, 30.40, 29.78, 29.29, 29.17, 27.90, 27.37, 23.40, 23.25, 22.78, 18.12, 18.08.

HRMS (ESI): calculated for C₃₄H₄₂N₂O₇Na [M+Na]⁺: 613.2884, found: 613.2903.

### 5-Amino-pentanyl 4-N-formamido-4,6-dideoxy-β-D-glucopyranoside (43)

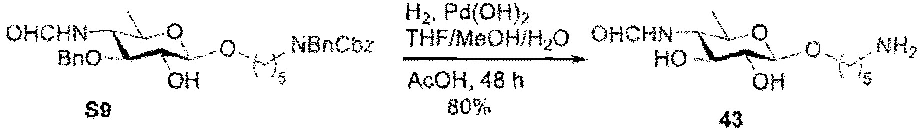

The formamido compound **S9** (45 mg, 0.076 mmol) was dissolved in THF/MeOH/H₂O (1/2/2, *v*/*v*/*v,* 5 mL) and two drops of AcOH was added to it. To this solution, a suspension of Pd(OH)₂ (90 mg) was added and stirred under hydrogen atmosphere (1 atm, balloon) for 48 h. The reaction mixture was then filtered through Celite^{®} 353, concentrated, purified by HPLC (Hypercarb Column, 150×10 mm at a flow rate of 3 mL/min, eluted by H₂O (with 0.1% formic acid, Solvent A) and acetonitrile (Solvent B) with a linear gradient from 0% to 30% of solvent B over 30 minutes and lyophilized to obtain linker coupled *N*-formamido monosaccharide **43** (17 mg, 80%) as a colorless sticky solid.

¹H NMR (600 MHz, D₂O) *δ* 7.97 (s, 1H, -NHCHO), 4.40 (d, *J* = 8.1 Hz, 1H, H-1), 3.86 (dt, *J =* 10.0, 6.6 Hz, 1H, -CH(linker)), 3.65 - 3.54 (m, 3H, H-4, H-5, -CH(linker)), 3.46 (m, *J* = 31.1, 9.5 Hz, 1H, H-3), 3.26 (td, *J* = 9.4, 8.0 Hz, 1H, H-2), 2.96 (t, *J* = 7.6 Hz, 2H, -C*H*₂(linker)), 1.67 - 1.58 (m, 4H, -C*H*₂(linker)), 1.44 - 1.36 (m, 2H, -C*H*₂(linker)), 1.24 - 1.12 (m, 3H, H-6).

¹³C NMR (151 MHz, D₂O) *δ* 167.68, 164.67, 101.94 (C-1), 73.52, 73.28, 70.72, 70.26, 70.02, 55.42, 39.25, 28.13, 26.35, 22.03, 16.67.

HRMS (ESI): calculated for C₁₂H₂₅N₂O₅ [M+H]+: 277.1577, found: 277.1783.

### Example B: Glycan microarray binding studies

### Material and Methods

The sera were investigated and collected at the National Reference Laboratory for Tularemia for diagnostic and screening purposes.

The glycans were dissolved at 0.1 mM in 50 mM sodium phosphate buffer pH 8.5 and printed in 64 identical fields to NHS activated hydrogel glass slides (TRIDIA^{™} Activated Slides, Surmodics) using a non-contact sciFLEXARRAYER S3 microarray spotter (Scienion, Berlin, Germany). After incubation overnight in a humidified box, the remaining NHS groups of the slides were quenched with ethanolamine. The slides were blocked with 1% (w/v) bovine serum albumin (BSA) in phosphate buffered saline (PBS) and a 64 well incubation gasket (FlexWell Grid, Grace Bio Labs) was attached. The slides were incubated with pig, horse, dog, rabbit, cat or cheetah serum diluted 1:100 in 1% BSA-PBS for 1 h at 37° C. After three washes with PBS containing 0.1% (v/v) Tween-20 (PBS-T) the slides were incubated with goat anti-pig IgG (H+L) FITC conjugate (Invitrogen, Cat. PA1-84850), rabbit anti-horse IgG (whole molecule) - Fluorescein isothiocyanate (Sigma-Aldrich, Cat. F7759), rabbit anti-dog IgG (whole molecule) - Fluorescein isothiocyanate (Sigma-Aldrich, Cat. F4012), goat anti-rabbit IgG (H+L) Alexa Fluor^{®} 647 AffiniPure^{™} (Jackson ImmunoResearch, Cat. 111-605-003) or goat anti-cat IgG (whole molecule) - Fluorescein isothiocyanate (Sigma-Aldrich, Cat. F4262) diluted 1:400 for 1 h at 37°C. The slides were washed twice with PBS-T. After removing the gasket, the slides were washed once with PBS and once with water. The dried slides were scanned with an InnoScan 1100 Fluorescence Scanner (Innopsys). Intensities were evaluated with Mapix (Innopsys). The statistical analysis was performed with the software GraphPad Prism 9.3.1 (GraphPad Software, Inc.).

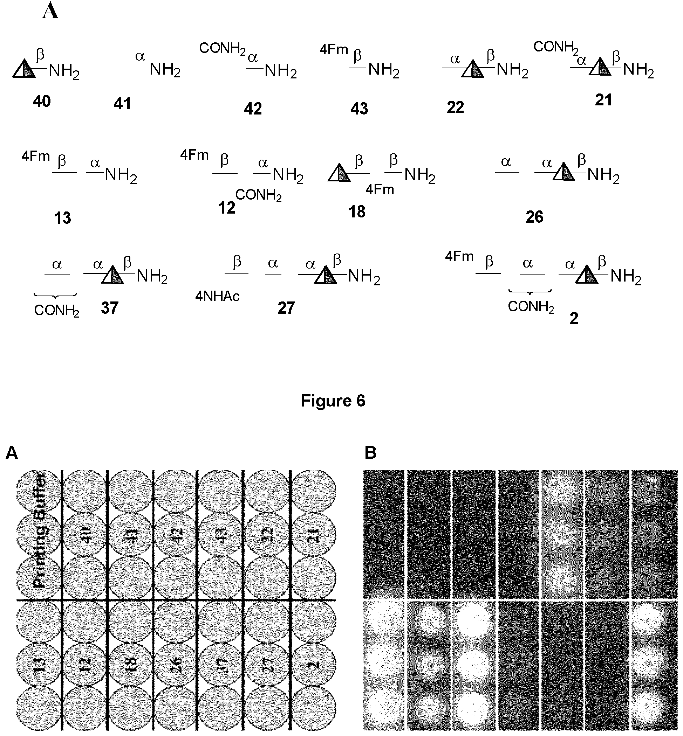

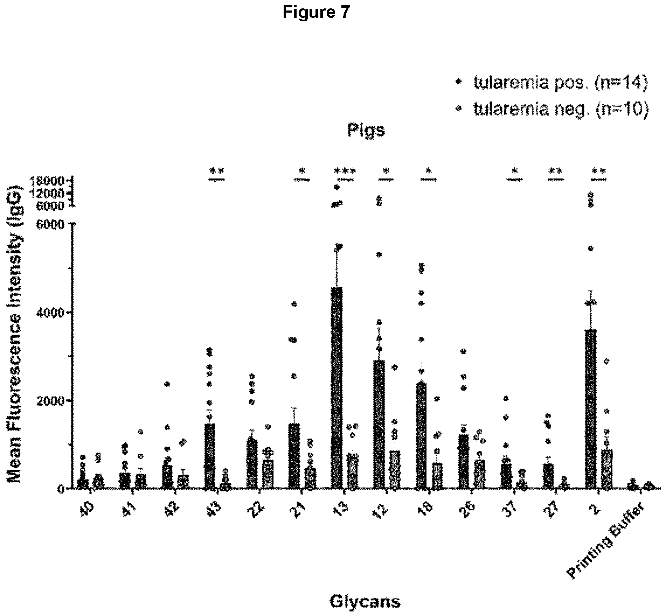

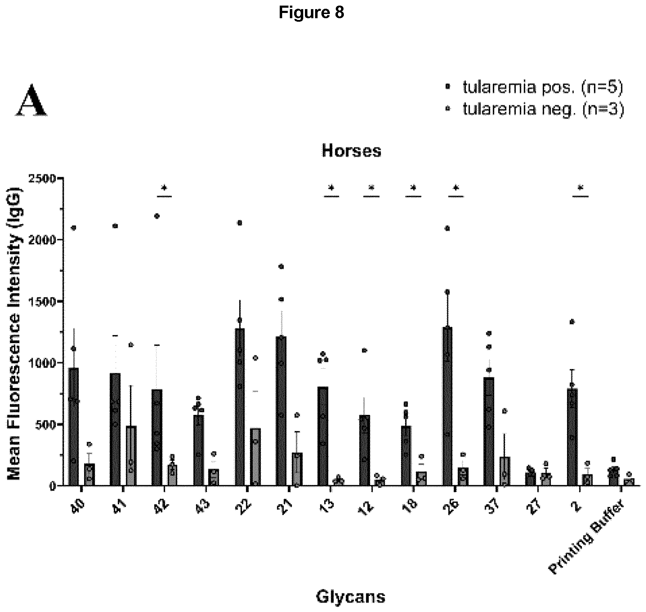

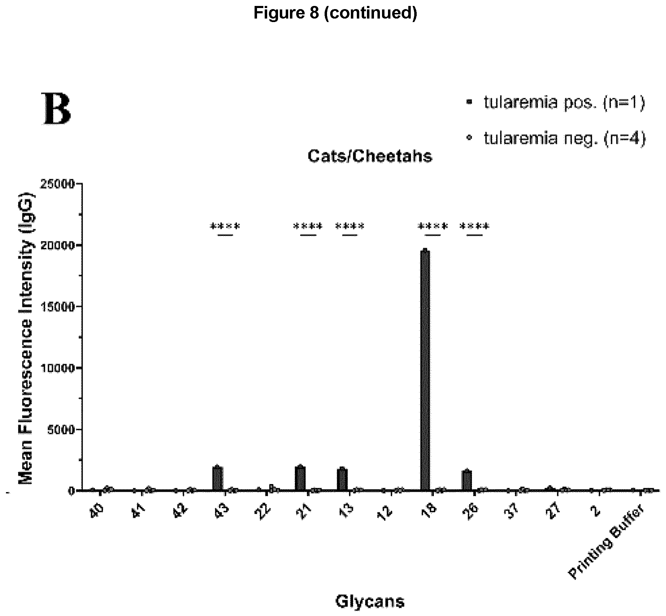

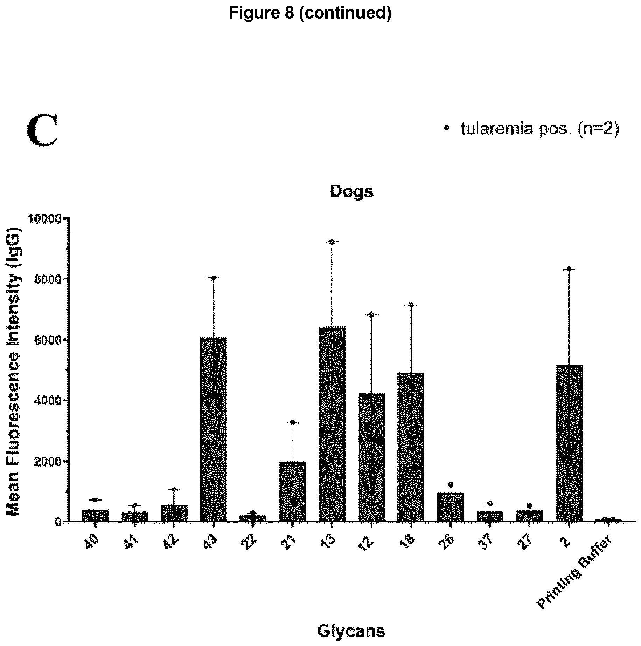

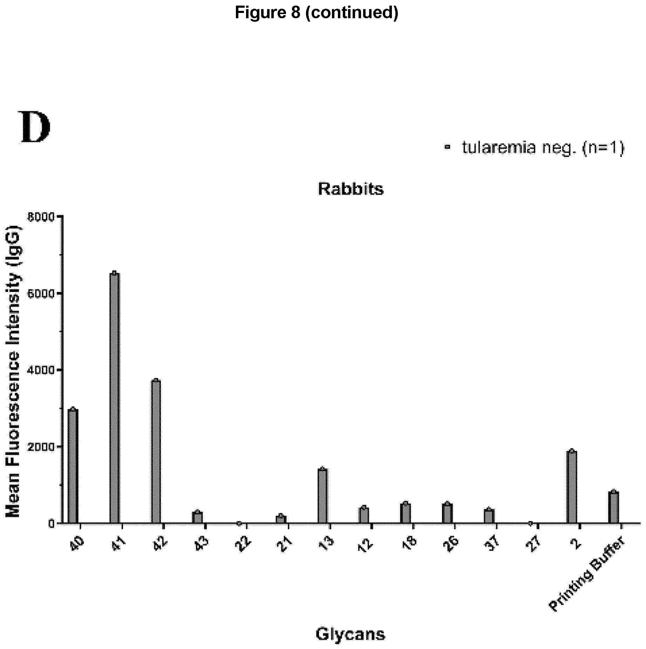

The synthetic glycans **2, 12, 13, 18, 21, 22, 26, 27, 37,** and **40-43** were immobilized in triplicates on glass slides to detect antibodies in serum samples derived from tularemia positive and negative animals (Figures 7 and Figures 8A-6D). The sera of tularemia positive pigs showed significantly higher IgG antibody binding to the monosaccharide **43,** the disaccharides **12, 13, 18, 21** and trisaccharide **37** as well as tetrasaccharides 2 and **27** compared to tularemia negative pigs (Figure 7). Significant higher IgG antibody binding to monosaccharide **42,** disaccharides **12, 13** and **18,** trisaccharide **26** and tetrasaccharide **2** were observed in sera of tularemia positive horses. Monosaccharide **43,** disaccharides **21, 13** and **18** as well as trisaccharide **26** were recognized by antibodies in sera of tularemia positive cats and cheetahs. Tularemia positive dogs showed IgG antibody binding to the monosaccharide **43,** the disaccharides **13, 12, 18** and tetrasaccharide **2,** whereas the sera of tularemia negative rabbits showed IgG antibody binding of monosaccharides **40, 41** and **42** (Figure 8). These experiments identified disaccharides **13** and **18** as leads for the development of diagnostic tools and therapeutics.

### Example C: Synthesis and characterization of conjugates

### General procedure synthesis

For further immunization studies, glycan **13** and **18** were conjugated to CRM₁₉₇ to generate semi-synthetic glycoconjugate vaccine candidates.

The saccharide **(13:** 0.657 mg; **18:** 0.635 mg) was dissolved in 200 µL DMSO, 25 µL pyridine and 10 µL triethylamine were added. 6.5 equivalents of (4-nitrophenyl)-adipate linker in DMSO were added to the saccharide solution. After stirring for 5 hours, the reaction mixture was frozen in liquid nitrogen and then lyophilized to obtain a crude white solid. The residue was washed with chloroform (5x 100 µL) and DCM (3x 1 µL) to remove the excess of bis(4-nitrophenyl) adipate. TLC was used to check that all excess linker was washed off and that no glycan had dissolved. 350 µL of water was added to a 10k Amicon filter and centrifuged at 10000 rpm for 8 minutes to wet the filter. Then CRM₁₉₇was transferred to the Amicon 10k filter (180 µL, 1 mg) and the tube was washed with 300 µL of water and also added to the Amicon filter. After centrifugation at 10000 rpm for 8 minutes, the CRM₁₉₇ vial was washed with 350 µL water and transferred to the filter. After centrifugation, the CRM₁₉₇ vial was washed with 350 µL of 0.1 M phosphate buffer pH 8. The filter was removed after centrifugation, inverted into a clean vial and centrifuged at 1000 rpm for 2 min, yielding a colorless filtrate containing CRM₁₉₇. The filtrate (~70 µL) was transferred to the vial containing the sugar-PNP ester, the tube was washed with phosphate buffer pH 8 (2 x 30 µL) and added to the reaction solution. The reaction was stirred slowly for 24 hours. The solution was transferred to an Amicon 10K filter and the reaction vial was washed with 300 µL of the reaction buffer. The solution was washed three times with 400 µL of water. The solution was then washed with 400 µL of PBS. Finally, the filter was placed upside down in a clean vial and centrifuged at 1,000 rpm for 2 minutes. The filtrate was diluted with 350 µL PBS and stored at -20°C.

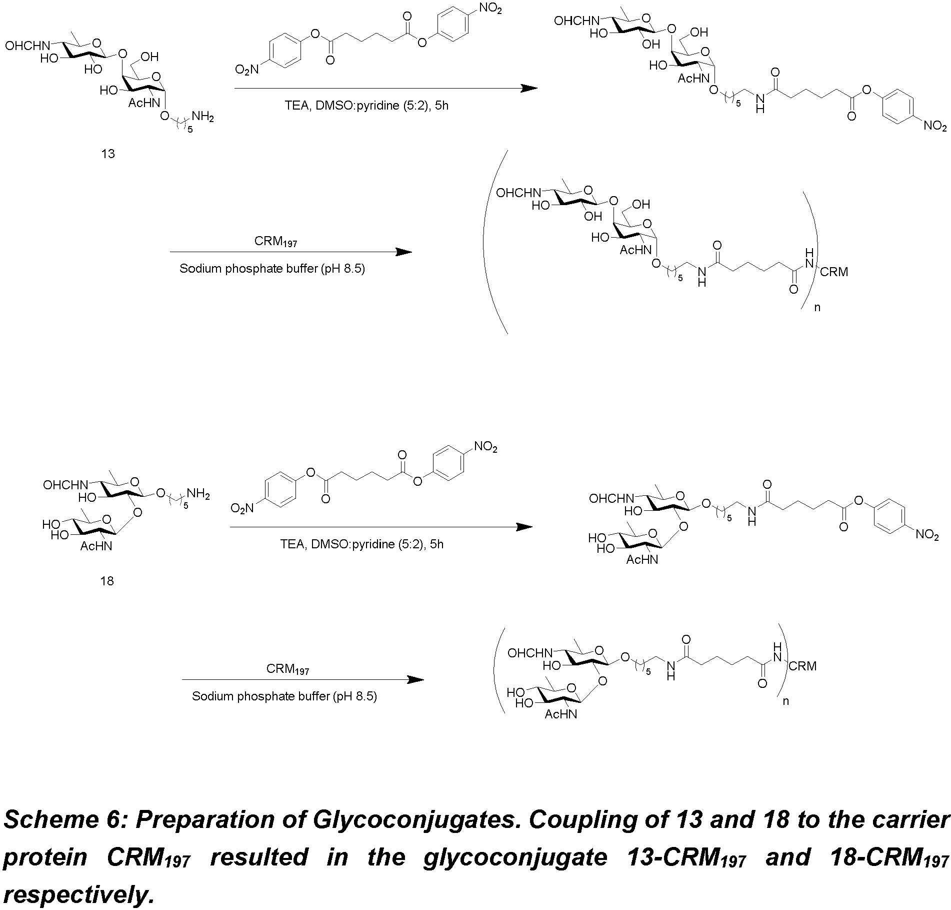

### Characterization of glycoconjugates13-CRM₁₉₇ and 18-CRM₁₉₇

Mass spectra were recorded using an Autoflex Speed MALDI-TOF system (Bruker Daltonics; Bremen, Germany). Samples were spotted onto MTP 384 ground steel target plates (Bruker Daltonics) using the dried droplet technique with 2,5-dihydroxyacetophenone (DHAP) as matrix. The mass spectrometer was operated in linear positive mode. Mass spectra were acquired over an m/z range of 30,000 to 210,000 and the data analyzed using the FlexAnalysis software supplied with the instrument.

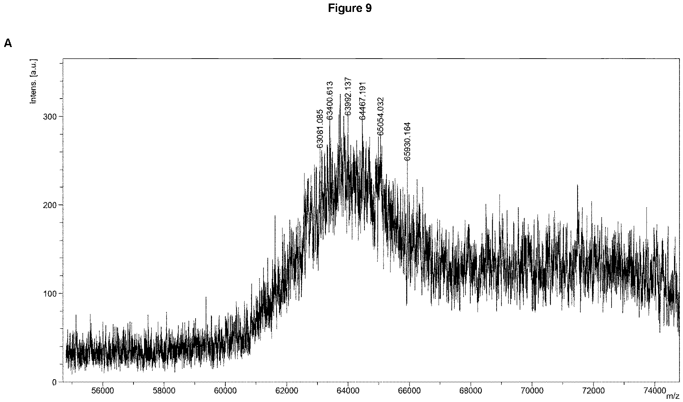

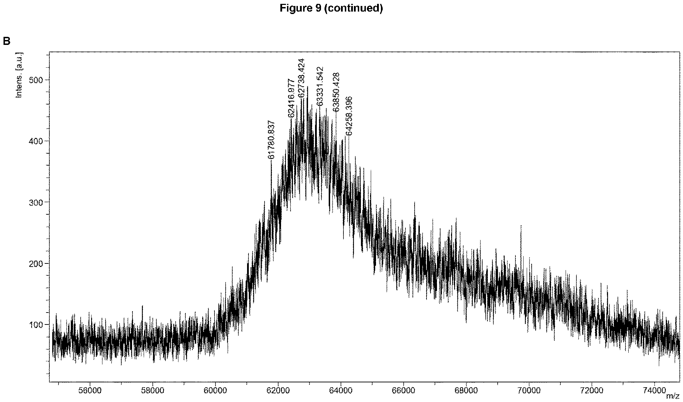

Conjugation was checked by MALDI-TOF-MS (Figure 9). The results showed that glycan **13** and **18** were successfully conjugated to CRM₁₉₇. The average loading was 7 units of glycan **13** and 5 units of glycan 18 per protein monomer.

### Example D: Mice immunization and generation of polyclonal sera

In order to test the ability of 13 and 18 to induce a natural immune response, mice were immunized subcutaneously on day 0 with 13-CRM197 or 18-CRM197. They were boosted on days 14 and 28. Blood samples were collected every two weeks and a final bleeding and spleen extraction took place on day 31. The sera were stored at -20°C for further testing on glycan arrays or bacterial ELISA.

Immunization with the glycoconjugate vaccines lead to the development of glycan specific antibodies in sera of mice.

The immunizations were conducted by BioGenes GmbH. 8 weeks old C57BL/6NRj mice (Janvier, Le Genest-Saint_lsle, France) were housed in groups of five animals in individually ventilated cages (Tecniplast, Hohenpeißenberg, Germany) under specific pathogen-free conditions. Housing and experiments were in accordance with institutional guidelines and with the regulations of the Federation of European Laboratory Animal Science Associations (FELASA).

Mice were immunized subcutaneously (s.c.) with 1µg of glycoconjugate per injection. The glycoconjugates, together with Alum adjuvant, were diluted in sterile PBS to a final volume of 100 µL per dose. The animals were immunized on day 0 (primary immunization) and boosted on days 14 and 28. Blood (max. 80µL) samples were collected every two weeks. After coagulation at room temperature, serum was separated by centrifugation at 2,000 g for 10 min. The sera and saliva were frozen at -20°C until further usage.

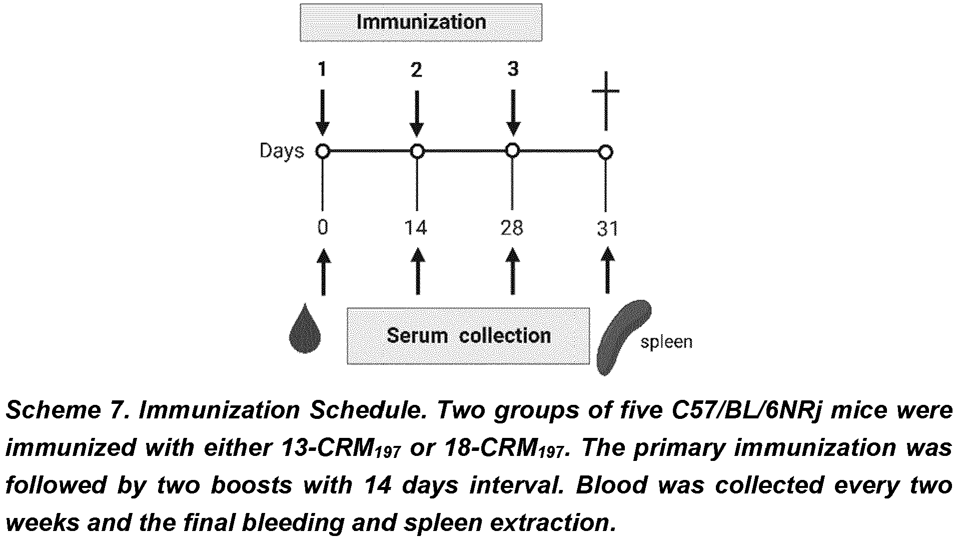

### Hybridoma Generation

The spleens were taken out of the mice and mashed with 5 mL serum-free RPMI w/o additions. The supernatant was collected in a 50 mL Falcon and filled up with serum-free RPMI w/o additions. The tube was centrifuged at 300 g for 10 min at RT and the pellet was washed with 50 mL serum-free RPMI w/o additions and resuspended in 15 mL serum-free RPMI w/o additions. X63-Ag8 myeloma cells were washed two times in 50 mL serum-free RPMI w/o additions and then transferred to the spleen cells. After centrifugation the pellet was gently disrupted by tapping the bottom of the tube and placed in a 37°C water bath and kept in there during the fusion. 1.5 mL pre-warmed 50% PEG 1500 were gradually added to the pellet over a period of 1 min, while continually stirring the cells gently with the pipette tip. The cells
were stirred for 1 min and while gently swirling the tube, pre-warmed serum-free RPMI w/o additions medium was slowly added: 1 ml over 60 s, 3 ml over 60 s and 16 ml over 120 s. The cells were immediately centrifuged at 300 g for 10 min in an uncooled centrifuge and incubated for 5 min at RT. The supernatant was removed and the cells gently resuspended in 200 mL selection medium (RPMI 500mL, 2 mM L-Glutamine, 0.05 mg/mL Gentamycin, 1% Penicillin/Streptomycin, 10% FCS, 1% Non-Essential Amino Acids, 1xHAT (Hypoxanthin/Aminopterin/Thymidin), 1x BM-Condimed H1 supplement). 200 µL/well of cells were plated onto 96-well flat-bottom plates and incubated at 37°C and 5% CO2

Binding to synthetic glycans was checked via Glycan microarray analysis after 10 days. Subclonings were performed until the clones were monoclonal. The cells were expanded and slowly adapted to ISF-1 w/o FCS. The supernatant containing the antibody was harvested and sterile filtered 10 days after the last splitting, when the cells were about 100% dead. The antibodies were purified using a protein A/G column.

### Glycan Microarray Experiments (sera and hybridoma)

The glycans were dissolved at 0.1 mM in 50 mM sodium phosphate buffer pH 8.5 and printed in 64 identical fields to NHS activated hydrogel glass slides (TRIDIA^{™} Activated Slides, Surmodics) using a non-contact sciFLEXARRAYER S3 microarray spotter (Scienion, Berlin, Germany). After incubation overnight in a humidified box, the remaining NHS groups of the slides were quenched with ethanolamine. The slides were blocked with 1% (w/v) bovine serum albumin (BSA) in phosphate buffered saline (PBS) and a 64 well incubation gasket (FlexWell Grid, Grace Bio Labs) was attached. The slides were incubated with serum of the immunized mice diluted 1:100 and 10 ng/mL, 100ng/mL, 1000 ng/mL and 10000 ng/mL of monoclonal antibody in 1% BSA-PBS for 1 h at 37° C. After three washes with PBS containing 0.1% (v/v) Tween-20 (PBS-T) the slides were incubated with Alexa Fluor^{®} 488 AffiniPure Goat Anti-Mouse IgG, Fcγ fragment specific, Jackson ImmunoResearch diluted 1:400 for 1 h at 37°C. The slides were washed twice with PBS-T. After removing the gasket, the slides were washed once with PBS and once with water. The dried slides were scanned with an InnoScan 1100 Fluorescence Scanner (Innopsys). Intensities were evaluated with Mapix (Innopsys). The statistical analysis was performed with the software GraphPad Prism 9.3.1 (GraphPad Software, Inc.).

### ELISA

Bacteria were cultured overnight. Bacteria were grown to an OD of 0.7 and centrifuged at 3000 g during 5 min. Supernatant was discarded. Bacteria were washed three times with PBS and were afterwards diluted in 10 ml of carbonate bicarbonate coating buffer. High-binding flat bottom ELISA plates (Corning) were coated with 100 ul of diluted bacteria and were incubated at 4 °C overnight. ELISA plates were washed 3 times with PBST. 50 ul of 100µg/mL of monoclonal antibodies in triplicates were pipetted into corresponding wells and incubated at RT for 2 hours. Plates were washed 3 times with PBST.

Per well 50 ul of goat- anti mouse IgG HRP antibody (Dianova) was added at a dilution of 1:10000 and incubated for 1 hour. Plates were washed 3 times with PBST. 100 ul of substrate TMB was added to each well. After 10 min the reaction was stopped by adding H2SO4. The absorbance was read at 450 nm in a CLARIOstar Plus plate reader (BMG Labtech).

## Claims

1. A saccharide of general formula (I)
H-Uₓ₊₁-Uₓ-T-O-L-NH₂ (I)
wherein
**x** is an integer selected from 1, 2, 3, and 4; 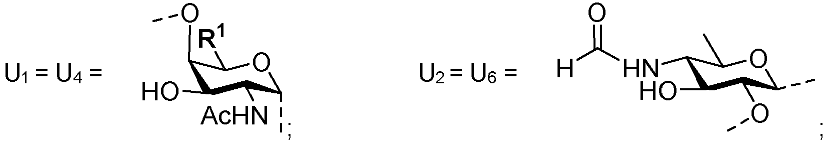 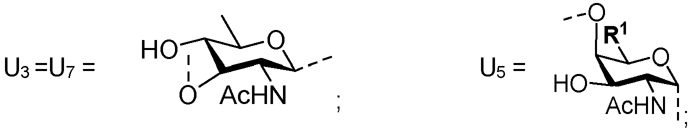
-T- represents a bond, -Uₓ₊₃-, -Uₓ₊₃-Uₓ₊₂-, -Uₓ₊₃-Uₓ₊₂-Uₓ₊₁-, or
-[Uₓ₊₃-Uₓ₊₂-Uₓ₊₁-Uₓ]ₙ- with n being an integer selected from 1, 2 and 3;
**R¹** represents -CH₂OH or -CONH₂;
**L** represents a linker;
or a pharmaceutically acceptable salt thereof.

2. The saccharide according to claim 1, wherein **x** represents 1 or 2, or a pharmaceutically acceptable salt thereof.

3. The saccharide according to claim 1 or 2, wherein **-T-** represents a bond, -Uₓ₊₃- or -Uₓ₊₃-Uₓ₊₂-, or a pharmaceutically acceptable salt thereof.

4. The saccharide according to any one of the claims 1 to 3, wherein
**-L-** represents **-L^{a}-, -L^{a}-L^{e}-, -L^{a}-L^{b}-L^{e}-,** or **-L^{a}-L^{d}-L^{e}- ;**
**-L^{a}-** represents -(CH₂)ₒ-, -(CH₂-CH₂-O)ₒ-C₂H₄-, or -(CH₂-CH₂-O)ₒ-CH₂;
-**L^{b}**- represents -O-, -NH-CO-NH-, -NH-CO-CH₂-NH-, -NH-CO-;
**-L^{d}-** represents -(CH₂)_{q}-, -(CH(OH))_{q}-, -(CF₂)_{q}-, -(CH₂-CH₂-O)_{q}-C₂H₄-, or -(CH₂-CH₂-O)_{q}-CH₂- ;
**-L^{e}-** represents -(CH₂)ₚ₁-, -(CF₂)ₚ₁-, -C₂H₄-(O-CH₂-CH₂)ₚ₁-, -CH₂-(O-CH₂-CH₂)ₚ₁- or -(CH₂)ₚ₁-O-(CH₂)ₚ₂-; and
**o, q, p1** and **p2** are independently of each other an integer selected from 1, 2, 3, 4, 5, and 6.

5. The saccharide according to any one of the claims 1 to 3, wherein
**-L-** represents -(CH₂)ₒ- and o is an integer selected from 2, 3, 4, 5, 6, 7, and 8.

6. A conjugate comprising a saccharide of general formula (I) according to any of the claims 1 - 5 covalently linked to an immunogenic carrier through the nitrogen atom of the -O-L-NH₂ group, wherein the immunogenic carrier is a carrier protein or a glycosphingolipid.

7. The conjugate according to claim 6 of general formula (VI)
**[H-Uₓ₊₁-Uₓ-T-O-L-NH-W]ₘ-IM** **(VI)**
wherein m is comprised between about 2 and about 18;
**-W-** is selected from: 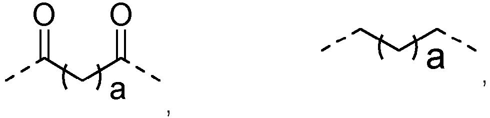 and 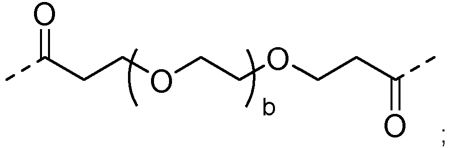
**a** represents an integer from 1 to 10;
**b** represents an integer from 1 to 4;
**IM** represents an immunogenic carrier, and
**Uₓ, Uₓ₊₁, T, x, n** and **L** have the meanings as defined in any one of the claims 1 -5.

8. The conjugate according to claim 6 of general formula **(VII)**
**[H-Uₓ₊₁-Uₓ-T-O-L-NH-W]ₘ-CRM₁₉₇** **(VII)**
wherein **m** is comprised between about 2 and about 18;
**-W-** is selected from: 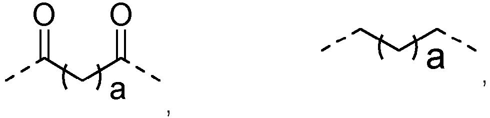 and 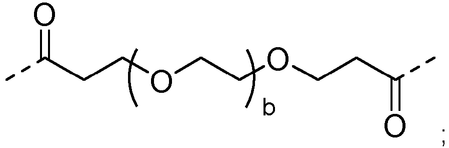
**a** represents an integer from 1 to 10;
**b** represents an integer from 1 to 4; and
**Uₓ, Uₓ₊₁, T, x, n** and **L** have the meanings as defined in any one of the claims 1 - 5.

9. The conjugate according to claim 7 or 8, wherein
**-L-** represents -**L^{a}**-, -**L^{a}-L^{e}**-, -**L^{a}-L^{b}-L^{e}**-, or -**L^{a}-L^{d}-L^{e}-** ;
-**L^{a}**- represents -(CH₂)ₒ-, -(CH₂CH₂O)ₒ-C₂H₄-, or -(CH₂-CH₂-O)ₒ-CH₂;
**-L^{b}**- represents -O-, -NH-CO-NH-, -NH-CO-CH₂-NH-, -NH-CO-;
**-L^{d}**- represents -(CH₂)_{q}-, -(CH(OH))_{q}-, -(CF₂)_{q}-, -(CH₂-CH₂-O)_{q}-C₂H₄-, or -(CH₂-CH₂-O)_{q}-CH₂-;
**-L^{e}-** represents -(CH₂)ₚ₁-, -(CF₂)ₚ₁-, -C₂H₄-(O-CH₂-CH₂)ₚ₁-, -CH₂-(O-CH₂-CH₂)ₚ₁- or -(CH₂)ₚ₁-O-(CH₂)ₚ₂-; and
**o, q, p1** and **p2** are independently of each other an integer selected from 1, 2, 3, 4, 5, and 6.

10. The conjugate according to claim 6, wherein the glycosphingolipid is (2S,3S,4R)-1-(α-D-galactopyranosyl)-2-hexacosanoylaminooctadecane-3,4-diol.

11. A pharmaceutical composition comprising at least one saccharide according to any one of the claims 1 - 5 and/or at least one conjugate according any one of the claims 6 - 10 as an active ingredient together with at least one pharmaceutically acceptable adjuvant and/or excipient.

12. The saccharide according to any one of the claims 1 - 5, the conjugate according to any one of the claims 6 - 10, or the pharmaceutical composition according to claim 11 for use in prevention and/or treatment of a *Francisella tularensis* infection in an animal susceptible to a *Francisella tularensis* infection.

13. A monoclonal antibody or a fragment thereof having specificity for a saccharide according to any one of the claims 1 - 5.

14. A diagnostic kit for conducting an immunological assay for detection of antibodies against *Francisella tularensis* comprising at least one saccharide according to any one of the claims 1 - 5 on a solid support.

15. A diagnostic kit for detection of *Francisella tularensis* bacteria in a specimen comprising a monoclonal antibody according to claim 13.
